# EUROPEAN PATENT SPECIFICATION

(11) **EP 1 539 141 B1**
(45) Date of publication and mention of the grant of the patent: **14.07.2010**
(21) Application number: 03791670.7
(22) Date of filing: 12.08.2003
(51) Int. Cl.: A61K 31/4045, A61P 29/00, C07D 209/16, C07D 209/30, C07D 409/12, C07D 417/12, C07D 413/12, C07D 401/12, C07D 409/14, C07D 403/12

(54) **3-(SULFONAMIDOETHYL)-INDOLE DERIVATIVES FOR USE AS GLUCOCORTICOID MIMETICS IN THE TREATMENT OF INFLAMMATORY, ALLERGIC AND PROLIFERATIVE DISEASES**
3-(SULFONAMIDOETHYL)-INDOL-DERIVATE ZUR VERWENDUNG ALS GLUCOCORTICOID-MIMETIKA BEI DER BEHANDLUNG VON ENTZÜNDLICHEN, ALLERGISCHEN UND PROLIFERATIVEN ERKRANKUNGEN
DERIVES DE 3-(SULFONAMIDOETHYL)-INDOLE DESTINES A ETRE UTILISES COMME AGENTS MIMETIQUES GLUCOCORTICOIDES DANS LE TRAITEMENT DES MALADIES INFLAMMATOIRES, ALLERGIQUES ET PROLIFERATIVES

(30) Priority: 29.08.2002 US 407438 P
(43) Date of publication of application: 15.06.2005
(73) Proprietor: Boehringer Ingelheim Pharmaceuticals Inc., Ridgefield, Connecticut 06877-0368 (US)
(72) Inventor: MARSHALL, Daniel Richard Boehringer Ingelheim, Ridgefield, CT 06877-0368 (US)
(74) Representative: Hammann, Heinz
(86) International application number: PCT/US2003/025062
(87) International publication number: WO 2004/019935

(56) References cited:
- WO-A-01/83471
- WO-A-02/09702
- US-A- 4 774 240
- HO, B.T. ET AL.: "Central Nervous System Depressive Activity of Some Amides of Tryptamine" J. MED. CHEM., vol. 14, no. 6, 1971, pages 553-554, XP002261118
- NENAJDENKO V G ET AL: "A new convenient approach to chiral beta-aryl(heteroaryl)alkylamines" TETRAHEDRON: ASYMMETRY, ELSEVIER SCIENCE PUBLISHERS, AMSTERDAM, NL, vol. 12, no. 18, 15 October 2001 (2001-10-15), pages 2517-2527, XP004323477 ISSN: 0957-4166
- SHONO T ET AL: "Electroorganic chemistry 81: Anodic oxidation of sulfonamides and amidophosphates" JOURNAL OF ORGANIC CHEMISTRY, AMERICAN CHEMICAL SOCIETY. EASTON, US, vol. 49, 1984, pages 3711-3716, XP002241082 ISSN: 0022-3263
- BAILEY A S ET AL: "FURTHER EXAMINATION OF THE REACTIONS OF SIMPLE INDOLES WITH ARENESULPHONYL AZIDES" JOURNAL OF THE CHEMICAL SOCIETY, PERKIN TRANSACTIONS 1, CHEMICAL SOCIETY. LETCHWORTH, GB, no. 15, 1973, pages 1602-1606, XP009014880 ISSN: 0300-922X
- PALMISANO G ET AL: "SYNTHETIC STUDIES ON INDOLE ALKALOIDS. A STEREOCONTROLLED ENTRY TO THE CUANZINE STRUCTURAL UNIT" TETRAHEDRON, ELSEVIER SCIENCE PUBLISHERS, AMSTERDAM, NL, vol. 45, no. 11, 1989, pages 3583-3596, XP002029549 ISSN: 0040-4020
- DATABASE CROSSFIRE BEILSTEIN [Online] Beilstein Institut zur Förderung der Chemischen Wissenschaften, Frankfurt am Main, DE; Database accession no. BRN 7178110,7178430 XP002261121 & ONISTSCHENKO, A. & STAMM, H.: CHEM. BER., vol. 122, 1989, pages 2397-2398,
- DATABASE CROSSFIRE BEILSTEIN [Online] Beilstein Institut zur Förderung der Chemischen Wissenschaften, Frankfurt am Main, DE; Database accession no. BRN 318403,341608 XP002261122 & HOSHINO & KOBAYASHI: JUSTUS LIEBIGS ANN. CHEM., vol. 520, 1935, pages 11-17,
- KUTNEY, J.P. ET AL.: "Total synthesis of dregamine and epidregamine. A general route to 2-acylindole alkaloids" J. AM. CHEM. SOC., vol. 100, no. 3, 1978, pages 938-943, XP002261119
- CHEMICAL ABSTRACTS, vol. 94, no. 19, 11 May 1981 (1981-05-11) Columbus, Ohio, US; abstract no. 156681z, LAGIDZE, D.R. ET AL.: "Synthesis of some new analogs of melatonin and beta-carboline from 4-phenylpentanoic acid" page 637; XP002261120 & IZV. AKAD. NAUK. GRUZ. SSR, SER. KHIM. , vol. 6, no. 3, 1980, pages 225-231,
- PATENT ABSTRACTS OF JAPAN vol. 010, no. 004 (C-322), 9 January 1986 (1986-01-09) & JP 60 163814 A (WATARU MORI), 26 August 1985 (1985-08-26)

## Description

### Field of the Invention

The present invention relates to glucocorticoid mimetics or ligands, methods of making such compounds, their use in pharmaceutical compositions, and their use in modulating the glucocorticoid receptor function, treating disease-states or conditions mediated by the glucocorticoid receptor function in a patient in need of such treatment, and other uses.

### Background of the Invention

Glucocorticoids, a class of corticosteroids, are endogenous hormones with profound effects on the immune system and multiple organ systems. They suppress a variety of immune and inflammatory functions by inhibition of inflammatory cytokines such as IL-1, IL-2, IL-6, and TNF, inhibition of arachidonic acid metabolites including prostaglandins and leukotrienes, depletion of T-lymphocytes, and reduction of the expression of adhesion molecules on endothelial cells (P.J. Barnes, Clin. Sci., 1998, 94, pp. 557-572; P.J. Barnes et al., Trends Pharmacol. Sci., 1993, 14, pp. 436-441). In addition to these effects, glucocorticoids stimulate glucose production in the liver and catabolism of proteins, play a role in electrolyte and water balance, reduce calcium absorption, and inhibit osteoblast function.

The anti-inflammatory and immune suppressive activities of endogenous glucocorticoids have stimulated the development of synthetic glucocorticoid derivatives including dexamethasone, prednisone, and prednisolone (L. Parente, Glucocorticoids, N.J. Goulding and R.J. Flowers (eds.), Boston: Birkhauser, 2001, pp. 35-54). These have found wide use in the treatment of inflammatory, immune, and allergic disorders including rheumatic diseases such as rheumatoid arthritis, juvenile arthritis, and ankylosing spondylitis, dermatological diseases including psoriasis and pemphigus, allergic disorders including allergic rhinitis, atopic dermatitis, and contact dermatitis, pulmonary conditions including asthma and chronic obstructive pulmonary disease (COPD), and other immune and inflammatory diseases including Crohn disease, ulcerative colitis, systemic lupus erythematosus, autoimmune chronic active hepatitis, osteoarthritis, tendonitis, and bursitis (J. Toogood, Glucocorticoids, N.J. Goulding and R.J. Flowers (eds.), Boston: Birkhauser, 2001, pp. 161-174). They have also been used to help prevent rejection in organ transplantation.

Unfortunately, in addition to the desired therapeutic effects of glucocorticoids, their use is associated with a number of adverse side effects, some of which can be severe and life-threatening. These include alterations in fluid and electrolyte balance, edema, weight gain, hypertension, muscle weakness, development or aggravation of diabetes mellitus, and osteoporosis. Therefore, a compound that exhibited a reduced side effect profile while maintaining the potent anti-inflammatory effects would be particularly desirable, especially when treating a chronic disease.

The effects of glucocorticoids are mediated at the cellular level by the glucocorticoid receptor (R.H. Oakley and J. Cidlowski, Glucocorticoids, N.J. Goulding and R.J. Flowers (eds.), Boston: Birkhauser, 2001, pp. 55-80). The glucocorticoid receptor is a member of a class of structurally related intracellular receptors that when coupled with a ligand can function as a transcription factor that affects gene expression (R.M. Evans, Science, 1988, 240, pp. 889-895). Other members of the family of steroid receptors include the mineralocorticoid, progesterone, estrogen, and androgen receptors. In addition to the effects mentioned above for glucocorticoids, hormones that act on this receptor family have a profound influence on body homeostasis, mineral metabolism, the stress response, and development of sexual characteristics. Glucocorticoids, N.J. Goulding and R.J. Flowers (eds.), Boston: Birkhauser, 2001, is hereby incorporated by reference in its entirety to better describe the state of the art.

A molecular mechanism which accounts for the beneficial anti-infiammatory effects and the undesired side effects has been proposed (e.g., S. Heck et al., EMBO J, 1994, 17, pp. 4087-4095; H.M. Reichardt et al., Cell, 1998, 93, pp. 531-541; F. Tronche et al., Curr. Opin. in Genetics and Dev., 1998, 8, pp. 532-538). Many of the metabolic and cardiovascular side effects are thought to be the result of a process called transactivation. In transactivation, the translocation of the ligand-bound glucocorticoid receptor to the nucleus is followed by binding to glucocorticoid response elements (GREs) in the promoter region of side effect-associated genes, for example, phosphoenolpyruvate carboxy kinase (PEPCK) in the case of increased glucose production. The result is an increased transcription rate of these genes which is believed to result, ultimately, in the observed side effects. The anti-inflammatory effects are thought to be due to a process called transrepression. In general, transrepression is a process independent of DNA binding that results from inhibition of NF-kB and AP-1-mediated pathways, leading to down regulation of many inflammatory and immune mediators. Additionally, it is believed that a number of the observed side effects may be due to the cross-reactivity of the currently available glucocorticoids with other steroid receptors, particularly the mineralocorticoid and progesterone receptors.

Thus, it may be possible to discover ligands for the glucocorticoid receptor that are highly selective and, upon binding, can dissociate the transactivation and transrepression pathways, providing therapeutic agents with a reduced side effect profile. Assay systems to determine effects on transactivation and transrepression have been described (e.g., C.M. Bamberger and H.M. Schulte, Eur. J. Clin. Invest., 2000, 30 (suppl. 3), pp. 6-9). Selectivity for the glucocorticoid receptor may be determined by comparing the binding affinity for this receptor with that of other steroid family receptors including those mentioned above.

Glucocorticoids also stimulate the production of glucose in the liver by a process called gluconeogenesis and it is believed that this process is mediated by transactivation events. Increased glucose production can exacerbate type II diabetes, therefore a compound that selectivity inhibited glucocorticoid mediated glucose production may have therapeutic utility in this indication (J.E. Freidman et al., J. Biol. Chem., 1997, 272, pp. 31475-31481).

Novel ligands for the glucocorticoid receptor have been described in the scientific and patent literature. For example, PCT International Publication No. WO 99/33786 discloses triphenylpropanamide compounds with potential use in treating inflammatory diseases. PCT International Publication No. WO 00/66522 describes non-steroidal compounds as selective modulators of the glucocorticoid receptor potentially useful in treating metabolic and inflammatory diseases. PCT International Publication No. WO 99/41256 describes tetracyclic modulators of the glucocorticoid receptor potentially useful in treating immune, autoimmune, and inflammatory diseases. U.S. Patent No. 5,688,810 describes various non-steroidal compounds as modulators of glucocorticoid and other steroid receptors. PCT International Publication No. WO 99/63976 describes a non-steroidal, liver-selective glucocorticoid antagonist potentially useful in the treatment of diabetes. PCT International Publication No. WO 00/32584 discloses non-steroidal compounds having anti-inflammatory activity with dissociation between anti-inflammatory and metabolic effects. PCT International Publication No. WO 98/54159 describes non-steroidal cyclically substituted acylanilides with mixed gestagen and androgen activity. U.S. Patent No. 4,880,839 describes acylanilides having progestational activity and EP 253503 discloses acylanilides with antiandrogenic properties. PCT International Publication No. WO 97/27852 describes amides that are inhibitors of farnesyl-protein transferase.

A compound that is found to interact with the glucocorticoid receptor in a binding assay could be an agonist or an antagonist. The agonist properties of the compound could be evaluated in the transactivation or transrepression assays described above. Given the efficacy demonstrated by available glucocorticoid drugs in inflammatory and immune diseases and their adverse side effects, there remains a need for novel glucocorticoid receptor agonists with selectivity over other members of the steroid receptor family and a dissociation of the transactivation and transrepression activities. Alternatively, the compound may be found to have antagonist activity. As mentioned above, glucocorticoids stimulate glucose production in the liver. Increased glucose production induced by glucocorticoid excess can exacerbate existing diabetes, or trigger latent diabetes. Thus a ligand for the glucocorticoid receptor that is found to be an antagonist may be useful, *inter alia,* for treating or preventing diabetes.

### Summary of the Invention

The instant invention is directed to compounds of Formula (I) 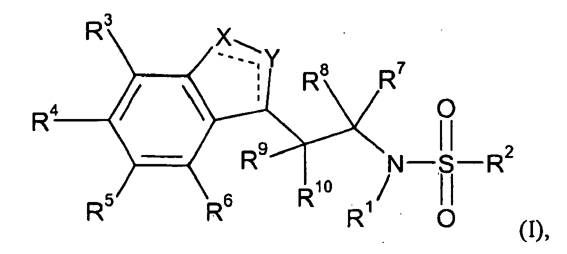 wherein:
- R¹: is hydrogen;
- R²: is an aryl, group, with one to five substituent groups, provided the -SO₂-R² group is not a p-toluene sulfonyl group
wherein each substituent group of R² is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁- C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, trifluoromethyl-, trifluoromethoxy, nitro, or amino wherein the nitrogen atom is optionally independently mono- or di-substituted by C₁-C₅ alkyl or aryl; or ureido wherein either nitrogen atom is optionally independently substituted with C₁-C₅ alkyl; or C₁-C₅ alkylthio wherein the sulfur atom is optionally oxidized to a sulfoxide or sulfone;
wherein each substituent group of R² is optionally independently substituted with one to three substituent groups selected from C₁-C₅ alkyl, C₁-C₅ alkoxy, halogen, hydroxy, oxo, cyano, or amino wherein the nitrogen atom is optionally independently mono- or di-substituted by C₁-C₅ alkyl,
- R³, R⁴, R⁵, and R⁶: are each independently hydrogen or C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁- C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein R³, R⁴, R⁵, and R⁶ are each optionally independently substituted with one to three substituent groups selected from C₁-C₃ alkyl, C₁-C₃ alkoxy, halogen, hydroxy, oxo, cyano, amino, or trifluoromethyl;
- R⁷ and R⁸: are each independently hydrogen or C₁-C₅ alkyl, each optionally substituted with one to three substituent groups,
wherein each substituent group of R⁷ and R⁸ is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁- C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein each substituent group of R⁷ and R⁸ is optionally independently substituted with one to three substituent groups selected from C₁-C₃ alkyl, C₁-C₃ alkoxy, halogen, hydroxy, oxo, cyano, amino, or trifluoromethyl,
- R⁹ and R¹⁰: are each hydrogen or C₁-C₅ alkyl, each optionally substituted with one to three substituent groups,
wherein each substituent group of R⁹ and R¹⁰ is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy,
wherein C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁- C₅ alkanoylamino, C₁-C₅ alkoxycarbonylamino, C₁-C₅ alkylsulfonylamino, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein each substituent group of R⁹ and R¹⁰ is optionally independently substituted with one to three substituent groups selected from C₁-C₃ alkyl, C₁-C₃ alkoxy, halogen, hydroxy, oxo, cyano, amino, or trifluoromethyl, at least one of R⁷, R⁸, R⁹, or R¹⁰ is optionally substituted C₇-C₅ alkyl;
- X: is a CH₂ group optionally substituted with one or two substituent groups or an NH group optionally substituted with one substituent group if no optional bond to X is present, or is a CH group optionally substituted with one substituent group or an N if an optional bond to X is present; and
- Y: is a CH₂ group optionally substituted with one or two substituent groups or an NH group optionally substituted with one substituent group if no optional bond to Y is present, is a CH group optionally substituted with one substituent group or an N if an optional bond to Y is present, wherein each substituent group of X and Y is independently C₁-C₃ alkyl, C₁-C₃ alkoxy, halogen, hydroxy, oxo, cyano, amino, or trifluoromethyl,
wherein each dashed line represents an optional bond, provided that zero or one of the optional bonds is present in the compound of Formula (I),
or a tautomer, ester, amide, solvate, or salt thereof.

One aspect of the invention includes compounds of Formula (I), wherein:
- R¹: is hydrogen
- R²: is a phenyl or naphthyl group, each optionally substituted with one to five substituent groups,
wherein each substituent group of R² is independently C₁-C₅ alkyl, C₂-C₅ alkenyl, C₂-C₅ alkynyl, C₃-C₈ cycloalkyl, heterocyclyl, phenyl, naphthyl, heteroaryl, C₁-C₅ alkoxy, C₂- C₅ alkenyloxy, C₂-C₅ alkynyloxy, aryloxy, trifluoromethyl, trifluoromethoxy, halogen, or amino wherein the nitrogen atom is optionally independently mono- or di-substituted by C₁-C₅ alkyl or aryl, or C₁-C₅ alkylthio;
wherein each substituent group of R² is optionally independently substituted with one to three substituent groups selected from C₁-C₅ alkyl, C₁-C₅ alkoxy, halogen, hydroxy, oxo, cyano, or amino wherein the nitrogen atom is optionally independently mono- or di-substituted by C₁-C₅ alkyl,
- R³, R⁴, R³, and R⁶: are each independently hydrogen or C₁-C₅ alkyl, C₂-C₅ alkenyl, C₂-C₅ alkynyl, C₃-C₈ cycloalkyl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, halogen, hydroxy, carboxy, cyano, trifluoromethyl, trifluoromethoxy, trifluoromethylthio, or C₁-C₅ alkylthio wherein the sulfur atom is optionally oxidized to a sulfoxide or sulfone;
- R⁷ and R⁸: are each independently hydrogen or C₁-C₅ alkyl, each optionally substituted with one to three substituent groups,
wherein each substituent group of R⁷ and R⁸ is independently C₁-C₅ alkyl, C₂-C₅ alkenyl, C₂-C₅ alkynyl, C₃-C₈ cycloalkyl, aryl, halogen, trifluoromethyl, trifluoromethoxy, trifluoromethylthio, or C₁-C₅ alkylthio wherein the sulfur atom is optionally oxidized to a sulfoxide or sulfone,
wherein each substituent group of R⁷ and R⁸ is optionally independently substituted with one to three substituent groups selected from C₁-C₃ alkyl, C₁-C₃ alkoxy, halogen, hydroxy, oxo, cyano, amino, or trifluoromethyl,
- R⁹ and R¹⁰: are each hydrogen or C₁-C₅ alkyl, each optionally substituted with one to three substituent groups,
wherein each substituent group of R⁹ and R¹⁰ is independently C₁-C₅ alkyl, C₂-C₅ alkenyl, C₂-C₅ alkynyl, C₃-C₈ cycloalkyl, aryl, halogen, trifluoromethyl, trifluoromethoxy, trifluoromethylthio, or C₁-C₅ alkylthio wherein the sulfur atom is optionally oxidized to a sulfoxide or sulfone,
wherein each substituent group of R⁹ and R¹⁰ is optionally independently substituted with one to three substituent groups selected from C₁-C₃ alkyl, C₁-C₃ alkoxy, halogen, hydroxy, oxo, cyano, amino, or trifluoromethyl,
or a tautomer, ester, amide, solvate, or salt thereof

Another aspect of the invention includes compounds of Formula (I), wherein:
- R¹: is hydrogen;
- R²: is a phenyl group, substituted with one to three substituent groups,
wherein each substituent group of R² is independently C₁-C₅ alkyl or halogen,
- R³, R⁴, R⁵, and R⁶: are each independently hydrogen or halogen;
- R⁷ and R⁸: are each independently hydrogen or C₁-C₅ alkyl, each optionally substituted with one to three substituent groups, wherein each substituent group of R⁷ and R⁸ is independently C₁-C₅ alkyl, wherein each substituent group of R⁷ and R⁸ is optionally independently substituted with one to three substituent groups selected from C₁-C₃ alkyl; and
- R⁹ and R¹⁰: are each hydrogen,
or a tautomer, ester, amide, solvate, or salt thereof.

Other aspects of the invention includes compounds of Formula (I), wherein least one, two, or three of R⁷, R⁸, R⁹, or R¹⁰ is C₁-C₅ alkyl, or a tautomer, ester, amide, solvate, or salt thereof.

Still another aspect of the invention includes compounds of Formula (I), wherein each substituent group of R² is optionally independently substituted with one to three substituent groups selected from methyl, methoxy, chloro, bromo, or dimethylamino, or a tautomer, ester, amide, solvate, or salt thereof.

Yet another aspect of the invention includes compounds of Formula (I), wherein R² is a substituted phenyl, or naphthyl.

Other aspects of the invention includes compounds of Formula (I), wherein X and Y are unsubstituted.

The following are representative compounds of Formula (I) according to the invention:

| **Compound Name** | **Compound Structure** |
|---|---|
| 4-*tert*-Butyl-*N*-[2-(1*H*-indol-3-yl)-1-methylethyl]benzenesulfonamide | 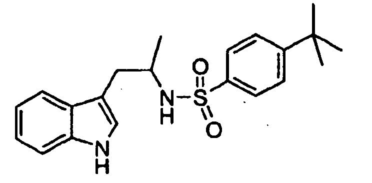 |
| 2,5-Dichloro-*N-*[2-(1*H*-indol-3-yl)-1-methylethyl]benzenesulfonamide | 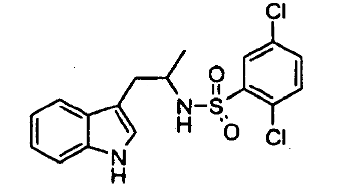 |
| (*R*)-2-(4-*tert*-Butylbenzenesulfonylamino)-3-(1*H*-indol-3-yl)propionic acid methyl ester | 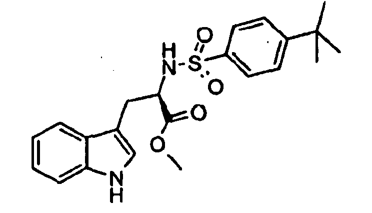 |
| 4*-tert*-Butyl-*N-*[2-(5-fluoro-1*H-*indol-3-yl)-1-methylethyl]benzenesulfonamide | 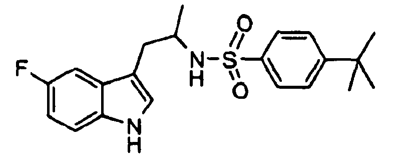 |
| 4-*tert*-Butyl-N-[1-methyl-2-(1-methyl-1*H-*indol-3-yl)ethyl]benzenesulfonamide | 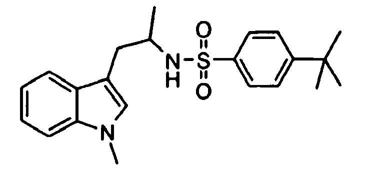 |
| 4-*tert*-Butyl*-N-*[1-hydroxymethyl-2-(1*H-*indol-3-yl)ethyl]benzenesulfonamide | 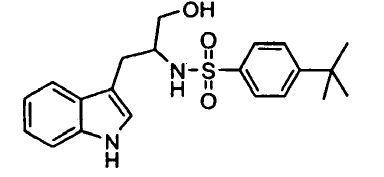 |
| 5-Fluoro-*N-*[2-(1*H-*indol-3-yl)-1-methylethyl]-2-methoxybenzenesulfonamide | 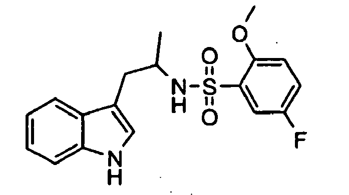 |
| 5-Dimethylaminonaphthalene-1-sulfonic acid [2-(1*H-*indol-3-yl)-1-methylethyl]amide | 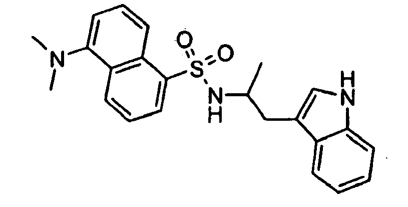 |
| 2,4,5-Trichloro-*N-*[2-(1*H-*indol-3-yl)-1-methylethyl]benzenesulfonamide | 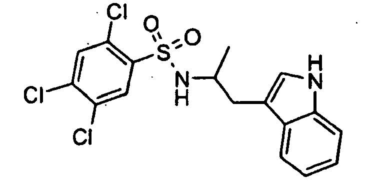 |
| *N-*[2-(1*H-*Indol-3-yl)-1-methylethyl]-2,4,6-trimethylbenzenesulfonamide | 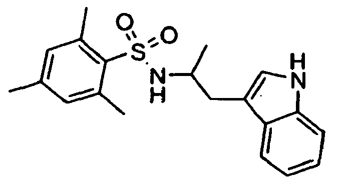 |
| *N-*[2-(1*H-*Indol-3-yl)-1-methylethyl]-2,3,4,5,6-pentamethylbenzenesulfonamide | 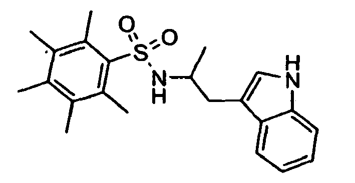 |
| *N-*[2-(1*H-*Indol-3-yl)-1-methylethyl]-4-methoxy-2,3,6-trimethylbenzenesulfonamide | 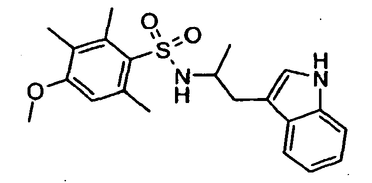 |
| 3,4-Dichloro-*N-*[2-(1*H-*indol-3-yl)-1-methylethyl]benzenesulfonamide | 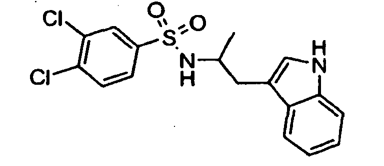 |
| 4-(1,1-Dimethylpropyl)-*N-*[2-(1*H-*indol-3-yl)-1-methylethyl]benzenesulfonamide | 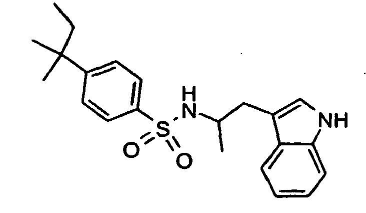 |
| 2-Chloro-*N-*[2-(1*H-*indol-3-yl)-1-methylethyl]-2-methylbenzenesulfonamide | 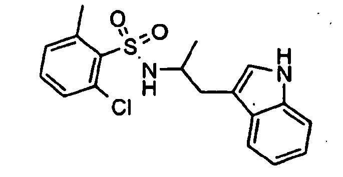 |
| 3-Chloro-*N-*[2-(1*H-*indol-3-yl)-1-methylethyl]-2-methylbenzenesulfonamide | 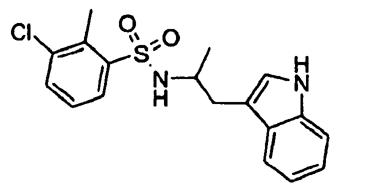 |
| 2,6-Dichloro-*N-*[2-(1*H-*indol-3-yl)-1-methylethyl]benzenesulfonamide | 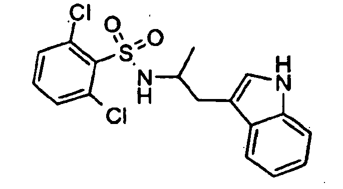 |
| 2,4-Dichloro-*N-*[2-(1*H-*indol-3-yl)-1-methylethyl]benzenesulfonamide | 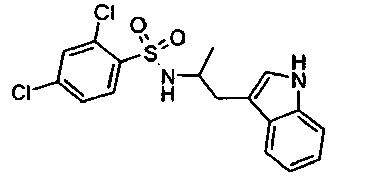 |
| 5-Fluoro-*N-*[2-(1*H-*indol-3-yl)-1-methylethyl]-2-methylbenzenesulfonamide | 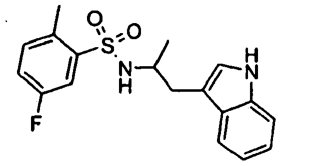 |
| 2,4,6-Trichloro-*N-*[2-(1*H-*indol-3-yl)-1-methylethyl]benzenesulfonamide | 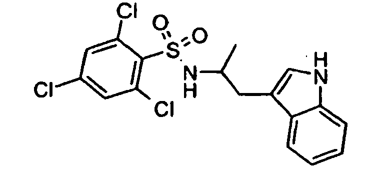 |
| 4-Butyl-*N-*[2-(1*H-*indol-3-yl)-1-methylethyl]benzenesulfonamide | 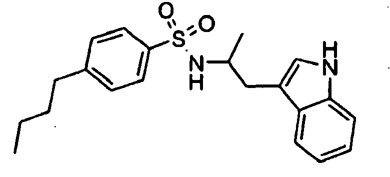 |
| *N-*[2-(1*H-*Indol-3-yl)-1-methylethyl]-2,4,6-triisopropylbenzenesulfonamide | 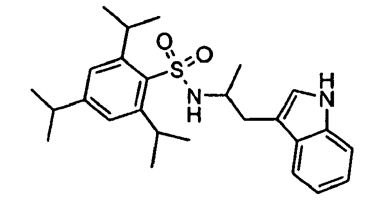 |
| 2,5-Dibromo-3,6-difluoro-*N-*[2-(1*H-*indol-3-yl)-1-methylethyl]benzenesulfonamide | 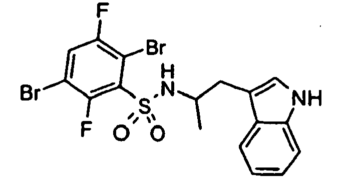 |
| *N-*[2-(1*H-*Indol-3-yl)-1-methylethyl]-2,3,5,6-tetramethylbenzenesulfonamide | 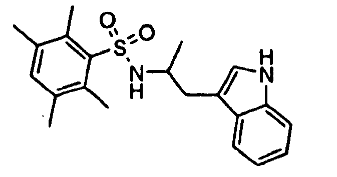 |
| 4-*tert*-Butyl-*N-*[1-(1*H-*indol-3-ylmethyl)propyl]benzenesulfonamide | 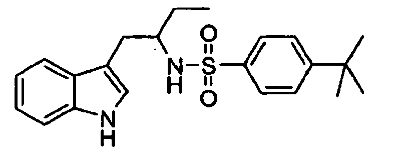 |
| 2,4,6-Trichloro-*N-*[1-(1*H-*indol-3-ylmethyl)propyl]benzenesulfonamide | 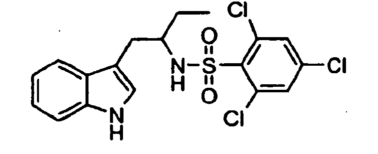 |
| 4-*tert*-Butyl-*N-*[2-(5-methoxy-1*H-*indol-3-yl)-1--methylethyl]benzenesulfonamide | 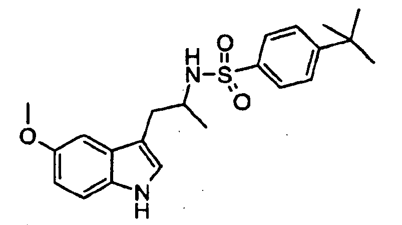 |
| 2,4,6-Trichloro-*N-*[2-(5-methoxy-1*H-*indol-3-yl)-1-methylethyl] benzenesulfonamide | 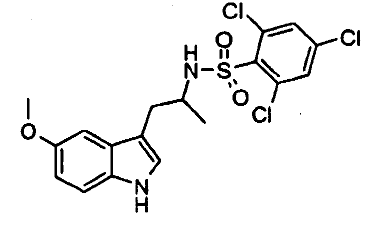 |
| *4-tert-*Butyl-*N-*[1-methyl-2-(2-methyl-1*H-*indol-3-yl)ethyl]benzenesulfonamide | 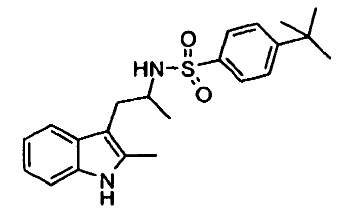 |
| 2,4,6-Trichloro-*N-*[1-methyl-2-(2-methyl-1*H-*indol-3-yl)ethyl]benzenesulfonamide | 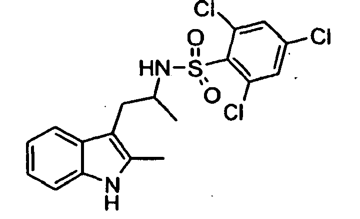 |
| 4-*tert*-Butyl-*N-*[2-(1*H-*indol-3-yl)-2-methylpropyl]benzenesulfonamide | 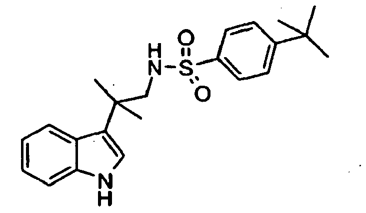 |
| 2,4,6-Trichloro-*N-*[2-(1*H-*indol-3-yl)-2-methylpropyl]benzenesulfonamide | 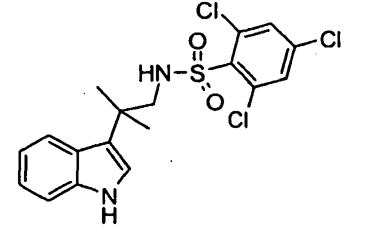 |
| *N-*[2-(5-Fluoro-1*H-*indol-3-yl)-1-methylethyl]-2,4,6-trimethyl-benzenesulfonamide | 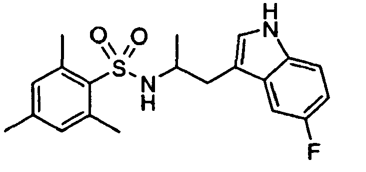 |
| *N-*[1-Hydroxymethyl-2-(1*H-*indol-3-yl)ethyl]-2,4,6-trimethylbenzenesulfonamide | 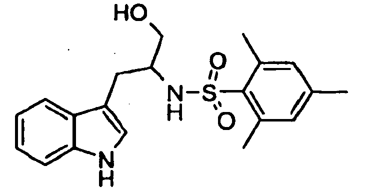 |
| 2,4,6-Trimethyl-*N-*[1-methyl-2-(1-methyl-1*H-*indol-3-yl)ethyl]benzenesulfonamide | 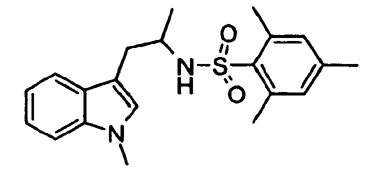 |
| (*S*)-3-(1*H-*Indol-3-yl)-2-(2,4,6-trimethylbenzenesulfonylamino)propionic acid methyl ester | 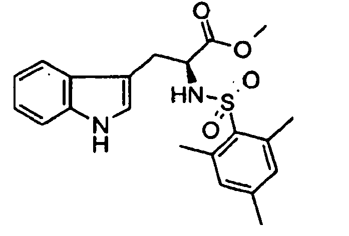 |
| (*R*)-3-(1*H-*Indol-3-yl)-2-(2,4,6-trimethylbenzenesulfonylamino)propionic acid methyl ester | 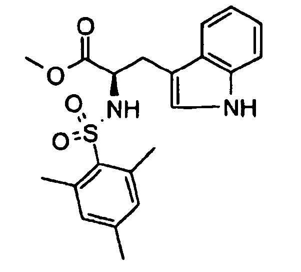 |
| *N*-[1-(1*H-*Indol-3-ylmethyl)propyl]-2,4,6-trimethylbenzenesulfonamide | 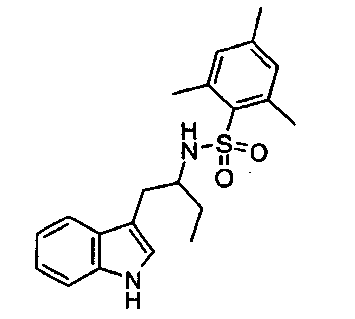 |
| *N-*[2-(6-Fluoro-1*H-*indol-3-yl)-1-methylethyl]-2,4,6-trimethylbenzenesulfonamide | 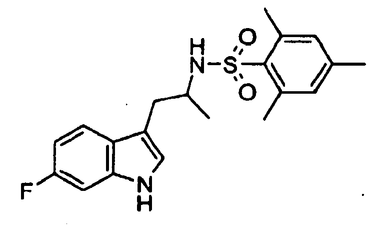 |
| 2,4,6-Trimethyl-*N-*[1-methyl-2-(7-methyl-1*H-*indol-3-yl)ethyl]benzenesulfonamide | 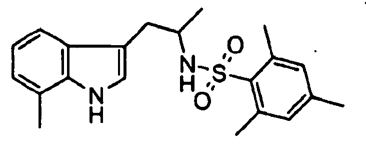 |
| 2,4,6-Trimethyl-*N-*[1-methyl-2-(6-methyl-1*H-*indol-3-yl)ethyl]benzenesulfonamide | 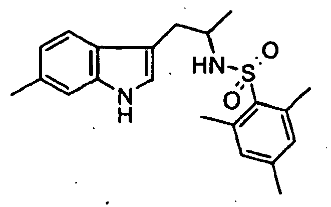 |
| *N-*[2-(1*H-*Indol-3-yl)-1-methylethyl]-4-iodobenzenesulfonamide | 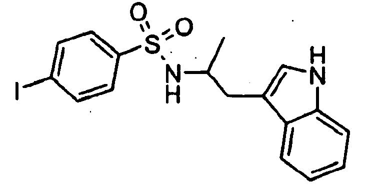 |
| *N-*[2-(1*H-*Indol-3-yl)-1-methytethyl]-2,4-dinitrobenzenesulfonamide | 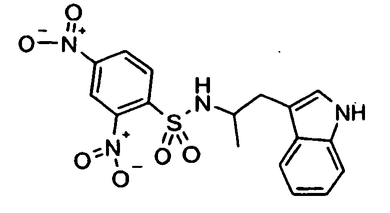 |
| *N-*[2-(1*H-*Indol-3-yl)-1-methylethyl]-2-nitrobenzenesulfonamide | 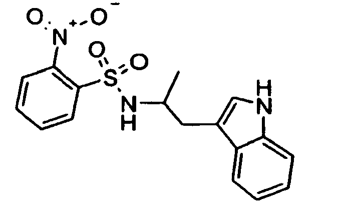 |
| 3,5-Dichloro-2-hydroxy-*N-*[2-(1*H-*indol-3-yl)-1-methylethyl]benzenesulfonamide | 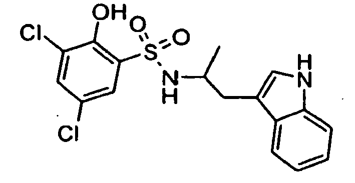 |
| *N-*[2-(1*H-*Indol-3-yl)-1-methylethyl]-3-nitrobenzenesulfonamide | 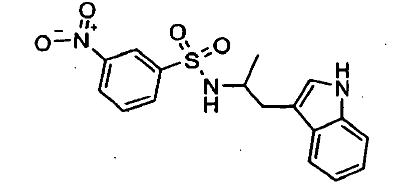 |
| 4-Bromo-*N-*[2-(1*H-*indol-3-yl)-1-methylethyl]benzenesulfonamide | 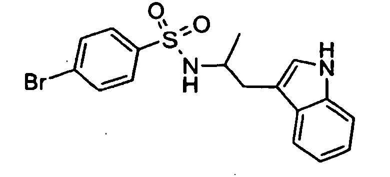 |
| 4-Fluoro-*N-*[2-(1*H-*indol-3-yl)-1-methylethyl]benzenesulfonamide | 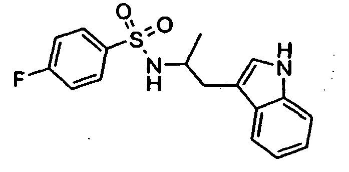 |
| 4-Chloro-*N-*[2-(1*H-*indol-3-yl)-1-methylethyl]benzenesulfonamide | 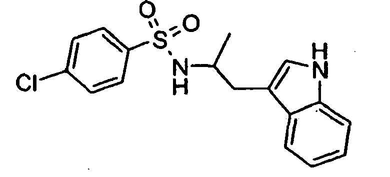 |
| 4-Chloro-*N-*[2-(1*H-*indol-3-yl)-1-methylethyl]-3-nitrobenzenesulfonamide | 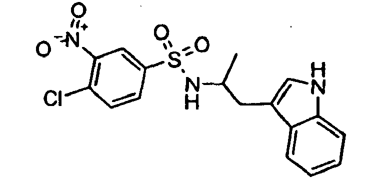 |
| *N-*{4-[2-(1*H-*Indol-3-yl)-1-methylethylsulfamoyl]phenyl}acetamide | 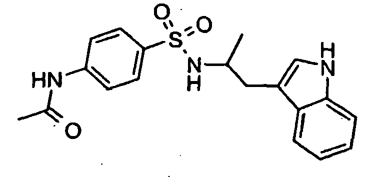 |
| *N-*[2-(1*H-*Indol-3-yl)-1-methylethyl]-4-nitrobenzenesulfonamide | 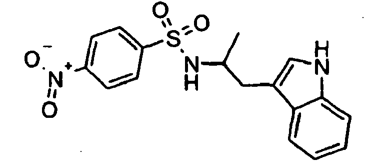 |
| *N-*[2-(1*H-*Indol-3-yl)-1-methylethyl]-4-methoxybenzenesulfonamide | 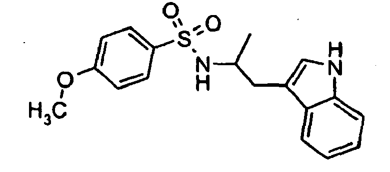 |
| *N-*[2-(1*H-*Indol-3-yl)-1-methylethyl]-3-methylbenzenesulfonamide | 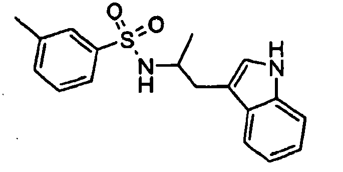 |
| *N-*[2-(1*H-*Indol-3-yl)-1-methylethyl]-2-nitro-4-trifluoromethylbenzenesulfonamide | 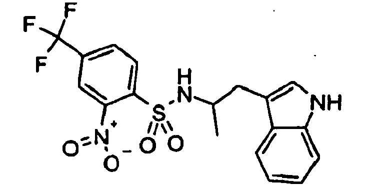 |
| *N-*[2-(1*H-*Indol-3-yl)-1-methylethyl]-3-trifluoromethylbenzenesulfonamide | 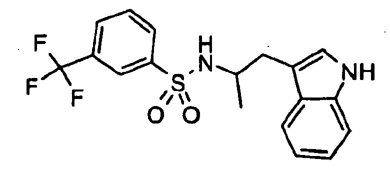 |
| 2,3,4-Trichloro-*N-*[2-(1*H-*indol-3-yl)-1-methylethyl]benzenesulfonamide | 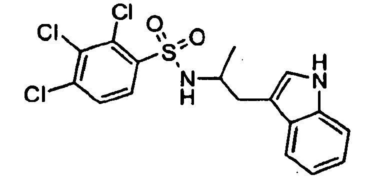 |
| *N-*[2-(1*H-*Indol-3-yl)-1-methylethyl]-2,5-dimethoxybenzenesulfonamide | 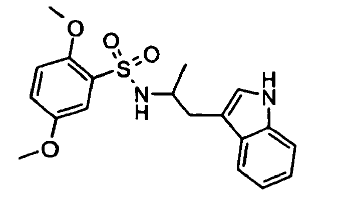 |
| 3,4-Dichloro-*N-*[2-(1*H-*indol-3-yl)-1-methylethyl]benzenesulfonamide | 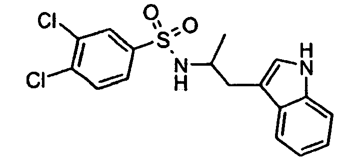 |
| 3-Chloro-4-fluoro-*N-*[2-(1*H-*indol-3-yl)-1-methylethyl]benzenesulfonamide | 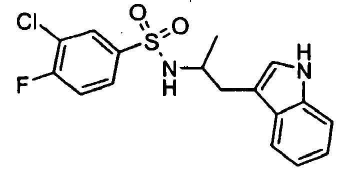 |
| 4-Ethyl-*N-*[2-(1*H-*indol-3-yl)-1-methylethyl]benzenesulfonamide | 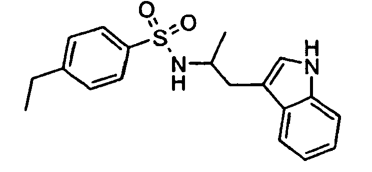 |
| *N-*[2-(1*H-*Indol-3-yl)-1-methylethyl]-4-propylbenzenesulfonamide | 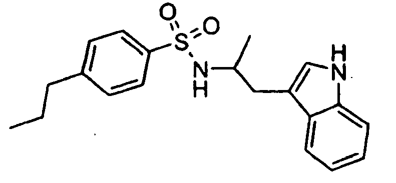 |
| 4-Bromo-2,5-difluoro-*N-*[2-(1*H-*indol-3-yl)-1-methylethyl]benzenesulfonamide | 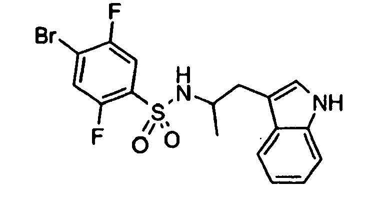 |
| 2-Fluoro-*N-*[2-(1*H-*indol-3-yl)-1-methylethyl]benzenesulfonamide | 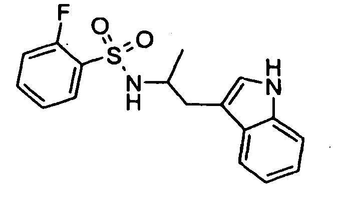 |
| *N-*[2-(1*H-*Indol-3-yl)-1-methylethyl]-4-trifluoromethoxybenzenesulfonamide | 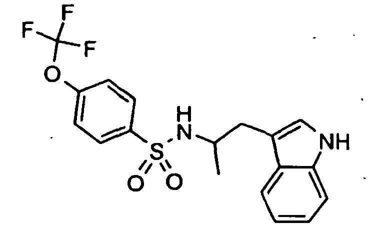 |
| *N-*[2-(1*H-*Indol-3-yl)-1-methylethyl]-4-trifluoromethylbenzenesulfonamide | 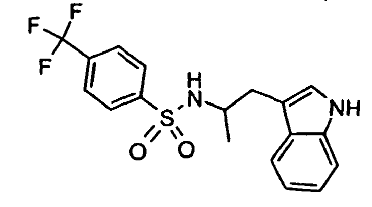 |
| 2,4-Difluoro-*N-*[2-(1*H-*indol-3-yl)-1-methylethyl]benzenesulfonamide | 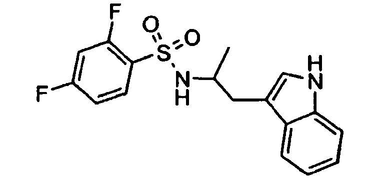 |
| 4-Chloro-*N-*[2-(1*H-*indol-3-yl)-1-methylethyl]-2,5-dimethylbenzenesulfonamide | 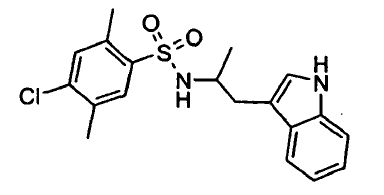 |
| 2-Chloro-*N-*[2-(1*H-*indol-3-yl)-1-methylethyl]benzenesulfonamide | 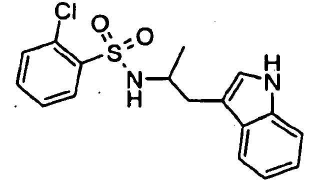 |
| *N-*[2-(1*H-*Indol-3-yl)-1-methylethyl]-2-methyl-5-nitrobenzenesulfonamide | 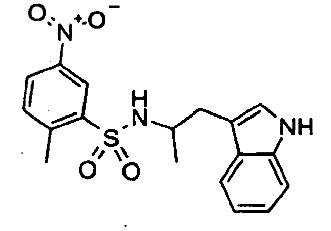 |
| *N-*[2-(1*H-*Indol-3-yl)-1-methylethl]-2-trifluoromethylbenzenesulfonamide | 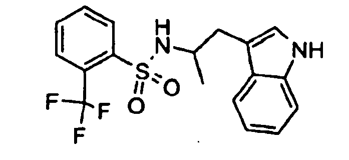 |
| 3-Chloro-*N-*[2-(1*H-*indol-3-yl)-1-methylethyl]benzenesulfonamide | 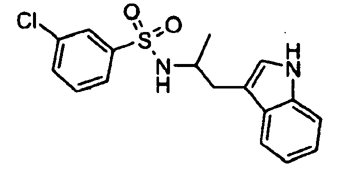 |
| 5-Bromo-*N-*[2-(1*H-*indol-3-yl)-1-methylethyl]-2-methoxybenzenesulfonamide | 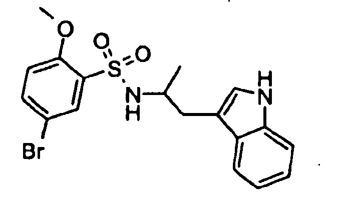 |
| *N-*[2-(1*H-*Indol-3-yl)-1-methylethyl]-3,4-dimethoxybenzenesulfonamide | 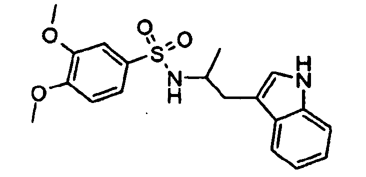 |
| 2,3-Dichloro-*N-*[2-(1*H-*indol-3-yl)-1-methylethyl]benzenesulfonamide | 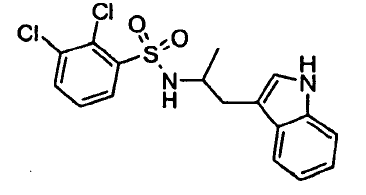 |
| 2-Bromo-*N-*[2-(1*H-*indol-3-yl)-1-methylethyl]benzenesulfonamide | 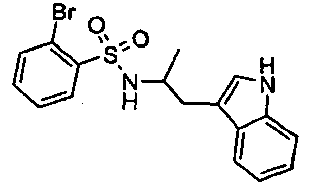 |
| 3-Bromo-*N-*[2-(1*H-*indol-3-yl)-1-methylethyl]benzenesulfonamide | 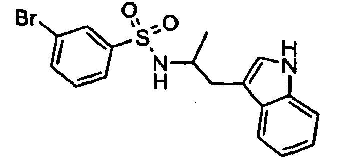 |
| *N-*[2-(1*H-*indol-3-yl)-1-methylethyl]-2-trifluoromethoxybenzenesulfonamide | 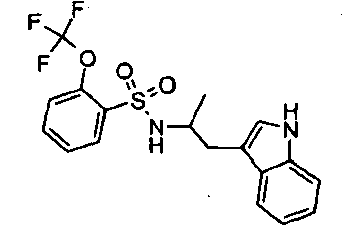 |
| 3-Cyano-*N-*[2-(1*H-*indol-3-yl)-1-methylethyl]benzenesulfonamide | 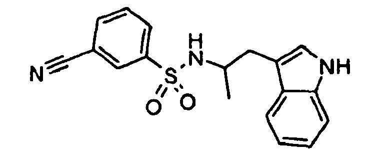 |
| 2-Cyano-*N-*[2-(1*H-*indol-3-yl)-1-methylethyl]benzenesulfonamide | 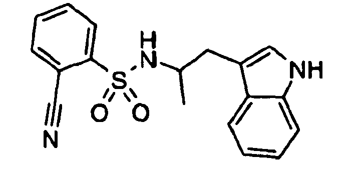 |
| *N-*[2-(1*H-*Indol-3-yl)-1-methylethyl]-2-methoxy-5-methylbenzenesulfonamide | 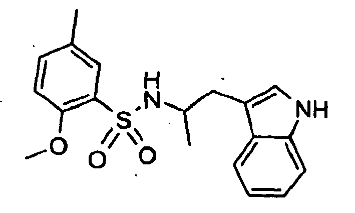 |
| 2-Chloro-4-fluoro-*N-*[2-(1*H-*indol-3-yl)-1-methylethyl]benzenesulfonamide | 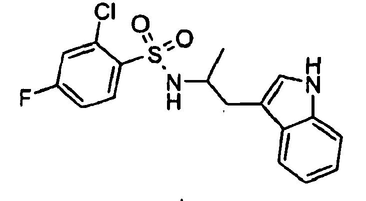 |
| 5-Chloro-*N-*[2-(1*H-*indol-3-yl)-1-methylethyl]-2-methoxybenzenesulfonamide | 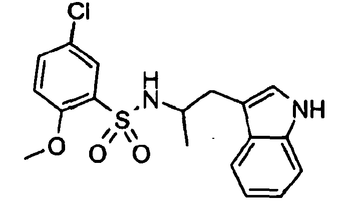 |
| Biphenyl-4-sulfonic acid [2-(1*H-*indol-3-yl)-1-methylethyl]amide | 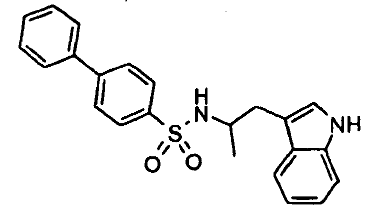 |
| 2,6-Difluoro-*N-*[2-(1*H-*indol-3-yl)-1-methylethyl]benzenesulfonamide | 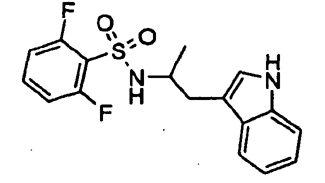 |
| *N-*[2-(1*H-*Indol-3-yl)-1-methylethyl]-4-methoxy-3,5-dinitrobenzenesulfonamide | 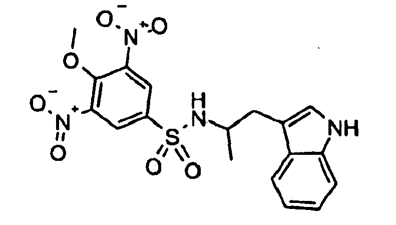 |
| *N-*[2-(1*H-*Indol-3-yl)-1-methylethyl]-4-methanesul fonylbenzenesulfonamide | 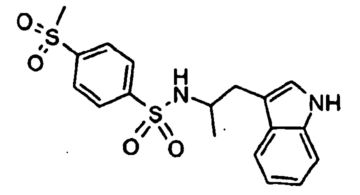 |
| *N-*[2-(1*H-*Indol-3-yl)-1-methylethyl]-2-methanesulfonylbenzenesulfonamide | 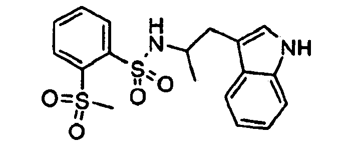 |
| 4-Acetyl-*N-*[2-(1*H-*indol-3-yl)-1-methylethyl]benzenesulfonamide | 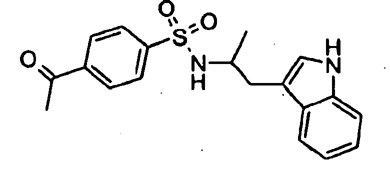 |
| 3,5-Dichloro-*N-*[2-(1*H-*indol-3-yl)-1-methylethyl]benzenesulfonamide | 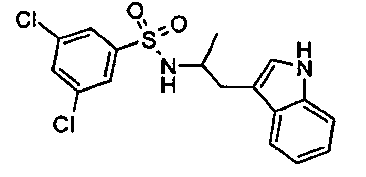 |
| *N-*[2-(1*H-*Endol-3-yl)-1-methylethyl]-3,5. bis-trifluoromethylbenzenesulfonamide | 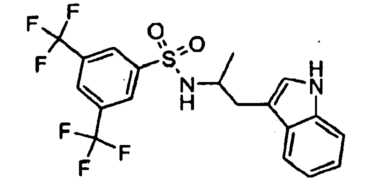 |
| or a tautomer, ester, amide, solvate, or salt thereof. | |

Preferred compounds of Formula (I) include the following:
5-Dimethylaminonaphthalene-1-sulfonic acid [2-(1*H-*indol-3-yl)-1-methylethyl]amide ;
*N-*[2-(1*H-*Indol-3-yl)-1-methylethyl]-2,4,6-trimethylbenzenesulfonamide;
*N-*[2-(1*H-*Indol-3-yl)-1-methylethyl]-2,3,4,5,6-pentamethylbenzenesulfonamide;
*N-*[2-(1*H-*Indol-3-yl)-1-methylethyl]-4-methoxy-2,3,6-trimethylbenzenesulfonamide;
2-Chloro-*N-*[2-(1*H-*indol-3-yl)-1-methylethyl]-6-methylbenzenesulfonamide;
2,6-Dichloro-*N-*[2-(1*H-*indol-3-yl)-1-methylethyl]benzenesulfonamide;
2,4,6-Trichloro-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide;
*4-tert-*Butyl-*N-*[1-(1*H-*indol-3-ylmethyl)propyl]benzenesulfonamide;
2,4,6-Trichloro-*N-*[1-(1*H-*indol-3-ylmethyl)propyl]benzenesulfonamide;
*N-*[2-(5-Fluoro-1*H-*indol-3-yl)-1-methylethyl]-2,4,6-trimethylbenzenesulfonamide;
*N-*[1-(1*H-*Indol-3-ylmethyl)propyl]-2,4,6-trimethylbenzenesulfonamide;
*N-*[2-(6-Fluoro-1*H-*indol-3-yl)-1-methylethyl]-2,4,6-trimethylbenzenesulfonamide;
2,4,6-Trimethyl-*N-*[1-methyl-2-(6-methyl-1*H-*indol-3-yl)ethyl]benzenesulfonamide; and
2,4,6-Trimethyl-*N-*[1-methyl-2-(7-methyl-1*H-*indol-3-yl)ethyl]benzenesulfonamide,
or a tautomer, ester, amide, solvate, or salt thereof.

More preferred compounds of Formula (I) include:
*N-*[2-(1*H-*Indol-3-yl)-1-methylethyl]-2,4,6-trimethylbenzenesulfonamide;
2,4,6-Trichloro-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide;
2,4,6-Trichloro-*N-*1-(1*H-*indol-3-ylmethyl)propyl]benzenesulfonamide;
*N-*[2-(5-Fluoro-1*H-*indol-3-yl)-1-methylethyl]-2,4,6-trimethylbenzenesulfonamide;
*N-*[1-(1*H-*Indol-3-ylmethyl)propyl]-2,4,6-trimethylbenzenesulfonamide;
*N-*[2-(6-Fluoro-1*H-*indol-3-yl)-1-methylethyl]-2,4,6-trimethylbenzenesulfonamide; and
2,4,6-Trimethyl-*N-*[1-methyl-2-(6-methyl-1*H-*indol-3-yl)ethyl]benzenesulfonamide,
or a tautomer, ester, amide, solvate, or salt thereof.

The invention also provides a method of making a compound of Formula (I) 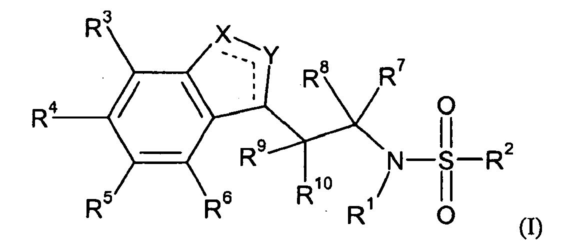 where R¹ is H and R², R³, R⁴, R⁵, R⁶, R⁷, R⁸, R⁹, R¹⁰, X, and Y are as defined in claim 1, the method comprising reacting the aminoethyl heterocycle of Formula (II) with a sulfonyl halide of Formula (III) in the presence of a suitable base to form the compound of Formula (I)

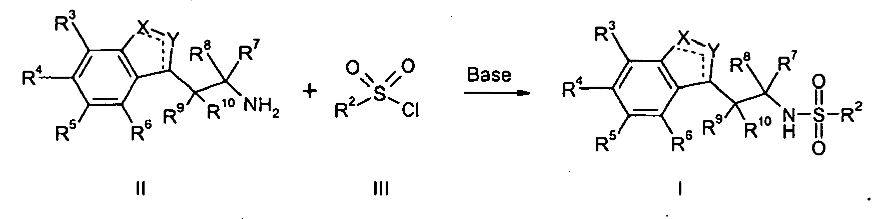

The invention further provides a method of making a compound of Formula (I) 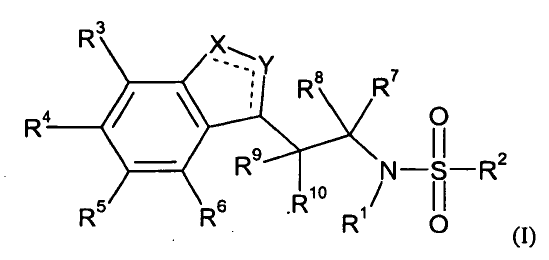 where R¹, R⁸, and R⁹ are each H and R², R³, R⁴, R⁵, R⁶, R⁷, R¹⁰, X, and Y are as defined in claim 1, the method comprising:
(a) reacting the aldehyde of Formula (V) with the nitro compound of Formula (VI) in the presence of ammonium acetate and acetic acid to form the nitroalkene of Formula (VII) 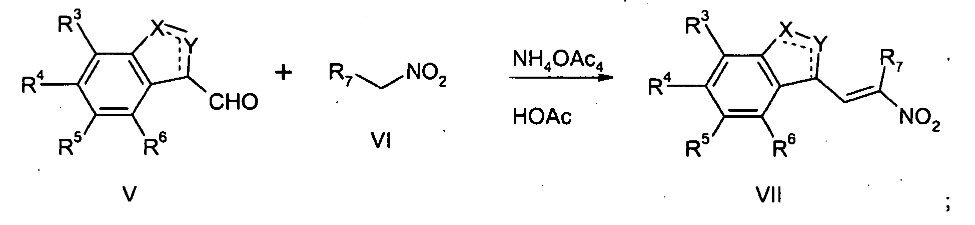
(b) reducing the nitroalkene of Formula (VII) with a suitable reducing agent to form the intermediate of Formula (II) 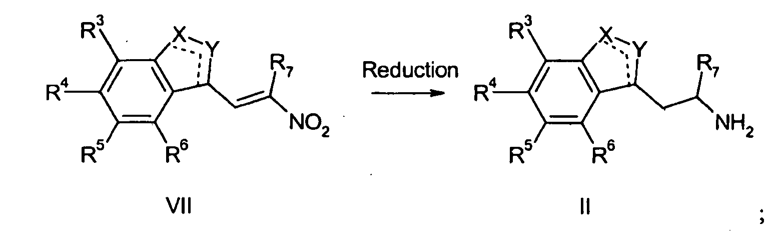 and
(c) reacting the intermediate of Formula (II) with a sulfonyl halide of Formula (III) in the presence of a suitable base to form the compound of Formula (I)

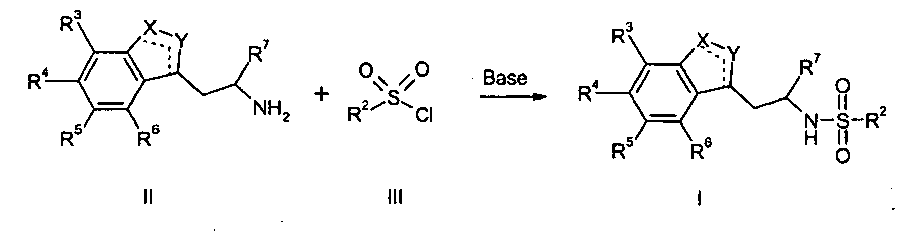

In another aspect of the invention, the compounds according to the invention are formulated into pharmaceutical compositions comprising an effective amount, preferably a pharmaceutically effective amount, of a compound according to the invention or a tautomer, ester, amide, solvate, or salt thereof, and a pharmaceutically acceptable excipient or carrier.

The invention allows for modulating the glucocorticoid receptor function in a patient, comprising administering to the patient an effective amount of a compound according to the invention or a tautomer, ester, amide, solvate, or salt thereof.

The invention further allows for treating a disease-state or condition mediated by the glucocorticoid receptor function in a patient in need of such treatment, comprising administering to the patient an effective amount of a pharmaceutically acceptable compound according to the invention or a tautomer, ester, amide, solvate, or salt thereof.

In addition, the invention allows for treating a disease-state or condition selected from: type II diabetes, obesity, cardiovascular diseases, hypertension, arteriosclerosis, neurological diseases, adrenal and pituitary tumors, and glaucoma, in a patient in need of such treatment, comprising administering to the patient an effective amount of a pharmaceutically acceptable compound according to the invention or a tautomer, ester, amide, solvate, or salt thereof.

The invention allows for treating a disease characterized by inflammatory, allergic, or proliferative processes, in a patient in need of such treatment, comprising administering to the patient an effective amount of a pharmaceutically acceptable compound according to the invention or a tautomer, ester, amide, solvate, or salt thereof. Preferably, the disease characterized by inflammatory, allergic, or proliferative processes is (i) lung diseases; (ii) rheumatic diseases or autoimmune diseases or joint diseases; (iii) allergic diseases; (iv) vasculitis diseases; (v) dermatological diseases; (vi) renal diseases; (vii) hepatic diseases; (viii) gastrointestinal diseases; (ix) proctological diseases; (x) eye diseases; (xi) diseases of the ear, nose, and throat (ENT) area; (xii) neurological diseases; (xiii) blood diseases; (xiv) tumor diseases; (xv) endocrine diseases; (xvi) organ and tissue transplantations and graft-versus-host diseases; (xvii) severe states of shock; (xviii) substitution therapy; and (xix) pain of inflammatory genesis. In particular the disease characterized by inflammatory, allergic, or proliferative processes can be type I diabetes, osteoarthritis, Guillain-Barre syndrome, restenosis following percutaneous transluminal coronary angioplasty, Alzheimer disease, acute and chronic pain, atherosclerosis, reperfusion injury, bone resorption diseases, congestive heart failure, myocardial infarction, thermal injury, multiple organ injury secondary to trauma, acute purulent meningitis, necrotizing enterocolitis, and syndromes associated with hemodialysis, leukopheresis, and granulocyte transfusion.

The invention also provides a kit for the *in vitro* diagnostic determination of the glucocorticoid receptor function in a sample, comprising: (a) a diagnostically effective amount of a compound according to the invention or a tautomer, ester, amide, solvate, or salt thereof; and (b) instructions for use of the diagnostic kit.

### Definition of Terms and Conventions Used

Terms not specifically defined herein should be given the meanings that would be given to them by one of skill in the art in light of the disclosure and the context. As used in the specification and appended claims, however, unless specified to the contrary, the following terms have the meaning indicated and the following conventions are adhered to.

### A. Chemical Nomenclature, Terms, and Conventions

In the groups, radicals, or moieties defined below, the number of carbon atoms is often specified preceding the group, for example, C₁-C₁₀ alkyl means an alkyl group or radical having 1 to 10 carbon atoms. The term "lower" applied to any carbon-containing group means a group containing from I to 8 carbon atoms, as appropriate to the group (i.e., a cyclic group must have at least 3 atoms to constitute a ring). In general, for groups comprising two or more subgroups, the last named group is the radical attachment point, for example, "alkylaryl" means a monovalent radical of the formula Alk-Ar-, while "arylalkyl" means a monovalent radical of the formula Ar-Alk- (where Alk is an alkyl group and Ar is an aryl group). Furthermore, the use of a term designating a monovalent radical where a divalent radical is appropriate shall be construed to designate the respective divalent radical and *vice versa.* Unless otherwise specified, conventional definitions of terms control and conventional stable atom valences are presumed and achieved in all formulas and groups.

The terms "alkyl" or "alkyl group" mean a branched or straight-chain saturated aliphatic hydrocarbon monovalent radical. This term is exemplified by groups such as methyl, ethyl, *n-*propyl, 1-methylethyl (isopropyl), *n-*butyl, *n-*pentyl, 1,1-dimethylethyl (*tert*-butyl), and the like. It may be abbreviated "Alk".

The terms "alkenyl" or "alkenyl group" mean a branched or straight-chain aliphatic hydrocarbon monovalent radical containing at least one carbon-carbon double bond. This term is exemplified by groups such as ethenyl, propenyl, *n-*butenyl, isobutenyl, 3-methylbut-2-enyl, *n-*pentenyl, heptenyl, octenyl, decenyl, and the like.

The terms "alkynyl" or "alkynyl group" mean a branched or straight-chain aliphatic hydrocarbon monovalent radical containing at least one carbon-carbon triple bond. This term is exemplified by groups such as ethynyl, propynyl, *n-*butynyl, 2-butynyl, 3-methylbutynyl, *n-*pentynyl, heptynyl, octynyl, decynyl, and the like.

The terms "alkylene" or "alkylene group" mean a branched or straight-chain saturated aliphatic hydrocarbon divalent radical having the specified number of carbon atoms. This term is exemplified by groups such as methylene, ethylene, propylene, *n-*butylene, and the like, and may alternatively and equivalently be denoted herein as -(alkyl)-.

The terms "alkenylene" or "alkenylene group" mean a branched or straight-chain aliphatic hydrocarbon divalent radical having the specified number of carbon atoms and at least one carbon-carbon double bond. This term is exemplified by groups such as ethenylene, propenylene, *n-*butenylene, and the like, and may alternatively and equivalently be denoted herein as -(alkylenyl)-.

The terms "alkynylene" or "alkynylene group" mean a branched or straight-chain aliphatic hydrocarbon divalent radical containing at least one carbon-carbon triple bond. This term is exemplified by groups such as ethynylene, propynylene, *n-*butynylene, 2-butynylene, 3-methylbutynylene, *n-*pentynylene, heptynylene, octynylene, decynylene, and the like, and may alternatively and equivalently be denoted herein as -(alkynyl)-.

The terms "alkoxy" or "alkoxy group" mean a monovalent radical of the formula AlkO-, where Alk is an alkyl group. This term is exemplified by groups such as methoxy, ethoxy, propoxy, isopropoxy, butoxy, sec-butoxy, *tert*-butoxy, pentoxy, and the like.

The terms "aryloxy", "aryloxy group", mean a monovalent radical of the formula ArO-, where Ar is aryl. This term is exemplified by groups such as phenoxy, naphthoxy, and the like.

The terms "alkylcarbonyl", "alkylcarbonyl group", "alkanoyl", or "alkanoyl group" mean a monovalent radical of the formula AlkC(O)-, where Alk is alkyl or hydrogen.

The terms "arylcarbonyl", "arylcarbonyl group", "aroyl" or "aroyl group" mean a monovalent radical of the formula ArC(O)-, where Ar is aryl.

The terms "acyl" or "acyl group" mean a monovalent radical of the formula RC(O)-, where R is a substituent selected from hydrogen or an organic substituent. Exemplary substituents include alkyl, aryl, arylalkyl, cycloalkyl, heterocyclyl, heteroaryl, heteroarylalkyl, and the like. As such, the terms comprise alkylcarbonyl groups and arylcarbonyl groups.

The terms "acylamino" or "acylamino group" mean a monovalent radical of the formula RC(O)N(R)-, where each R is a substituent selected from hydrogen or a substituent group.

The terms "alkoxycarbonyl" or "alkoxycarbonyl group" mean a monovalent radical of the formula AlkO-C(O)-, where Alk is alkyl. Exemplary alkoxycarbonyl groups include methoxycarbonyl, ethoxycarbonyl, *tert*-butyloxycarbonyl, and the like.

The terms "aryloxycarbonyl" or "aryloxycarbonyl group" mean a monovalent radical of the formula ArO-C(O)-, where Ar is aryl.

The terms "alkylcarbonyloxy" or "alkylcarbonyloxy group" or "alkanoyloxy" or "alkanoyloxy group" mean a monovalent radical of the formula AlkC(O)O-, where Alk is alkyl.

The terms "arylcarbonyloxy" or "arylcarbonyloxy group" or "aroyloxy" or "aroyloxy group" mean a monovalent radical of the formula ArC(O)O-, where Ar is aryl.

The terms "alkylaminocarbonyloxy" or "alkylaminocarbonyloxy group" mean a monovalent radical of the formula R₂NC(O)O-, where each R is independently hydrogen or lower alkyl.

The term "alkoxycarbonylamino" or "alkoxycarbonylamino group" mean a monovalent radical of the formula ROC(O)NH-, where R is lower alkyl.

The terms "alkylcarbonylamino" or "alkylcarbonylamino group" or "alkanoylamino" or "alkanoylamino groups" mean a monovalent radical of the formula AlkC(O)NH-, where Alk is alkyl. Exemplary alkylcarbonylamino groups include acetamido (CH₃C(O)NH).

The terms "alkylaminocarbonyloxy" or "alkylaminocarbonyloxy group" mean a monovalent radical of the formula AlkNHC(O)O-, where Alk is alkyl.

The terms "amino" or "amino group" mean an -NH₂ group.

The terms "alkylamino" or "alkylamino group" mean a monovalent radical of the formula (Alk)NH-, where Alk is alkyl. Exemplary alkylamino groups include methylamino, ethylamino, propylamino, butylamino, *tert*-butylamino, and the like.

The terms "dialkylamino" or "dialkylamino group" mean a monovalent radical of the formula (Alk)(Alk)N*-*, where each Alk is independently alkyl. Exemplary dialkylamino groups include dimethylamino, methylethylamino, diethylamino, dipropylamino, ethylpropylamino, and the like.

The terms "substituted amino" or "substituted amino group" mean a monovalent radical of the formula -NR₂, where each R is independently a substituent selected from hydrogen or the specified substituents (but where both Rs cannot be hydrogen). Exemplary substituents include alkyl, alkanoyl, aryl, arylalkyl, cycloalkyl, heterocyclyl, heteroaryl, heteroarylalkyl, and the like.

The terms "alkoxycarbonylamino" or "alkoxycarbonylamino group" mean a monovalent radical of the formula AlkOC(O)NH-, where Alk is alkyl.

The terms "ureido" or "ureido group" mean a monovalent radical of the formula R₂NC(O)NH-, where each R is independently hydrogen or alkyl.

The terms "halogen" or "halogen group" mean a fluoro, chloro, bromo, or iodo group.

The term "halo" means one or more hydrogen atoms of the group are replaced by halogen groups.

The terms "haloalkyl" or "haloalkyl group" mean a branched or straight-chain saturated aliphatic hydrocarbon monovalent radical, wherein one or more hydrogen atoms thereof are each independently replaced with halogen atoms. This term is exemplified by groups such as chloromethyl, 1,2-dibromoethyl, 1,1,1-trifluoropropyl, 2-iodobutyl, 1-chloro-2-bromo-3-fluoropentyl, and the like.

The terms "sulfanyl", "sulfanyl group", "thioether", or "thioether group" mean a divalent radical of the formula -S-.

The terms "alkylthio" or "alkylthio group" mean a monovalent radical of the formula AlkS-, where Alk is alkyl. Exemplary groups include methylthio, ethylthio, *n-*propylthio, isopropylthio, *n-*butylthio, and the like.

The terms "arylthio" or "arylthio group" mean a monovalent radical of the formula ArS-, where Ar is aryl.

The terms "sulfinyl", "sulfinyl group", "thionyl", or "thionyl group" mean a divalent radical of the formula -SO-.

The terms "sulfonyl" or "sulfonyl group" mean a divalent radical of the formula -SO₂-.

The terms "sulfonylamino" or "sulfonylamino group" mean a divalent radical of the formula -SO₂NR- where R is a hydrogen or a substituent group.

The terms "aminosulfonyl" or "aminosulfonyl group" mean a monovalent radical of the formula NR₂SO₂-, where R is each independently a hydrogen or a substituent group.

The terms "carbocycle" or "carbocyclic group" mean a stable aliphatic 3- to 15-membered monocyclic or polycyclic monovalent or divalent radical consisting solely of carbon and hydrogen atoms which may comprise one or more fused or bridged ring(s), preferably a 5- to 7-membered monocyclic or 7- to 10-membered bicyclic ring. Unless otherwise specified, the carbocycle may be attached at any carbon atom which results in a stable structure and, if substituted, may be substituted at any suitable carbon atom which results in a stable structure. The term comprises cycloalkyl (including spiro cycloalkyl), cycloalkylene, cycloalkenyl, cycloalkenylene, cycloalkynyl, and cycloalkynylene, and the like.

The terms "cycloalkyl" or "cycloalkyl group" mean a stable aliphatic saturated 3- to 15- membered monocyclic or polycyclic monovalent radical consisting solely of carbon and hydrogen atoms which may comprise one or more fused or bridged ring(s), preferably a 5- to 7- membered monocyclic or 7- to 10-membered bicyclic ring. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornanyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

The terms "cycloalkenyl" or "cycloalkenyl group" mean a stable aliphatic 5- to 15-membered monocyclic or polycyclic monovalent radical having at least one carbon-carbon double bond and consisting solely of carbon and hydrogen atoms which may comprise one or more fused or bridged ring(s), preferably a 5- to 7-membered monocyclic or 7- to 10-membered bicyclic ring. Unless otherwise specified, the cycloalkenyl ring may be attached at any carbon atom which results in a stable structure and, if substituted, may be substituted at any suitable carbon atom which results in a stable structure. Exemplary cycloalkenyl groups include cyclopentenyl, cyclohexenyl, cycloheptenyl, cyclooctenyl, cyclononenyl, cyclodecenyl, norbornenyl, 2-methylcyclopentenyl, 2-methylcyclooctenyl, and the like.

The terms "cycloalkynyl" or "cycloalkynyl group" mean a stable aliphatic 8- to 15-membered monocyclic or polycyclic monovalent radical having at least one carbon-carbon triple bond and consisting solely of carbon and hydrogen atoms which may comprise one or more fused or bridged ring(s), preferably a 8- to 10-membered monocyclic or 12- to 15-membered bicyclic ring. Unless otherwise specified, the cycloalkynyl ring may be attached at any carbon atom which results in a stable structure and, if substituted, may be substituted at any suitable carbon atom which results in a stable structure. Exemplary cycloalkynyl groups include, cyclooctynyl, cyclononynyl, cyclodecynyl, 2-methylcyclooctynyl, and the like.

The terms "cycloalkylene" or "cycloalkylene group" mean a stable saturated aliphatic 3- to 15- membered monocyclic or polycyclic divalent radical consisting solely of carbon and hydrogen atoms which may comprise one or more fused or bridged ring(s), preferably a 5- to 7- membered monocyclic or 7- to 10-membered bicyclic ring. Unless otherwise specified, the cycloalkyl ring may be attached at any carbon atom which results in a stable structure and, if substituted, may be substituted at any suitable carbon atom which results in a stable structure. Exemplary cycloalkylene groups include cyclopentylene, and the like.

The terms "cycloalkenylene" or "cycloalkenylene group" mean a stable aliphatic 5- to 15- membered monocyclic or polycyclic divalent radical having at least one carbon-carbon double bond and consisting solely of carbon and hydrogen atoms which may comprise one or more fused or bridged ring(s), preferably a 5- to 7-membered monocyclic or 7- to 10-membered bicyclic ring. Unless otherwise specified, the cycloalkenylene ring may be attached at any carbon atom which results in a stable structure and, if substituted, may be substituted at any suitable carbon atom which results in a stable structure. Exemplary cycloalkenylene groups include cyclopentenylene, cyclohexenylene, cycloheptenylene, cyclooctenylene, cyclononenylene, cyclodecenylene, norbornenylene, 2-methylcyclopentenylene, 2-methylcyclooctenylene, and the like.

The terms "cycloalkynylene" or "cycloalkynylene group" mean a stable aliphatic 8- to 15- membered monocyclic or polycyclic divalent radical having at least one carbon-carbon triple bond and consisting solely of carbon and hydrogen atoms which may comprise one or more fused or bridged ring(s), preferably a 8- to 10-membered monocyclic or 12- to 15-membered bicyclic ring. Unless otherwise specified, the cycloalkynylene ring may be attached at any carbon atom which results in a stable structure and, if substituted, may be substituted at any suitable carbon atom which results in a stable structure. Exemplary cycloalkynylene groups include cyclooctynylene, cyclononynylene, cyclodecynylene, 2-methylcyclooctynylene, and the like.

The terms "aryl" or "aryl group" mean an aromatic carbocyclic monovalent or divalent radical of from 6 to 14 carbon atoms having a single ring (e.g., phenyl or phenylene) or multiple condensed rings (e.g., naphthyl or anthranyl). Unless otherwise specified, the aryl ring may be attached at any suitable carbon atom which results in a stable structure and, if substituted, may be substituted at any suitable carbon atom which results in a stable structure. Exemplary aryl groups include phenyl, naphthyl, anthryl, phenanthryl, indanyl, indenyl, biphenyl, and the like. It may be abbreviated "Ar".

The terms "heteroaryl" or "heteroaryl group" mean a stable aromatic 5- to 14-membered, monocyclic or polycyclic monovalent or divalent radical which may comprise one or more fused or bridged ring(s), preferably a 5- to 7-membered monocyclic or 7- to 10-membered bicyclic radical, having from one to four heteroatoms in the ring(s) independently selected from nitrogen, oxygen, and sulfur, wherein any sulfur heteroatoms may optionally be oxidized and any nitrogen heteroatom may optionally be oxidized or be quatemized. Unless otherwise specified, the heteroaryl ring may be attached at any suitable heteroatom or carbon atom which results in a stable structure and, if substituted, may be substituted at any suitable heteroatom or carbon atom which results in a stable structure. Exemplary and preferred heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, azaindolyl, dihydroindolyl, isoindolyl, benzofuranyl, dihydrobenzofuranyl, benzothienyl, dihydrobenzothienyl, indazolyl, benzimidazolyl, benzthiazolyl, benzoxazolyl, benzisoxazolyl, benzpyrazolyl, purinyl, quinolizinyl, quinolinyl, dihydroquinolinyl, tetrahydroquinolinyl, isoquinolinyl, dihydroisoquinolinyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl, and the like.

The terms "heterocycle", "heterocycle group", "heterocyclyl", or "heterocyclyl group" mean a stable non-aromatic 5- to 14-membered monocyclic or polycyclic, monovalent or divalent, ring which may comprise one or more fused or bridged ring(s), preferably a 5- to 7-membered monocyclic or 7- to 10-membered bicyclic ring, having from one to three heteroatoms in the ring(s) independently selected from nitrogen, oxygen, and sulfur, wherein any sulfur heteroatoms may optionally be oxidized and any nitrogen heteroatom may optionally be oxidized or be quatemized. Unless otherwise specified, the heterocyclyl ring may be attached at any suitable heteroatom or carbon atom which results in a stable structure and, if substituted, may be substituted at any suitable heteroatom or carbon atom which results in a stable structure. Exemplary and preferred heterocycles include pyrrolinyl, pyrrolidinyl, pyrazolinyl, pyrazolidinyl, piperidinyl, morpholinyl, thiomorpholinyl, piperazinyl, tetrahydropyranyl, tetrahydrothiopyranyl, tetrahydrofuranyl, hexahydropyrimidinyl, hexahydropyridazinyl, and the like.

The term "compounds of the invention" and equivalent expressions are meant to embrace compounds of Formula (I) as herein described, including the tautomers, esters, amides, the salts, particularly the pharmaceutically acceptable salts, and the solvates and hydrates thereof, where the context so permits. In general and preferably, the compounds of the invention and the formulas designating the compounds of the invention are understood to only include the stable compounds thereof and exclude unstable compounds, even if an unstable compound might be considered to be literally embraced by the compound formula. Similarly, reference to intermediates, whether or not they themselves are claimed, is meant to embrace their salts and solvates, where the context so permits. For the sake of clarity, particular instances when the context so permits are sometimes indicated in the text, but these instances are purely illustrative and it is not intended to exclude other instances when the context so permits.

The terms "optional" or "optionally" mean that the subsequently described event or circumstances may or may not occur, and that the description includes instances where the event or circumstance occurs and instances in which it does not. For example, "optionally substituted aryl" means that the aryl radical may or may not be substituted and that the description includes both substituted aryl radicals and aryl radicals having no substitution.

The terms "stable compound" or "stable structure" mean a compound that is sufficiently robust to survive isolation to a useful degree of purity from a reaction mixture, and formulation into an efficacious therapeutic or diagnostic agent. For example, a compound which would have a "dangling valency" or is a carbanion is not a compound contemplated by the invention.

The term "substituted" means that any one or more hydrogens on an atom of a group or moiety, whether specifically designated or not, is replaced with a selection from the indicated group of substituents, provided that the atom's normal valency is not exceeded and that the substitution results in a stable compound. If a bond to a substituent is shown to cross the bond connecting two atoms in a ring, then such substituent may be bonded to any atom on the ring. When a substituent is listed without indicating the atom via which such substituent is bonded to the rest of the compound, then such substituent may be bonded via any atom in such substituent. For example, when the substituent is piperazinyl, piperidinyl, or tetrazolyl, unless specified otherwise, such piperazinyl, piperidinyl, or tetrazolyl group may be bonded to the rest of the compound of the invention via any atom in such piperazinyl, piperidinyl, or tetrazolyl group. Generally, when any substituent or group occurs more than one time in any constituent or compound, its definition on each occurrence is independent of its definition at every other occurrence. Thus, for example, if a group is shown to be substituted with 0 to 2 R⁵, then such group is optionally substituted with up to two R⁵ groups and R⁵ at each occurrence is selected independently from the defined list of possible R⁵. Such combinations of substituents and/or variables, however, are permissible only if such combinations result in stable compounds.

In a specific embodiment, the term "about" or "approximately" means within 20%, preferably within 10%, and more preferably within 5% of a given value or range.

The yield of each of the reactions described herein is expressed as a percentage of the theoretical yield.

### B. Salt, Derivative, and Solvate Terms and Conventions

The term "salt" means an ionic form of the parent compound or the product of the reaction between the parent compound with a suitable acid or base to make the acid salt or base salt of the parent compound. Salts of the compounds of the present invention can be synthesized from the parent compounds which contain a basic or acidic moiety by conventional chemical methods. Generally, the salts are prepared by reacting the free base or acid parent compound with stoichiometric amounts or with an excess of the desired salt-forming inorganic or organic acid or base in a suitable solvent or various combinations of solvents.

The term "pharmaceutically acceptable salt" means a salt of a compound of the invention which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. As the compounds of the present invention are useful in both free base and salt form, in practice, the use of the salt form amounts to use of the base form. Lists of suitable salts are found in, e.g., S.M. Birge et al., J. Pharm. Sci., 1977, 66, pp. 1-19, which is hereby incorporated by reference in its entirety.

The term "pharmaceutically-acceptable acid addition salt" means those salts which retain the biological effectiveness and properties of the free bases and which are not biologically or otherwise undesirable, formed with inorganic acids such as hydrochloric acid, hydrobromic acid, hydroiodic acid; sulfuric acid, sulfamic acid, nitric acid, phosphoric acid, and the like, and organic acids such as acetic acid, trichloroacetic acid, trifluoroacetic acid, adipic acid, alginic acid, ascorbic acid, aspartic acid, benzenesulfonic acid, benzoic acid, 2-acetoxybenzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, heptanoic acid, hexanoic acid, formic acid, fumaric acid, 2-hydroxyethanesulfonic acid (isethionic acid), lactic acid, maleic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, picric acid, pivalic acid, propionic acid, pyruvic acid, pyruvic acid, salicylic acid, stearic acid, succinic acid, sulfanilic acid, tartaric acid, p-toluenesulfonic acid, undecanoic acid, and the like.

The term "pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, *N-*ethylpiperidine, tetramethylammonium compounds, tetraethylammonium compounds, pyridine, *N*,*N-*dimethylaniline, *N-*methylpiperidine, *N-*methylmorpholine, dicyclohexylamine, dibenzylamine, *N*,*N-*dibenzylphenethylamine, 1-ephenamine, *N,N'*-dibenzylethylenediamine, polyamine resins, and the like. Particularly preferred organic nontoxic bases are isopropylamine, diethylamine, ethanolamine, trimethylamine, dicyclohexylamine, choline, and caffeine.

The term "solvate" means a physical association of a compound with one or more solvent molecules or a complex of variable stoichiometry formed by a solute (for example, a compound of Formula (I)) and a solvent, for example, water, ethanol, or acetic acid. This physical association may involve varying degrees of ionic and covalent bonding, including hydrogen bonding. In certain instances, the solvate will be capable of isolation, for example, when one or more solvent molecules are incorporated in the crystal lattice of the crystalline solid. In general, the solvents selected do not interfere with the biological activity of the solute. Solvates encompasses both solution-phase and isolatable solvates. Representative solvates include hydrates, ethanolates, methanolates, and the like.

The term "hydrate" means a solvate wherein the solvent molecule(s) is/are H₂O.

The compounds of the present invention as discussed below include the free base or acid thereof, their salts, solvates, and esters and amides, and may include oxidized sulfur atoms or quatemized nitrogen atoms in their structure, although not explicitly stated or shown, particularly the pharmaceutically acceptable forms thereof. Such forms, particularly the pharmaceutically acceptable forms, are intended to be embraced by the appended claims.

### C. Isomer Terms and Conventions

The term "isomers" means compounds having the same number and kind of atoms, and hence the same molecular weight, but differing with respect to the arrangement or configuration of their atoms in space. The term includes stereoisomers and geometric isomers.

The terms "stereoisomer" or "optical isomer" means a stable isomer that has at least one chiral atom or restricted rotation giving rise to perpendicular dissymmetric planes (e.g., certain biphenyls, allenes, and spiro compounds) and can rotate plane-polarized light. Because asymmetric centers and other chemical structure exist in the compounds of the invention which may give rise to stereoisomerism, the invention contemplates stereoisomers and mixtures thereof. The compounds of the invention and their salts include asymmetric carbon atoms and may therefore exist as single stereoisomers, racemates, and as mixtures of enantiomers and diastereomers. Typically, such compounds will be prepared as a racemic mixture. If desired, however, such compounds can be prepared or isolated as pure stereoisomers, i.e., as individual enantiomers or diastereomers, or as stereoisomer-enriched mixtures. As discussed in more detail below, individual stereoisomers of compounds are prepared by synthesis from optically active starting materials containing the desired chiral centers or by preparation of mixtures of enantiomeric products followed by separation or resolution, such as conversion to a mixture of diastereomers followed by separation or recrystallization, chromatographic techniques, use of chiral resolving agents, or direct separation of the enantiomers on chiral chromatographic columns. Starting compounds of particular stereochemistry are either commercially available or are made by the methods described below and resolved by techniques well-known in the art.

The term "enantiomers" means a pair of stereoisomers that are non-superimposable mirror images of each other.

The terms "diastereoisomers" or "diastereomers" mean stereoisomers which are not mirror images of each other.

The terms "racemic mixture" or "racemate" mean a mixture containing equal parts of individual enantiomers.

The term "non-racemic mixture" means a mixture containing unequal parts of individual enantiomers.

The term "geometrical isomer" means a stable isomer which results from restricted freedom of rotation about double bonds (e.g., *cis*-2-butene and *trans*-2-butene) or in a cyclic structure (e.g., *cis*-1,3-dichlorocyclobutane and *trans*-1,3-dichlorocyclobutane). Because carbon-carbon double (olefinic) bonds, C=N double bonds, cyclic structures, and the like may be present in the compounds of the invention, the invention contemplates each of the various stable geometric isomers and mixtures thereof resulting from the arrangement of substituents around these double bonds and in these cyclic structures. The substituents and the isomers are designated using the *cis*/*trans* convention or using the *E* or *Z* system, wherein the term *"E"* means higher order substituents on opposite sides of the double bond, and the term *"Z"* means higher order substituents on the same side of the double bond. A thorough discussion of *E* and *Z* isomerism is provided in J. March, Advanced Organic Chemistry: Reactions, Mechanisms, and Structure, 4th ed., John Wiley & Sons, 1992, which is hereby incorporated by reference in its entirety. Several of the following examples represent single *E* isomers, single *Z* isomers, and mixtures of *E*/*Z* isomers. Determination of the *E* and *Z* isomers can be done by analytical methods such as x-ray crystallography, ¹H NMR, and ¹³C NMR.

Some of the compounds of the invention can exist in more than one tautomeric form. As mentioned above, the compounds of the invention include all such tautomers.

It is well-known in the art that the biological and pharmacological activity of a compound is sensitive to the stereochemistry of the compound. Thus, for example, enantiomers often exhibit strikingly different biological activity including differences in pharmacokinetic properties, including metabolism, protein binding, and the like, and pharmacological properties, including the type of activity displayed, the degree of activity, toxicity, and the like. Thus, one skilled in the art will appreciate that one enantiomer may be more active or may exhibit beneficial effects when enriched relative to the other enantiomer or when separated from the other enantiomer. Additionally, one skilled in the art would know how to separate, enrich, or selectively prepare the enantiomers of the compounds of the invention from this disclosure and the knowledge of the prior art.

Thus, although the racemic form of drug may be used, it is often less effective than administering an equal amount of enantiomerically pure drug; indeed, in some cases, one enantiomer may be pharmacologically inactive and would merely serve as a simple diluent. For example, although ibuprofen had been previously administered as a racemate, it has been shown that only the *S*-isomer of ibuprofen is effective as an anti-inflammatory agent (in the case of ibuprofen, however, although the *R*-isomer is inactive, it is converted *in vivo* to the *S-*isomer, thus, the rapidity of action of the racemic form of the drug is less than that of the pure *S*-isomer). Furthermore, the pharmacological activities of enantiomers may have distinct biological activity. For example, S-penicillamine is a therapeutic agent for chronic arthritis, while *R-*penicillamine is toxic. Indeed, some purified enantiomers have advantages over the racemates, as it has been reported that purified individual isomers have faster transdermal penetration rates compared to the racemic mixture. See U.S. Pat. Nos. 5,114,946 and 4,818,541.

Thus, if one enantiomer is pharmacologically more active, less toxic, or has a preferred disposition in the body than the other enantiomer, it would be therapeutically more beneficial to administer that enantiomer preferentially. In this way, the patient undergoing treatment would be exposed to a lower total dose of the drug and to a lower dose of an enantiomer that is possibly toxic or an inhibitor of the other enantiomer.

Preparation of pure enantiomers or mixtures of desired enantiomeric excess (ee) or enantiomeric purity are accomplished by one or more of the many methods of (a) separation or resolution of enantiomers, or (b) enantioselective synthesis known to those of skill in the art, or a combination thereof. These resolution methods generally rely on chiral recognition and include, for example, chromatography using chiral stationary phases, enantioselective host-guest complexation, resolution or synthesis using chiral auxiliaries, enantioselective synthesis, enzymatic and nonenzymatic kinetic resolution, or spontaneous enantioselective crystallization. Such methods are disclosed generally in Chiral Separation Techniques: A Practical Approach (2nd Ed.), G. Subramanian (ed.), Wiley-VCH, 2000; T.E. Beesley and R.P.W. Scott, Chiral Chromatography, John Wiley & Sons, 1999; and Satinder Ahuja, Chiral Separations by Chromatography, Am. Chem. Soc., 2000. Furthermore, there are equally well-known methods for the quantitation of enantiomeric excess or purity, for example, GC, HPLC, CE, or NMR, and assignment of absolute configuration and conformation, for example, CD ORD, X-ray crystallography, or NMR.

In general, all tautomeric forms and isomeric forms and mixtures, whether individual geometric isomers or stereoisomers or racemic or non-racemic mixtures, of a chemical structure or compound is intended, unless the specific stereochemistry or isomeric form is specifically indicated in the compound name or structure.

### D. Pharmaceutical Administration and Diagnostic and Treatment Terms and Conventions

The term "patient" includes both human and non-human mammals.

The term "effective amount" means an amount of a compound according to the invention which, in the context of which it is administered or used, is sufficient to achieve the desired effect or result. Depending on the context, the term effective amount may include or be synonymous with a pharmaceutically effective amount or a diagnostically effective amount.

The terms "pharmaceutically effective amount" or "therapeutically effective amount" means an amount of a compound according to the invention which, when administered to a patient in need thereof, is sufficient to effect treatment for disease-states, conditions, or disorders for which the compounds have utility. Such an amount would be sufficient to elicit the biological or medical response of a tissue, system, or patient that is sought by a researcher or clinician. The amount of a compound of according to the invention which constitutes a therapeutically effective amount will vary depending on such factors as the compound and its biological activity, the composition used for administration, the time of administration, the route of administration, the rate of excretion of the compound, the duration of treatment, the type of disease-state or disorder being treated and its severity, drugs used in combination with or coincidentally with the compounds of the invention, and the age, body weight, general health, sex, and diet of the patient. Such a therapeutically effective amount can be determined routinely by one of ordinary skill in the art having regard to their own knowledge, the prior art, and this disclosure.

The term "diagnostically effective amount" means an amount of a compound according to the invention which, when used in a diagnostic method, apparatus, or assay, is sufficient to achieve the desired diagnostic effect or the desired biological activity necessary for the diagnostic method, apparatus, or assay. Such an amount would be sufficient to elicit the biological or medical response in a diagnostic method, apparatus, or assay, which may include a biological or medical response in a patient or in a *in vitro* or *in vivo* tissue or system, that is sought by a " researcher or clinician. The amount of a compound according to the invention which constitutes a diagnostically effective amount will vary depending on such factors as the compound and its biological activity, the diagnostic method, apparatus, or assay used, the composition used for administration, the time of administration, the route of administration, the rate of excretion of the compound, the duration of administration, drugs and other compounds used in combination with or coincidentally with the compounds of the invention, and, if a patient is the subject of the diagnostic administration, the age, body weight, general health, sex, and diet of the patient. Such a diagnostically effective amount can be determined routinely by one of ordinary skill in the art having regard to their own knowledge, the prior art, and this disclosure.

The term "modulate" means the ability of a compound to alter the function of the glucocorticoid receptor by, for example, binding to and stimulating or inhibiting the glucocorticoid receptor functional responses.

The term "modulator" in the context of describing compounds according to the invention means a compound that modulates the glucocorticoid receptor function. As such, modulators include, but are not limited to, agonists, partial agonists, antagonists, and partial antagonists.

The term "agonist" in the context of describing compounds according to the invention means a compound that, when bound to the glucocorticoid receptor, enhances or increases the glucocorticoid receptor function. As such, agonists include partial agonists and full agonists.

The term "full agonist" in the context of describing compounds according to the invention means a compound that evokes the maximal stimulatory response from the glucocorticoid receptor, even when there are spare (unoccupied) glucocorticoid receptors present.

The term "partial agonist" in the context of describing compounds according to the invention means a compound that is unable to evoke the maximal stimulatory response from the glucocorticoid receptor, even at concentrations sufficient to saturate the glucocorticoid receptors present.

The term "antagonist" in the context of describing compounds according to the invention means a compound that directly or indirectly inhibits or suppresses the glucocorticoid receptor function. As such, antagonists include partial antagonists and full antagonists.

The term "full antagonist" in the context of describing compounds according to the invention means a compound that evokes the maximal inhibitory response from the glucocorticoid receptor, even when there are spare (unoccupied) glucocorticoid receptors present.

The term "partial antagonist" in the context of describing compounds according to the invention means a compound that is unable to evoke the maximal inhibitory response from the glucocorticoid receptor, even at concentrations sufficient to saturate the glucocorticoid receptors present.

The terms "treating" or "treatment" mean the treatment of a disease-state in a patient, and include:
(i) preventing the disease-state from occurring in a patient, in particular, when such patient is genetically or otherwise predisposed to the disease-state but has not yet been diagnosed as having it;
(ii) inhibiting or ameliorating the disease-state in a patient, i.e., arresting or slowing its development; or
(iii) relieving the disease-state in a patient, i.e., causing regression or cure of the disease-state.

### Detailed Description of the Invention

### General Synthetic Methods for Making Compounds of Formula (I)

The invention also provides processes for making compounds of Formula (I). In all schemes, unless specified otherwise, R¹, R², R³, R⁴, R⁵, R⁶, R⁷, R⁸, R⁹, R¹⁰, X, and Y in the formulas below shall have the meaning of R¹, R², R³, R⁴, R⁵, R⁶, R⁷, R⁸, R⁹, R¹⁰, X, and Y in the Formula (I) of the invention described hereinabove. Intermediates used in the preparation of compounds of the invention are either commercially available or readily prepared by methods known to those skilled in the art.

Optimum reaction conditions and reaction times may vary depending on the particular reactants used. Unless otherwise specified, solvents, temperatures, pressures, and other reaction conditions may be readily selected by one of ordinary skill in the art. Specific procedures are provided in the Synthetic Examples section. Typically, reaction progress may be monitored by thin layer chromatography (TLC), if desired, and intermediates and products may be purified by chromatography on silica gel and/or by recrystallization.

Compounds of Formula (I) may be prepared by the method outlined in Scheme I.

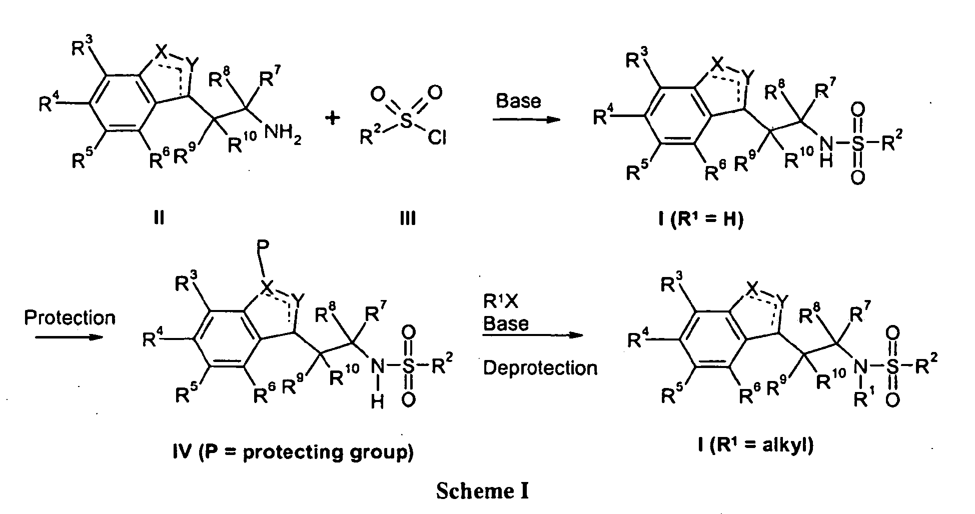

As illustrated in Scheme I, the optionally substituted aminoethyl heterocycle (II) is reacted with a sulfonyl halide, preferably a sulfonyl chloride, in the presence of a suitable base, such as triethylamine, to produce the desired compound of formula (I) where R¹ is H. If one desires an alkyl group at R¹, one would protect any active hydrogen if present (if X or Y is NH). An example of a suitable protecting group is the *tert*-butoxycarbonyl (*t-*Boc) group which may be attached by reaction of a compound of Formula (I) where R¹ is H with di-*tert*-butyldicarbonate in the presence of a base such as 4-dimethylaminopyridine. One then reacts the protected intermediate (IV) with an alkyl halide R¹X, where X is a halogen, preferably I or Br, in the presence of a base, such as potassium carbonate. Deprotection, for example, by treating with trifluoroacetic acid, or with heat for a *t-*Boc protecting group, provides the desired compound of Formula (I) where R¹ is alkyl or substituted alkyl.

The sulfonyl chlorides R²SO₂Cl (III) are commercially available or may be readily prepared by methods known to those skilled in the art. Intermediates (II) may also be commercially available or prepared by methods known in the art. For example, as illustrated in Scheme II, intermediates of Formula (II) may be prepared from the aldehyde (V).

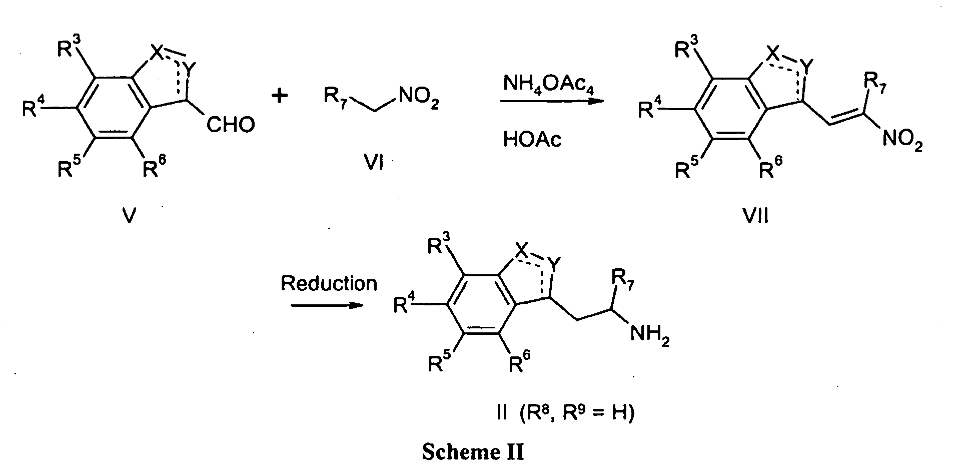

As illustrated in Scheme II, aldehyde (II) is reacted with nitro compound (VI) in the presence of ammonium acetate and acetic acid to provide nitroalkene (VII). Reduction of the alkene and nitro group, for example, with borane/THF and trimethylsilyl chloride provides the intermediate (II) where R⁸, R⁹, and R¹⁰ are each H.

A method by which compounds of Formula (I) where R⁹ and/or R¹⁰ are alkyl groups may be prepared is illustrated in Scheme III.

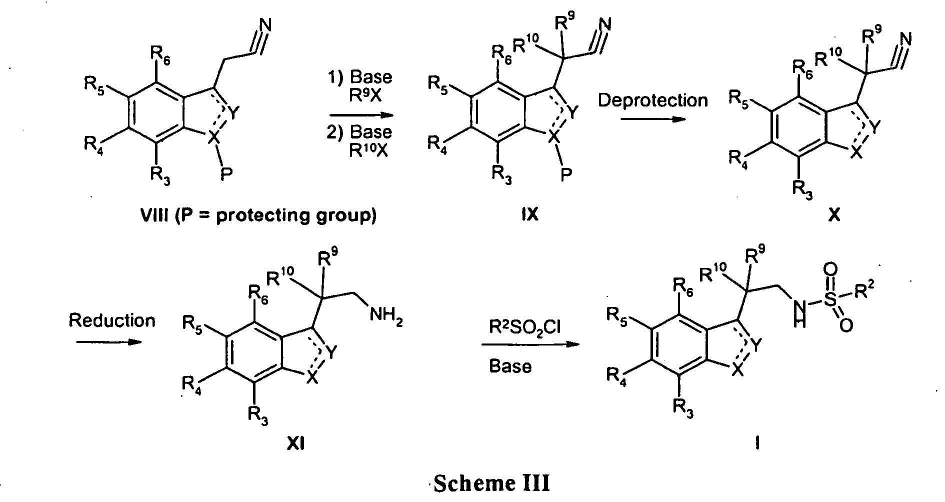

As illustrated in Scheme III, an optionally substituted cyanomethylheterocycle (VIII) is treated with a base such as sodium hydride in a suitable solvent such as DMSO or THF. If X or Y is NH, it would be protected with a suitable protecting group P as shown in VIII. An example of a suitable protecting group is the *tert*-butoxycarbonyl (*t*-Boc) group. Treatment with base is followed by addition of R⁹X, where X is a halogen. If one desires a compound with R¹⁰ also being an alkyl group, one would repeat the process with a base and R¹⁰X. If one desires R⁹ and R¹⁰ to be the same alkyl group, one may add both alkyl groups at the same time by treating the cyanomethylheterocycle (VIII) with at least two equivalents of base and alkyl halide. Removal of the protecting group, for example, by treatment of the compound of Formula (IX) with acid if P is a *t-*Boc group, then provides the compound of Formula (X). Reduction of the cyano group in the compound of Formula (X), by treating the compound of Formula (X) with a reducing agent such as lithium aluminum hydride provides the compound of Formula (XI). Reaction of the compound of Formula (XI) with a sulfonyl halide in the presence of a base, as described for intermediate II in Scheme I, provides the desired compound of Formula (I).

In order that this invention be more fully understood, the following examples are set forth. These examples are for the purpose of illustrating embodiments of this invention, and are not to be construed as limiting the scope of the invention in any way since, as recognized by one skilled in the art, particular reagents or conditions could be modified as needed for individual compounds. Starting materials used are either commercially available or easily prepared from commercially available materials by those skilled in the art.

### Experimental Examples

### Example 1: Synthesis of N-[2-(1H-Indol-3-yl)-1-methylethyl]-2,4,6-trimethyl-benzenesulfonamide

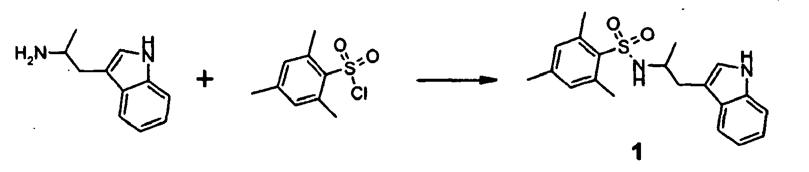

α-Methyl tryptamine (34.9 mg, 0.2 mmol) was placed in a 20 mL scintillation vial and 4 mL of CH₂Cl₂ was added. To this was added mesitylenesulfonyl chloride (43.7 mg, 0.2 mmol) and triethylamine (0.042 mL, 0.3 mmol). The homogeneous mixture was stirred for 14 hours. The reaction mixture was placed directly onto a CH₂Cl₂ pre-washed NH₂/CBA-layered solid-phase extraction cartridge (1 g sorbent each, Varian Bond-elut) and pulled through by vacuum filtration. The cartridge was washed with two 3 mL portions of CH₂Cl₂. The filtrate was concentrated *in vacuo* and the residue purified by Prep-plate chromatography (silica gel, 1.0 mm plate, 50% EtOAc/hexane), providing 29.2 mg of the title compound (39% yield) as a white solid.

Resolution of the above racemate into enantiomers was achieved as follows: The racemate (19.0 mg, 0.05 mmol) was dissolved in 8 mL of a 10% isopropanol/hexane solution. 2.0 mL of this solution was injected onto an HPLC (column: Chiracel OD 1.0 mm, flowrate: 5 mL/min). The first eluting enantiomer was collected at 30.8 minutes and the second at 35.2 minutes. This cycle was repeated three more times to give 5.0 of the first eluting enantiomer (53% yield) and 5.5 mg of the second (58% yield).

### Example 2: Synthesis of N-[2-(1H-Indol-3-yl)-1-methylethyl]-2,4,6,N-tetramethylbenzenesulfonamide

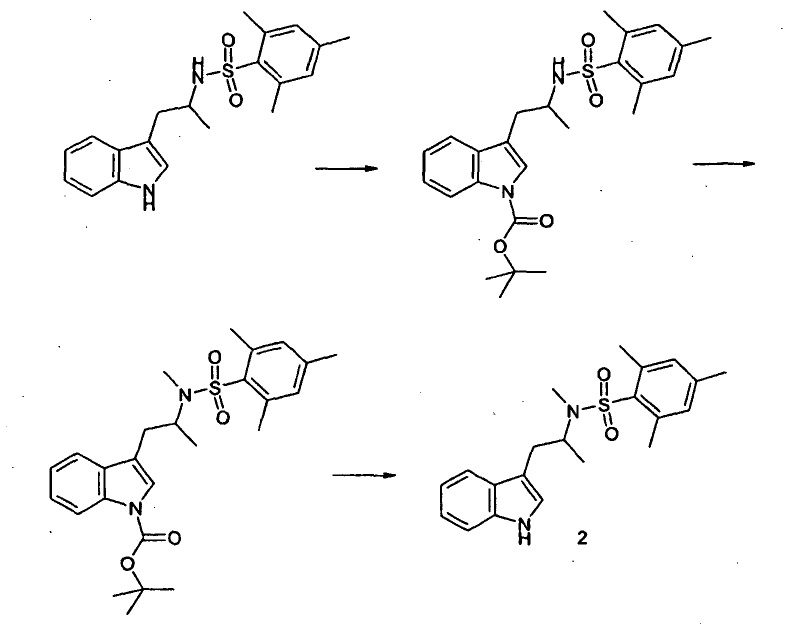

*N-*[2-(1*H-*Indol-3-yl)-1-methylethyl]-2,4,6-trimethylbenzenesulfonamide (400 mg, 1.12 mmol) (Example 1) was dissolved in 15 mL of acetonitrile. To this was added 4-dimethylaminopyridine (14.0 mg, 0.12 mmol) and di-*tert*-butyl dicarbonate (244 mg, 1.12 mmol). The reaction mixture was stirred overnight. The solvent was removed *in vacuo* and residue was dissolved in 20 mL of EtOAc. The organic phase was washed with two 20 mL portions of saturated ammonium chloride (NH₄Cl) solution and 20 mL of brine. The organic phase was dried over magnesium sulfate (MgSO₄) and concentrated *in vacuo* to give 416 mg of 3-[2-(2,4,6-trimethylbenzenesulfonylamino)propyl]indole-1-carboxylic acid *tert*-butyl ester as an off-white solid (81% yield). ¹H NMR and LC-MS analysis indicated material of sufficient purity to carry on.

3-[2-(2,4,6-Trimethylbenzenesulfonylamino)propyl]indole-1-carboxylic acid *tert*-butyl ester (200 mg, 0.44 mmol) was placed in a 5 mL round-bottom flask and dissolved in 2 mL of DMF. To this was added K₂CO₃ (338 mg, 2.20 mmol) and methyl iodide (0.035 mL, 0.55 mmol). The reaction was heated at 50°C for 16 hours. After cooling, the reaction mixture was diluted with 15 mL of EtOAc. The organic phase was washed with four 10 mL portions of H₂O and one 10 mL portion of brine. The organic phase was dried over magnesium sulfate and concentrated *in vacuo* to give a 201 mg of 3-{2-[methyl-(2,4,6-trimethylbenzenesulfonyl)amino]propyl}indole-1-carboxylic acid *tert*-butyl ester as a viscous oil (97% yield).

3-{2-[Methyl-(2,4,6-trimethylbenzenesulfonyl)amino]propyl}indole-1-carboxylic acid *tert-*butyl ester (100 mg, 0.21 mmol) was placed in a microwave tube and dissolved in dichloroethane. The solution was heated at 180°C for 20 minutes in three subsequent runs. TLC analysis indicated the formation of a more polar spot. The solution was applied directly to a prep-plate and purified (1.0 mm plate, 50% EtOAc/hexane) to give 29.8 mg of the title compound as a white solid (38% yield).

### Example 3: N-[2-(6-Fluoro-1H-indol-3-yl)-1-methylethyl]-2,4,6-trimethylbenzenesulfonamide

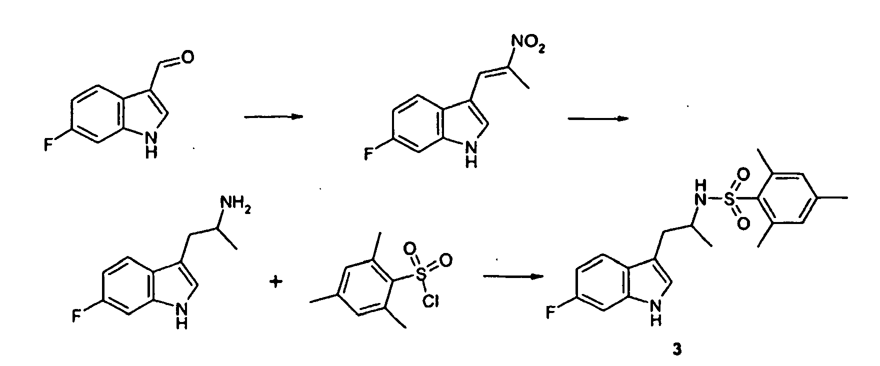

6-Fluoroindole-3-carboxaldehyde (206.8 mg, 1.27 mmol), ammonium acetate (245.0 mg, 3.18 mmol), and nitroethane (0.271 mL, 5.08 mmol) were placed in a scintillation vial. Acetic acid (0.21 mL) was added and the vial was heated with a heat gun until a homogeneous solution developed. The reaction vessel was placed in a sonicating bath set on maximum. After 10 minutes, the reaction became heterogenous. The vial was heated as previously described. This cycle was repeated until homogeneity persisted. The reaction vessel was sonicated for 14 hours. The reaction contents were diluted with 10 mL of CH₂Cl₂ and washed with 20 mL of H₂O. The organic phase was passed through a plug of silica and concentrated to give 243 mg of 6-fluoro-3-((*E*)-2-nitropropenyl)-1*H-*indole as a bright red powder (88% yield).

6-Fluoro-3-((*E*)-2-nitropropenyl)-1*H-*indole (101 mg, 0.46 mmol) was placed under nitrogen gas in a 10 mL round-bottom flask and 3 mL of dry THF was added. To this was added trimethylsilyl chloride (0.350 mL, 2.76 mmol) and 1 M BH₃/THF solution (2.76 mL, 2.76 mmol). The reaction stirred for 16 hours. TLC analysis indicated complete consumption of the starting material. MeOH was added dropwise to quench the residual BH₃. When gas evolution ceased, the reaction mixture was concentrated *in vacuo* to an oily residue. 5 mL of MeOH was added and the mixture concentrated *in vacuo* again. This cycle was completed 4 times to provide 2-(6-fluoro-1*H-*indol-3-yl)-1-methylethylamine. ¹H NMR and LC-MS indicated amine product of sufficient purity to carry on.

2-(6-Fluoro-1*H-*indol-3-yl)-1-methylethylamine (77.8 mg, 0.46 mmol) was suspended in 5 mL of CH₂Cl₂. Triethylamine (0.145 mL, 1.04 mmol) was added and the amine was fully dissolved. To this was added mesitylenesulfonyl chloride (135 mg. 0.62 mmol) and the reaction mixture was stirred overnight. The reaction mixture was placed directly onto a CH₂Cl₂ pre-washed NH₂/CBA-layered solid-phase extraction cartridge (1g sorbent each, Varian Bond-elut) and pulled through via vacuum filtration. The cartridge was washed with two 3 mL portions of CH₂Cl₂. The filtrate was concentrated *in vacuo* and the residue purified by Prep-plate chromatography (silica gel, 1.0 mm plate, 50% EtOAc/hexane). Purification yielded 37.9 mg of the title compound (22% yield) as a white solid.

### Example 4: Synthesis of 4-tert-butyl-N-[2-(1H-indol-3-yl)-2-methylpropyl]benzenesulfonamide

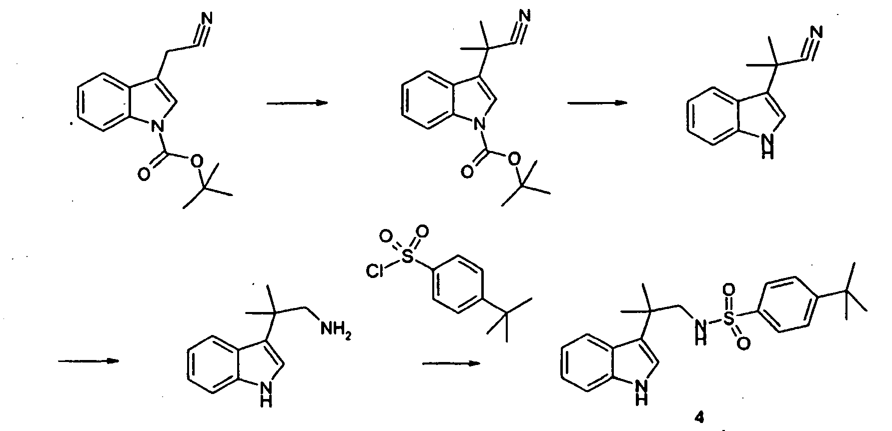

NaH 60% dispersion (600 mg, 15 mmol) was placed in a dry flask under nitrogen. The gray solid was washed with three 20 mL portions of hexanes. To this was added 20 mL of dry DMSO and 20 mL of THF. The flask was then cooled in an ice bath. To this mixture was added 3-cyanomethylindole-1-carboxylic acid-*tert*-butyl ester (2.56 g, 10.0 mL) in 5 mL of THF. The mixture was allowed to warm to room temperature over the period of 1 hour. The flask was then re-cooled and MeI (0.934 mL, 15.0 mmol) was added dropwise. The reaction mixture stirred for 1 hour. TLC analysis indicated complete consumption of starting material. The reaction was quenched with water, diluted with 50 mL of ether, and washed with 50 mL of brine. The organic phase was dried over magnesium sulfate and concentrated *in vacuo.* Chromatography (0-10% ether/hexanes) provided 0.883 g of 3-(cyanodimethylmethyl)indole-1-carboxylic acid-*tert*-butyl ester (31% yield) and 0.341 g of 3-(cyanomethylmethyl)indole-1-carboxylic acid-*tert*-butyl ester (13%).

3-(Cyanodimethylmethyl)indole-1-carboxylic acid-*tert*-butyl ester (0.883 g, 3.12 mmol) was dissolved in 5 mL of EtOAc. To this was added 5 drops of concentrated hydrochloric acid. The reaction stirred for 2 hours, then the solvent was removed *in vacuo* to give 0.517 g of 2-(1*H-*indol.3-yl)-2-methylpropionitrile (75% yield) as a clear oil.

2-(1*H-*Indol-3-yl)-2-methylpropionitrile (0.375g, 2.03 mmol) was dissolved in 10 mL of dry ether and the mixture was cooled in an ice bath. To this was added lithium aluminum hydride (LiAIH₄) (0.310 g, 8.14 mmol) in one portion. The reaction mixture was stirred for I hour. TLC analysis indicated complete consumption of the starting material. The reaction was quenched with 20 mL of saturated ammonium chloride solution and the mixture was diluted with 30 mL of EtOAc. The organic phase was washed with 20 mL of brine. The resulting organic phase was dried over magnesium sulfate and concentrated to give 0.173 g of 2-(1*H-*indol-3-yl)-2-methylpropylamine (46% yield).

2-(1*H-*Indol-3-yl)-2-methylpropylamine .(50 mg, 0.27 mmol), triethylamine (0.096 mL, 0.78 mmol), and 4-*tert*-butylphenylsulfonyl chloride were added to 2 mL of CH₂Cl₂. The reaction was stirred overnight and was placed directly onto a CH₂Cl₂ pre-washed NH₂/CBA-layered solid-phase extraction cartridge (1g sorbent each, Varian Bond-elut) and pulled through via vacuum filtration. The cartridge was washed with two 3 mL portions of CH₂Cl₂. The filtrate was concentrated *in vacuo* and the residue purified by Prep-plate chromatography (50% EtOAc/hexane). Purification yielded 12.2 mg of the title compound (12% yield).

### Assessment of Biological Properties

Compounds of the invention were evaluated for binding to the steroid receptor by a fluorescence polarization competitive binding assay. Detailed descriptions for preparation of recombinant glucocorticoid receptor (GR) complex used in the assay is described in U.S. Patent Application Publication No. US 2003/0017503, filed May 20, 2002, and the corresponding U.S. provisional application No. 60/291,877, filed May 18, 2001, each of which is incorporated herein by reference in its entirety. Preparation of the tetramethyl rhodamine (TAMRA)-labeled dexamethasone probe was accomplished using a standard literature procedure (M. Pons et al., J. Steroid Biochem., 1985, 22, pp. 267-273).

### A. Glucocorticoid Receptor Competitive Binding Assay

### Step 1. Characterization of the Fluorescent Probe

The wavelengths for maximum excitation and emission of the fluorescent probe should first be measured. An example of such a probe is rhodamine (TAMRA)-labeled dexamethasone.

The affinity of the probe for the steroid receptor was then determined in a titration experiment. The fluorescence polarization value of the probe in assay buffer was measured on an SLM-8100 fluorometer using the excitation and emission maximum values described above. Aliquots of expression vector lysate were added and fluorescence polarization was measured after each addition until no further change in polarization value was observed. Non-linear least squares regression analysis was used to calculate the dissociation constant of the probe from the polarization values obtained for lysate binding to the probe.

### Step 2. Screening for Inhibitors of Probe Binding

This assay uses fluorescence polarization (FP) to quantitate the ability of test compounds to compete with tetramethyl rhodamine (TAMRA)-labeled dexamethasone for binding to a human glucocorticoid receptor (GR) complex prepared from an insect expression system. The assay buffer was: 10 mM TES, 50 mM KCI, 20 mM Na₂MoO₄·2H₂O, 1.5 mM EDTA, 0.04% w/v CHAPS, 10% v/v glycerol, 1 mM dithiothreitol, pH 7.4. Test compounds were dissolved to 1 mM in neat DMSO and then further diluted to 10x assay concentration in assay buffer supplemented with 10% v/v DMSO. Test compounds were serially diluted at 10x assay concentrations in 10% DMSO-containing buffer in 96-well polypropylene plates. Binding reaction mixtures were prepared in 96-well black Dynex microtiter plates by sequential addition of the following assay components to each well: 15 µL of 10x test compound solution, 85 µL of GR-containing baculovirus lysate diluted 1:170 in assay buffer, and 50 µL of 15 nM TAMRA-labeled dexamethasone. Positive controls were reaction mixtures containing no test compound; negative controls (blanks) were reaction mixtures containing 0.7 µM to 2 µM dexamethasone. The binding reactions were incubated for I hour at room temperature and then read for fluorescence polarization in the LJL Analyst set to 550 nm excitation and 580 nm emission, with the Rhodamine 561 dichroic mirror installed. IC₅₀ values were determined by iterative non-linear curve fitting of the FP signal data to a 4-parameter logistic equation.

Compounds found to bind to the glucocorticoid receptor may be evaluated for binding to the progesterone receptor (PR), estrogen receptor (ER), and mineralocorticoid receptors to evaluate the compound's selectivity for GR. The protocols for PR and MR are identical to the above GR method, with the following exceptions: PR insect cell lysate is diluted 1:7.1 and MR lysate diluted 1:9.4. PR probe is TAMRA-labeled mifepristone, used at a final concentration of 5 nM in the assay, and the negative controls (blanks) were reactions containing mifepristone at 0.7 µM to 2 µM.

The ER protocol is similar to the above protocols, but uses PanVera kit receptor, fluorescein-labeled probe. The assay components are made in the same volumes as above, to produce final assay concentrations for ER of 15 nM and ES2 probe of 1 nM. In addition, the component order of addition is modified from the above assays: probe is added to the plate first, followed by receptor and test compound. The plates are read in the LJL Analyst set to 485 nm excitation and 530 nm emission, with the Fluorescein 505 dichroic mirror installed.

Compounds found to bind to the glucocorticoid receptor may be evaluated for dissociation of transactivation and transrepression by assays cited in the Background of the Invention (C.M. Bamberger and H.M. Schulte, Eur. J. Clin. Invest, 2000, 30 (suppl. 3) 6-9) or by the assays described below.

### B. Glucocorticoid Receptor Cell Assays

### 1. Induction of Aromatase in Fibroblasts (Cell Assay for Transactivation)

Dexamethasone, a synthetic ligand to the glucocorticoid receptor (GR), induces expression of aromatase in human foreskin fibroblast cells. The activity of aromatase is measured by the conversion of testosterone to estradiol in culture media. Compounds that exhibit binding to GR are evaluated for their ability to induce aromatase activity in human foreskin fibroblasts.

Human foreskin fibroblast cells (ATCC Cat. No. CRL-2429, designation CCD 112SK) are plated on 96 well plates at 50,000 cells per well 5 days before use, in Iscove's Modified Dulbecco's Media (GibcoBRL Life Technologies Cat No. 12440-053) supplemented with 10% charcoal filtered FBS (Clonetech Cat No. SH30068) and Gentamycin (GibcoBRL Life Technologies Cat. No. 15710-064). On the day of the experiment, the media in the wells is replaced with fresh media. Cells are treated with test compounds to final concentrations of 10⁻⁵ M to 10⁻⁸ M, and testosterone to a final concentration of 300 ng/mL. Each well has a total volume of 100 µL. Samples are made in duplicates. Control wells include: (a) wells that receive testosterone only, and (b) wells that receive testosterone plus 2 µM of dexamethasone to provide maximum induction of aromatase. Plates are incubated at 37°C overnight (15 to 18 hours), and supernatants are harvested at the end of incubation. Estradiol in the supernatant is measured using ELISA kits for estradiol (made by ALPCO, obtained from American Laboratory Products Cat. No. 020-DR-2693) according to the manufacture's instruction. The amount of estradiol is inversely proportional to the ELISA signals in each well. The extent of aroinatase induction by test compounds is expressed as a relative percentage to dexamethasone. EC₅₀ values of test compounds are derived by non-linear curve fitting.

### 2. Inhibition of IL-6 Production in Fibroblasts (Cell Assay for Transrepression)

Human foreskin fibroblast cells produce IL-6 in response to stimulation by pro-inflammatory cytokine IL-1. This inflammatory response, as measured by the production of IL-6, can be effectively inhibited by dexamethasone, a synthetic ligand to the glucocorticoid receptor (GR). Compounds that exhibit binding to GR are evaluated for their ability to inhibit IL-6 production in human foreskin fibroblasts.

Human foreskin fibroblast cells (ATCC Cat. No. CRL-2429) are plated on 96 well plates at 5,000 cells per well the day before use, in Iscove's Modified Dulbecco's Media (GibcoBRL Life Technologies Cat. No. 12440-053) supplemented with 10% charcoal filtered FBS (Clonetech Cat. No. SH30068) and Gentamycin (GibcoBRL Life Technologies Cat. No. 15710-064). On the next day, media in the wells is replaced with fresh media. Cells are treated with IL-1 (rhIL-1α, R&D Systems Cat. No. 200-LA) to a final concentration of 1 ng/mL, and with test compounds to final concentrations of 10⁻⁵ M to 10⁻⁸ M, in a total volume of 200 µL per well. Samples are done in duplicates. Background control wells do not receive test compounds or IL-1. Positive control wells receive IL-1 only and represent maximum (or 100%) amount of IL-6 production. Plates are incubated at 37°C overnight (15 to 18 hours), and supernatants are harvested at the end of incubation. IL-6 levels in the supernatants are determined by the ELISA kits for IL-6 (MedSystems Diagnostics GmbH, Vienna, Austria, Cat. No. BMS213TEN) according to manufacture's instructions. The extent of inhibition of IL-6 by test compounds is expressed in percentage relative to positive controls. IC₅₀ values of test compounds are derived by non-linear curve fitting.

Evaluation of agonist or antagonist activity of compounds binding to the glucocorticoid receptor may be determined by any of the assays.

### 3. Modulation of Tyrosine Aminotransferase (TAT) Induction in Rat Hepatoma Cells

Testing of compounds for agonist or antagonist activity in induction of tyrosine aminotransferase (TAT) in rat hepatoma cells.

H4-II-E-C3 cells were incubated overnight in 96 well plates (20,000 cells/100 µL/well) in MEM medium containing 10% heat inactivated FBS and 1% nonessential amino acids. On the next day, cells were stimulated with the indicated concentrations of dexamethasone or test compound (dissolved in DMSO, final DMSO concentration 0.2%) for 18 hours. Control cells were treated with 0.2% DMSO. After 18 hours, the cells were lysed in a buffer containing 0.1% Triton X-100 and the TAT activity was measured in a photometric assay using tyrosine and alpha-ketoglutarate as substrates.

For measuring antagonist activity, the hepatoma cells were pre-stimulated by addition of dexamethasone (concentration ranges from 3 x 10⁻⁹ M to 3 x 10⁻⁸ M) shortly before the test compound was applied to the cells. The steroidal non-selective GR/PR antagonist mifepristone was used as control.

### 4. Modulation of MMTV-Luc Induction in HeLa Cells

Testing of compounds for agonist or antagonist activity in stimulation of MMTV-(mouse mammary tumor virus) promoter in HeLa cells.

HeLa cells were stably co-transfected with the pHHLuc-plasmid containing a fragment of the MMTV-LTR (-200 to +100 relative to the transcription start site) cloned in front of the luciferase gene (Norden, 1988) and the pcDNA3.1 plasmid (Invitrogen) constitutively expressing the resistance for the selective antibiotic GENETICIN®. Clones with best induction of the MMTV-promoter were selected and used for further experiments.

Cells were cultured overnight in DMEM medium without phenol red, supplemented with 3% CCS (charcoal treated calf serum) and then transferred to 96 well plates (15,000 cells/100 µL/well). On the next day, activation of the MMTV-promoter was stimulated by addition of test compound or dexamethasone dissolved in DMSO (final concentration 0.2%). Control cells were treated with DMSO only. After 18 hours, the cells were lysed with cell lysis reagent (Promega, Cat. No. E1531), luciferase assay reagent (Promega, Cat. No. E1501) was added and the glow luminescence was measured using a luminometer (BMG, Offenburg).

For measuring antagonist activity, the MMTV-promoter was pre-stimulated by adding dexamethasone (3 x 10⁻⁹ M to 3 x 10⁻⁸ M) shortly before the test compound was applied to the cells. The steroidal non-selective GR/PR antagonist mifepristone was used as control.

### 5. Modulation of IL-8 Production in U937 Cells

Testing of compounds for agonist or antagonist activity in GR-mediated inhibition of LPS-induced IL-8 secretion in U-937 cells.

U-937 cells were incubated for 2 to 4 days in RPMI1640 medium containing 10% CCS (charcoal treated calf serum). The cells were transferred to 96 well plates (40,000 cells/100 µL/well) and stimulated with 1 µg/mL LPS (dissolved in PBS) in the presence or absence of dexamethasone or test compound (dissolved in DMSO, final concentration 0.2%). Control cells were treated with 0.2% DMSO. After 18 hours, the IL-8 concentration in the cell supernatant was measured by ELISA, using the "OptEIA human IL-8 set" (Pharmingen, Cat. No. 2654KI).

For measuring antagonist activity, the LPS-induced IL-8 secretion was inhibited by adding dexamethasone (3 x 10⁻⁹ M to 3 x 10⁻⁸ M) shortly before the test compound was applied to the cells. The steroidal non-selective GR/PR antagonist mifepristone was used as control.

### 6. Modulation of ICAM-Luc Expression in HeLa Cells

Testing of compounds for agonist or antagonist activity in inhibition of TNF-alpha-induced activation of the ICAM-promoter in HeLa cells.

HeLa cells were stably co-transfected with a plasmid containing a 1.3 kb fragment of the human ICAM-promoter (-1353 to -9 relative to the transcription start site, Ledebur and Parks, 1995) cloned in front of the luciferase gene and the pcDNA3:1 plasmid (Invitrogen) which constitutively expresses the resistance for the antibiotic GENETICIN®. Clones with best induction of the ICAM-promoter were selected and used for further experiments. Cells were transferred to 96 well plates (15,000 cells/100 µL/well) in DMEM medium supplemented with 3% CCS. On the following day the activation of the ICAM-promoter was induced by addition of 10 ng/mL recombinant TNF-alpha (R&D System, Cat. No. 210-TA). Simultaneously the cells were treated with the test compound or dexamethasone (dissolved in DMSO, final concentration 0.2%). Control cells were treated with DMSO only. After 18 hours, the cells were lysed with cell lysis reagent (Promega, Cat. No. E1531), luciferase assay reagent (Promega, Cat. No. EI501) was added and glow luminescence was measured using a luminometer (BMG, Offenburg).

For measuring antagonist activity, the TNF-alpha-induced activation of the ICAM-promoter was inhibited by adding dexamethasone (3 x 10⁻⁹ M to 3 x 10⁻⁸ M) shortly before the test compound was applied to the cells. The steroidal non-selective GR/PR antagonist mifepristone was used as control.

In general, the preferred potency range in the above assays is between 0.1 nM and 10 µM, the more preferred potency range is 0.1 nM to 1 µM, and the most preferred potency range is 0.1 nM to 100 nM.

Representative compounds of the invention have been tested and have shown activity as modulators of the glucocorticoid receptor function in one or more of the above assays. For example, the following compounds of the invention have demonstrated potent activity (100 nM or less) in the GR binding assay:
*N-*[2-(1*H-*Indol-3-yl)-1-methylethyl]-2,4,6-trimethylbenzenesulfonamide;
2,4,6-Trichloro-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide;
4-Bromo-2,5-dichlorothiophene-3-sulfonic acid [2-(1*H-*indol-3-yl)-1-methylethyl]amide;
2,4,6-Trichloro-*N-*[1-(1*H-*indol-3-ylmethyl)propyl]benzenesulfonamide;
*N-*[2-(5-Fluoro-1*H-*indol-3-yl)-1-methylethyl]-2,4,6-trimethylbenzenesulfonamide;
*N-*[1-(1*H-*Indol-3-ylmethyl)propyl]-2,4,6-trimethylbenzenesulfonamide; and
*N-*[2-(6-Fluoro-1*H-*indol-3-yl)-1-methylethyl]-2,4,6-trimethylbenzenesulfonamide.

In addition, the following compound of the invention has been tested and has shown activity as an agonist of the glucocorticoid receptor function in one or more of the above assays:
*N-*[2-(1*H-*Indol-3-yl)-1-methylethyl]-2,4,6-trimethylbenzenesulfonamide.

Furthermore, the following compounds of the invention have been tested and have shown activity as antagonists of the glucocorticoid receptor function in one or more of the above assays:
2-Chloro-*N-*[2-(1*H-*indol-3-yl)-1-methylethyl]-6-methylbenzenesulfonamide;
2,6-Dichloro-*N-*[2-(1*H-*indol-3-yl)-1-methylethyl]benzenesulfonamide;
2,4,6-Trichloro-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide; and
4-Bromo-2,5-dichlorothiophene-3-sulfonic acid [2-(1*H-*indol-3-yl)-1-methylethyl]amide.

The invention also provides methods of modulating the glucocorticoid receptor function in a patient comprising administering to the patient a compound according to the invention. If the purpose of modulating the glucocorticoid receptor function in a patient is to treat a disease-state or condition, the administration preferably comprises a therapeutically or pharmaceutically effective amount of a pharmaceutically acceptable compound according to the invention. If the purpose of modulating the glucocorticoid receptor function in a patient is for a diagnostic or other purpose (e.g., to determine the patient's suitability for therapy or sensitivity to various sub-therapeutic doses of the compounds according to the invention), the administration preferably comprises an effective amount of a compound according to the invention, that is, the amount necessary to obtain the desired effect or degree of modulation.

### Methods of Therapeutic Use

As pointed out above, the compounds of the invention are useful in modulating the glucocorticoid receptor function. In doing so, these compounds have therapeutic use in treating disease-states and conditions mediated by the glucocorticoid receptor function or that would benefit from modulation of the glucocorticoid receptor function.

As the compounds of the invention modulate the glucocorticoid receptor function, they have very useful anti-inflammatory and antiallergic, immune-suppressive, and anti-proliferative activity and they can be used in patients as drugs, particularly in the form of pharmaceutical compositions as set forth below, for the treatment of disease-states and conditions.

The agonist compounds according to the invention can be used in patients as drugs for the treatment of the following disease-states or indications that are accompanied by inflammatory, allergic, and/or proliferative processes:
(i) Lung diseases: chronic, obstructive lung diseases of any genesis, particularly bronchial asthma and chronic obstructive pulmonary disease (COPD); adult respiratory distress syndrome (ARDS); bronchiectasis; bronchitis of various genesis; all forms of restrictive lung diseases, particularly allergic alveolitis; all forms of lung edema, particularly toxic lung edema; all forms of interstitial lung diseases of any genesis, e.g., radiation pneumonitis; and sarcoidosis and granulomatoses, particularly Boeck disease;
(ii) Rheumatic diseases or autoimmune diseases or joint diseases: all forms of rheumatic diseases, especially rheumatoid arthritis, acute rheumatic fever, and polymyalgia rheumatica; reactive arthritis; rheumatic soft tissue diseases; inflammatory soft tissue diseases of other genesis; arthritic symptoms in degenerative joint diseases (arthroses); traumatic arthritis; collagenoses of any genesis, e.g., systemic lupus erythematosus, scleroderma, polymyositis, dermatomyositis, Sjögren syndrome, Still disease, and Felty syndrome;
(iii) Allergic diseases: all forms of allergic reactions, e.g., angioneurotic edema, hay fever, insect bites, allergic reactions to drugs, blood derivatives, contrast agents, etc., anaphylactic shock (anaphylaxis), urticaria, angioneurotic edema, and contact dermatitis;
(iv) Vasculitis diseases: panarteritis nodosa, polyarteritis nodosa, arteritis temporalis, Wegner granulomatosis, giant cell arthritis, and erythema nodosum;
(v) Dermatological diseases: atopic dermatitis, particularly in children; psoriasis; pityriasis rubra pilaris; erythematous diseases triggered by various noxa, e.g., rays, chemicals, burns, etc.; bullous dermatoses; diseases of the lichenoid complex; pruritus (e.g., of allergic genesis); seborrheic dermatitis; rosacea; pemphigus vulgaris; erythema multiforme exudativum; balanitis; vulvitis; hair loss, such as occurs in alopecia areata; and cutaneous T cell lymphomas;
(vi) Renal diseases: nephrotic syndrome; and all types of nephritis, e.g., glomerulonephritis;
(vii) Hepatic diseases: acute liver cell disintegration; acute hepatitis of various genesis, e.g., viral, toxic, drug-induced; and chronically aggressive and/or chronically intermittent hepatitis;
(viii) Gastrointestinal diseases: inflammatory bowel diseases, e.g., regional enteritis (Crohn disease), colitis ulcerosa; gastritis; peptic esophagitis (refluxoesophagitis); and gastroenteritis of other genesis, e.g., nontropical sprue;
(ix) Proctological diseases: anal eczema; fissures; hemorrhoids; and idiopathic proctitis;
(x) Eye diseases: allergic keratitis, uveitis, or iritis; conjunctivitis; blepharitis; neuritis nervi optici; choroiditis; and sympathetic ophthalmia;
(xi) Diseases of the ear, nose, and throat (ENT) area: allergic rhinitis or hay fever; otitis externa, e.g., caused by contact eczema, infection, etc.; and otitis media;
(xii) Neurological diseases: brain edema, particularly tumor-related brain edema; multiple sclerosis; acute encephalomyelitis; meningitis; acute spinal cord injury; stroke; and various forms of seizures, e.g., nodding spasms;
(xiii) Blood diseases: acquired hemolytic anemia; and idiopathic thrombocytopenia;
(xiv) Tumor diseases: acute lymphatic leukemia; malignant lymphoma; lymphogranulomatoses; lymphosarcoma; extensive metastases, particularly in mammary, bronchial, and prostatic carcinoma;
(xv) Endocrine diseases: endocrine ophthalmopathy; endocrine orbitopathia; thyrotoxic crisis; Thyroiditis de Quervain; Hashimoto thyroiditis; Morbus Basedow; granulomatous thyroiditis; struma lymphomatosa; and Grave disease;
(xvi) Organ and tissue transplantations and graft-versus-host diseases;
(xvii) Severe states of shock, e.g., septic shock, anaphylactic shock, and systemic inflammatory response syndrome (SIRS);
(xviii) Substitution therapy in: congenital primary adrenal insufficiency, e.g., adrenogenital syndrome; acquired primary adrenal insufficiency, e.g., Addison disease, autoimmune adrenalitis, post-infection, tumors, metastases, etc.; congenital secondary adrenal insufficiency, e.g., congenital hypopituitarism; and acquired secondary adrenal insufficiency, e.g., post-infection, tumors, metastases, etc.;
(xix) Pain of inflammatory genesis, e.g., lumbago; and
(xx) various other disease-states or conditions including type I diabetes (insulin-dependent diabetes), osteoarthritis, Guillain-Barre syndrome, restenosis following percutaneous transluminal coronary angioplasty, Alzheimer disease, acute and chronic pain, atherosclerosis, reperfusion injury, bone resorption diseases, congestive heart failure, myocardial infarction, thermal injury, multiple organ injury secondary to trauma, acute purulent meningitis, necrotizing enterocolitis and syndromes associated with hemodialysis, leukopheresis, and granulocyte transfusion.

In addition, the compounds according to the invention can be used for the treatment of any other disease-states or conditions not mentioned above which have been treated, are treated, or will be treated with synthetic glucocorticoids (see, e.g., H.J. Hatz, Glucocorticoide: Immunologische Grundlagen, Pharmakologie und Therapierichtlinien [Glucocorticoids: Immunological Fundamentals, Pharmacology, and Therapeutic Guidelines], Stuttgart: Verlagsgesellschaft mbH, 1998, which is hereby incorporated by reference in its entirety). Most or all of the indications (i) through (xx) mentioned above are described in detail in H.J. Hatz, Glucocorticoide: Immunologische Grundlagen, Pharmakologie und Therapierichtlinien. Furthermore, the compounds of the invention can also be used to treat disorders other than those listed above or mentioned or discussed herein, including in the Background of the Invention.

The antagonist compounds according to the invention, whether full antagonists or partial antagonists, can be used in patients as drugs for the treatment of the following disease-states or indications, without limitation: type II diabetes (non-insulin-dependent diabetes); obesity; cardiovascular diseases; hypertension; arteriosclerosis; neurological diseases, such as psychosis and depression; adrenal and pituitary tumors; glaucoma; and Cushing syndrome based on an ACTH-secreting tumor like pituitary adenoma. In particular, the compounds of the invention are useful for treating obesity and all disease-states and indications related to a deregulated fatty acids metabolism such as hypertension, atherosclerosis, and other cardiovascular diseases. Using the compounds of the invention that are GR antagonists, it should be possible to antagonize both the carbohydrate metabolism and fatty acids metabolism. Thus, the antagonist compounds of the invention are useful in treating all disease-states and conditions that involve increased carbohydrate, protein, and lipid metabolism and would include disease-states and conditions leading to catabolism like muscle frailty (as an example of protein metabolism).

### Methods of Diagnostic Use

The compounds of the invention may also be used in diagnostic applications and for commercial and other purposes as standards in competitive binding assays. In such uses, the compounds of the invention may be used in the form of the compounds themselves or they may be modified by attaching a radioisotope, luminescence, fluorescent label or the like in order to obtain a radioisotope, luminescence, or fluorescent probe, as would be known by one of skill in the art and as outlined in Handbook of Fluorescent Probes and Research Chemicals, 6th Edition, R.P. Haugland (ed.), Eugene: Molecular Probes, 1996; Fluorescence and Luminescence Probes for Biological Activity, W.T. Mason (ed.), San Diego: Academic Press, 1993; Receptor-Ligand Interaction, A Practical Approach, E.C. Hulme (ed.), Oxford: IRL Press, 1992, each of which is hereby incorporated by reference in their entireties.

### General Administration and Pharmaceutical Compositions

When used as pharmaceuticals, the compounds of the invention are typically administered in the form of a pharmaceutical composition. Such compositions can be prepared using procedures well known in the pharmaceutical art and comprise at least one compound of the invention. The compounds of the invention may also be administered alone or in combination with adjuvants that enhance stability of the compounds of the invention, facilitate administration of pharmaceutical compositions containing them in certain embodiments, provide increased dissolution or dispersion, increased inhibitory activity, provide adjunct therapy, and the like. The compounds according to the invention may be used on their own or in conjunction with other active substances according to the invention, optionally also in conjunction with other pharmacologically active substances. In general, the compounds of this invention are administered in a therapeutically or pharmaceutically effective amount, but may be administered in lower amounts for diagnostic or other purposes.

In particular, the compounds of the invention are useful in combination with glucocorticoids or corticosteroids. As pointed out above, standard therapy for a variety of immune and inflammatory disorders includes administration of corticosteroids, which have the ability to suppress immunologic and inflammatory responses. (A.P. Truhan et al., Annals of Allergy, 1989, 62, pp. 375-391; J.D. Baxter, Hospital Practice, 1992, 27, pp. 111-134; R.P. Kimberly, Curr. Opin. Rheumatol., 1992, 4, pp. 325-331; M.H. Weisman, Curr. Opin. Rheumatol., 1995, 7, pp. 183-190; W. Sterry, Arch. Dermatol. Res., 1992, 284 (Suppl.), pp. S27-S29). While therapeutically beneficial, however, the use of corticosteroids is associated with a number of side effects, ranging from mild to possibly life threatening, especially with prolonged and/or high dose steroid usage. Accordingly, methods and compositions that enable the use of a lower effective dosage of corticosteroids (referred to as the "steroid sparing effect") would be highly desirable to avoid unwanted side effects. The compounds of the invention provide such a steroid sparing effect by achieving the desired therapeutic effect while allowing the use of lower doses and less frequent administration of glucocorticoids or corticosteroids.

Administration of the compounds of the invention, in pure form or in an appropriate pharmaceutical composition, can be carried out using any of the accepted modes of administration of pharmaceutical compositions. Thus, administration can be, for example, orally, buccally (e.g., sublingually), nasally, parenterally, topically, transdermally, vaginally, or rectally, in the form of solid, semi-solid, lyophilized powder, or liquid dosage forms, such as, for example, tablets, suppositories, pills, soft elastic and hard gelatin capsules, powders, solutions, suspensions, or aerosols, or the like, preferably in unit dosage forms suitable for simple administration of precise dosages. The pharmaceutical compositions will generally include a conventional pharmaceutical carrier or excipient and a compound of the invention as the/an active agent, and, in addition, may include other medicinal agents, pharmaceutical agents, carriers, adjuvants, diluents, vehicles, or combinations thereof. Such pharmaceutically acceptable excipients, carriers, or additives as well as methods of making pharmaceutical compositions for various modes or administration are well-known to those of skill in the art.

The state of the art is evidenced, e.g., by Remington: The Science and Practice of Pharmacy, 20th Edition, A. Gennaro (ed.), Lippincott Williams & Wilkins, 2000; Handbook of Pharmaceutical Additives, Michael & Irene Ash (eds.), Gower, 1995; Handbook of Pharmaceutical Excipients, A.H. Kibbe (ed.), American Pharmaceutical Ass'n, 2000; H.C. Ansel and N.G. Popovish, Pharmaceutical Dosage Forms and Drug Delivery Systems, 5th ed., Lea and Febiger, 1990; each of which is incorporated herein by reference in their entireties to better describe the state of the art.

As one of skill in the art would expect, the forms of the compounds of the invention utilized in a particular pharmaceutical formulation will be selected (e.g., salts) that possess suitable physical characteristics (e.g., water solubility) that is required for the formulation to be efficacious.

Pharmaceutical compositions suitable for buccal (sub-lingual) administration include lozenges comprising a compound of the present invention in a flavored base, usually sucrose, and acacia or tragacanth, and pastilles comprising the compound in an inert base such as gelatin and glycerin or sucrose and acacia.

Pharmaceutical compositions suitable for parenteral administration comprise sterile aqueous preparations of a compound of the present invention. These preparations are preferably administered intravenously, although administration can also be effected by means of subcutaneous, intramuscular, or intradermal injection. Injectable pharmaceutical formulations are commonly based upon injectable sterile saline, phosphate-buffered saline, oleaginous suspensions, or other injectable carriers known in the art and are generally rendered sterile and isotonic with the blood. The injectable pharmaceutical formulations may therefore be provided as a sterile injectable solution or suspension in a nontoxic parenterally acceptable diluent or solvent, including 1,3-butanediol, water, Ringer's solution, isotonic sodium chloride solution, fixed oils such as synthetic mono- or diglycerides, fatty acids such as oleic acid, and the like. Such injectable pharmaceutical formulations are formulated according to the known art using suitable dispersing or setting agents and suspending agents. Injectable compositions will generally contain from 0.1 to 5% w/w of a compound of the invention.

Solid dosage forms for oral administration of the compounds include capsules, tablets, pills, powders, and granules. For such oral administration, a pharmaceutically acceptable composition containing a compound(s) of the invention is formed by the incorporation of any of the normally employed excipients, such as, for example, pharmaceutical grades of mannitol, lactose, starch, pregelatinized starch, magnesium stearate, sodium saccharine, talcum, cellulose ether derivatives, glucose, gelatin, sucrose, citrate, propyl gallate, and the like. Such solid pharmaceutical formulations may include formulations, as are well known in the art, to provide prolonged or sustained delivery of the drug to the gastrointestinal tract by any number of mechanisms, which include, but are not limited to, pH sensitive release from the dosage form based on the changing pH of the small intestine, slow erosion of a tablet or capsule, retention in the stomach based on the physical properties of the formulation, bioadhesion of the dosage form to the mucosal lining of the intestinal tract, or enzymatic release of the active drug from the dosage form.

Liquid dosage forms for oral administration of the compounds include emulsions, microemulsions, solutions, suspensions, syrups, and elixirs, optionally containing pharmaceutical adjuvants in a carrier, such as, for example, water, saline, aqueous dextrose, glycerol, ethanol and the like. These compositions can also contain additional adjuvants such as wetting, emulsifying, suspending, sweetening, flavoring, and perfuming agents.

Topical dosage forms of the compounds include ointments, pastes, creams, lotions, gels, powders, solutions, sprays, inhalants, eye ointments, eye or ear drops, impregnated dressings and aerosols, and may contain appropriate conventional additives such as preservatives, solvents to assist drug penetration and emollients in ointments and creams. Topical application may be once or more than once per day depending upon the usual medical considerations. Furthermore, preferred compounds for the present invention can be administered in intranasal form via topical use of suitable intranasal vehicles. The formulations may also contain compatible conventional carriers, such as cream or ointment bases and ethanol or oleyl alcohol for lotions. Such carriers may be present as from about 1% up to about 98% of the formulation, more usually they will form up to about 80% of the formulation.

Transdermal administration is also possible. Pharmaceutical compositions suitable for transdermal administration can be presented as discrete patches adapted to remain in intimate contact with the epidermis of the recipient for a prolonged period of time. To be administered in the form of a transdermal delivery system, the dosage administration will, of course, be continuous rather than intermittent throughout the dosage regimen. Such patches suitably contain a compound of the invention in an optionally buffered, aqueous solution, dissolved and/or dispersed in an adhesive, or dispersed in a polymer. A suitable concentration of the active compound is about 1% to 35%, preferably about 3% to 15%.

For administration by inhalation, the compounds of the invention are conveniently delivered in the form of an aerosol spray from a pump spray device not requiring a propellant gas or from a pressurized pack or a nebulizer with the use of a suitable propellant, e.g., dichlorodifluoromethane, trichlorofluoromethane, dichlorotetrafluoroethane, tetrafluoroethane, heptafluoropropane, carbon dioxide, or other suitable gas. In any case, the aerosol spray dosage unit may be determined by providing a valve to deliver a metered amount so that the resulting metered dose inhaler (MDI) is used to administer the compounds of the invention in a reproducible and controlled way. Such inhaler, nebulizer, or atomizer devices are known in the art, for example, in PCT International Publication Nos. WO 97/12687 (particularly Figure 6 thereof, which is the basis for the commercial RESPIMAT® nebulizer); WO 94/07607; WO 97/12683; and WO 97/20590, to which reference is hereby made and each of which is incorporated herein by reference in their entireties.

Rectal administration can be effected utilizing unit dose suppositories in which the compound is admixed with low-melting water-soluble or insoluble solids such as fats, cocoa butter, glycerinated gelatin, hydrogenated vegetable oils, mixtures of polyethylene glycols of various molecular weights, or fatty acid esters of polyethylene glycols, or the like. The active compound is usually a minor component, often from about 0.05 to 10% by weight, with the remainder being the base component.

In all of the above pharmaceutical compositions, the compounds of the invention are formulated with an acceptable carrier or excipient. The carriers or excipients used must, of course, be acceptable in the sense of being compatible with the other ingredients of the composition and must not be deleterious to the patient. The carrier or excipient can be a solid or a liquid, or both, and is preferably formulated with the compound of the invention as a unit-dose composition, for example, a tablet, which can contain from 0.05% to 95% by weight of the active compound. Such carriers or excipients include inert fillers or diluents, binders, lubricants, disintegrating agents, solution retardants, resorption accelerators, absorption agents, and coloring agents. Suitable binders include starch, gelatin, natural sugars such as glucose or β-lactose, corn sweeteners, natural and synthetic gums such as acacia, tragacanth or sodium alginate, carboxymethylcellulose, polyethylene glycol, waxes, and the like. Lubricants include sodium oleate, sodium stearate, magnesium stearate, sodium benzoate, sodium acetate, sodium chloride, and the like. Disintegrators include starch, methyl cellulose, agar, bentonite, xanthan gum, and the like.

Generally, a therapeutically effective daily dose is from about 0.001 mg to about 15 mg/kg of body weight per day of a compound of the invention; preferably, from about 0.1 mg to about 10 mg/kg of body weight per day; and most preferably, from about 0.1 mg to about 1.5 mg/kg of body weight per day. For example, for administration to a 70 kg person, the dosage range would be from about 0.07 mg to about 1050 mg per day of a compound of the invention, preferably from about 7.0 mg to about 700 mg per day, and most preferably from about 7.0 mg to about 105 mg per day. Some degree of routine dose optimization may be required to determine an optimal dosing level and pattern.

Pharmaceutically acceptable carriers and excipients encompass all the foregoing additives and the like.

### Examples of Pharmaceutical Formulations

| **A. TABLETS** | |
|---|---|
| **Component** | **Amount per tablet (mg)** |
| active substance | 100 |
| lactose | 140 |
| corn starch | 240 |
| polyvinylpyrrolidone | 15 |
| magnesium stearate | 5 |
| TOTAL | 500 |

The finely ground active substance, lactose, and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

| **B. TABLETS** | |
|---|---|
| **Component** | **Amount per tablet (mg)** |
| active substance | 80 |
| lactose | 55 |
| corn starch | 190 |
| polyvinylpyrrolidone | 15 |
| magnesium stearate | 2 |
| microcrystalline cellulose | 35 |
| sodium-carboxymethyl starch | 23 |
| TOTAL | 400 |

The finely ground active substance, some of the corn starch, lactose, microcrystalline cellulose, and polyvinylpyrrolidone are mixed together, the mixture is screened and worked with the remaining corn starch and water to form a granulate which is dried and screened. The sodiumcarboxymethyl starch and the magnesium stearate are added and mixed in and the mixture is compressed to form tablets of a suitable size.

| **C. COATED TABLETS** | |
|---|---|
| **Component** | **Amount per tablet (mg)** |
| active substance | 5 |
| lactose | 30 |
| corn starch | 41.5 |
| polyvinylpyrrolidone | 3 |
| magnesium stearate | 0.5 |
| TOTAL | 90 |

The active substance, corn starch, lactose, and polyvinylpyrrolidone are thoroughly mixed and moistened with water. The moist mass is pushed through a screen with a 1 mm mesh size, dried at about 45°C and the granules are then passed through the same screen. After the magnesium stearate has been mixed in, convex tablet cores with a diameter of 6 mm are compressed in a tablet-making machine. The tablet cores thus produced are coated in known manner with a covering consisting essentially of sugar and talc. The finished coated tablets are polished with wax.

| **D.CAPSULES** | |
|---|---|
| **Component** | **Amount per capsule (mg)** |
| active substance | 50 |
| corn starch | 268.5 |
| magnesium stearate | 1.5 |
| TOTAL | 320 |

The substance and corn starch are mixed and moistened with water. The moist mass is screened and dried. The dry granules are screened and mixed with magnesium stearate. The finished mixture is packed into size 1 hard gelatine capsules.

| **E. AMPOULE SOLUTION** | |
|---|---|
| **Component** | **Amount per ampoule** |
| active substance | 50 mg |
| sodium chloride | 50 mg |
| water for inj. | 5 mL |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilized and sealed by fusion. The ampoules contain 5 mg, 25 mg, and 50 mg of active substance.

| **F. SUPPOSITORIES** | |
|---|---|
| **Component** | **Amount per suppository (mg)** |
| active substance | 50 |
| solid fat | 1650 |
| TOTAL | 1700 |

The hard fat is melted. At 40°C, the ground active substance is homogeneously dispersed therein. The mixture is cooled to 38°C and poured into slightly chilled suppository molds.

| **G. METERING AEROSOL** | |
|---|---|
| **Component** | **Amount** |
| active substance | 0.005 |
| sorbitan trioleate | 0.1 |
| monofluorotrichloromethane and difluorodichloromethane (2:3) | to 100 |

The suspension is transferred into a conventional aerosol container with a metering valve. Preferably, 50 µL of suspension are delivered per spray. The active substance may also be metered in higher doses if desired (e.g., 0.02% by weight).

| **H. POWDER FOR INHALATION** | |
|---|---|
| **Component** | **Amount** |
| active substance | 1.0 mg |
| lactose monohydrate | to 25 mg |

| **I. POWDER FOR INHALATION** | |
|---|---|
| **Component** | **Amount** |
| active substance | 2.0 mg |
| lactose monohydrate | to 25 mg |

| **J. POWDER FOR INHALATION** | |
|---|---|
| **Component** | **Amount** |
| active substance | 1.0 mg |
| lactose monohydrate | to 5 mg |

| **K. POWDER FOR INHALATION** | |
|---|---|
| **Component** | **Amount** |
| active substance | 2.0 mg |
| lactose monohydrate | to 5 mg |

In Examples H, I, J, and K, the powder for inhalation is produced in the usual way by mixing the individual ingredients together.

## Claims

1. A compound of Formula (I) 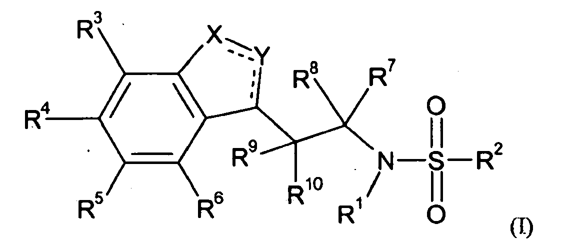 wherein:
R¹ is hydrogen;
R² is an aryl group substituted with one to five substituent groups provided the -SO₂- R² group is not a p-toluene sulfonyl group,
wherein each substituent group of R² is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂- C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁- C₅ alkylsulfonylamino, C₁-C₅ alkylaminosulfonyl, C₁-C₅ dialkylaminosulfonyl, halogen, hydroxy, carboxy, cyano, trifluoromethyl, trifluoromethoxy, nitro, or amino wherein the nitrogen atom is optionally independently mono- or di- substituted by C₁-C₅ alkyl or aryl; or ureido wherein either nitrogen atom is optionally independently substituted with C₁-C₅ alkyl; or C₁-C₅ alkylthio wherein the sulfur atom is optionally oxidized to a sulfoxide or sulfone;
wherein each substituent group of R² is optionally independently substituted with one to three substituent groups selected from C₁-C₅ alkyl, C₁-C₅ alkoxy, halogen, hydroxy, oxo, cyano, or amino wherein the nitrogen atom is optionally independently mono- or di-substituted by C₁-C₅ alkyl,
R³, R⁴, R⁵, and R⁶ are each independently hydrogen or C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁- C₅ alkylsulfonylamino, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein R³, R⁴, R⁵, and R⁶ are each optionally independently substituted with one to three substituent groups selected from C₁-C₃ alkyl, C₁-C₃ alkoxy, halogen, hydroxy, oxo, cyano, amino, or trifluoromethyl;
R⁷ and R⁸ are each independently hydrogen or C₁-C₅ alkyl, each optionally substituted with one to three substituent groups,
wherein each substituent group of R⁷ and R⁸ is independently C₁-C₅ alkyl, C₂-C5 alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁- C₅ alkylsulfonylamino, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein each substituent group of R⁷ and R⁸ is optionally independently substituted with one to three substituent groups selected from C₁-C₃ alkyl, C₁- C₃ alkoxy, halogen, hydroxy, oxo, cyano, amino, or trifluoromethyl,
R⁹ and R¹⁰ are each hydrogen or C₁-C₅ alkyl, each optionally substituted with one to three substituent groups,
wherein each substituent group of R⁹ and R¹⁰ is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, C₁-C₅ alkylaminocarbonyl, C₁-C₅ dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, C₁-C₅ alkylaminosulfonyl, C₁- C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein each substituent group of R⁹ and R¹⁰ is optionally independently substituted with one to three substituent groups selected from C₁-C₃ alkyl, C₁- C₃ alkoxy, halogen, hydroxy, oxo, cyano, amino, or trifluoromethyl,
wherein at least one of R⁷, R⁸, R⁹, or R¹⁰ is optionally substituted C₁-C₅ alkyl;
X is an NH group optionally substituted with one substituent group if no optional bond to X is present; and
Y is a CH₂ group optionally substituted with one or two substituent groups, or
is a CH group optionally substituted with one substituent group if an optional bond to Y is present,
wherein each substituent group of X and Y is independently C₁-C₃ alkyl, C₁-C₃ alkoxy, halogen, hydroxy, oxo, cyano, amino, or trifluoromethyl,
wherein each dashed line represents an optional bond, provided that zero or one of the optional bonds is present in the compound of Formula (I),
or a tautomer, ester, amide, solvate, or salt thereof.

2. The compound of Formula (I) according to claim 1, wherein R² is a substituted aryl group.

3. The compound of Formula (I) according to claim 1 or claim 2, wherein X is an NH group and Y is a CH group, or a tautomer, ester, amide, solvate, or salt thereof.

4. The compound of Formula (I) according to claim 3, wherein:
R² is a phenyl or naphthyl group, each substituted with one to five substituent groups,
wherein each substituent group of R² is independently C₁-C₅ alkyl, C₂-C₅ alkenyl, C₂-C₅ alkynyl, C₃-C₈ cycloalkyl, heterocyclyl, phenyl, naphthyl, heteroaryl, C₁-C₅ alkoxy, C₂-C₅ alkenyloxy, C₂-C₅ alkynyloxy, aryloxy, trifluoromethyl, trifluoromethoxy, halogen, or amino wherein the nitrogen atom is optionally independently mono- or di-substituted by C₁-C₅ alkyl or aryl, or C₁-C₅ alkylthio;
wherein each substituent group of R² is optionally independently substituted with one to three substituent groups selected from C₁-C₅ alkyl, C₁-C₅ alkoxy, halogen, hydroxy, oxo, cyano, or amino wherein the nitrogen atom is optionally independently mono- or di-substituted by C₁-C₅ alkyl,
R³, R⁴, R⁵, and R⁶ are each independently hydrogen or C₁-C₅ alkyl, C₂-C₅ alkenyl, C₂-C₅ alkynyl, C₃-C₈ cycloalkyl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, halogen, hydroxy, carboxy, cyano, trifluoromethyl, trifluoromethoxy, trifluoromethylthio, or C₁-C₅ alkylthio wherein the sulfur atom is optionally oxidized to a sulfoxide or sulfone;
R⁷ and R⁸ are each independently hydrogen or C₁-C₅ alkyl, each optionally substituted with one to three substituent groups,
wherein each substituent group of R⁷ and R⁸ is independently C₁-C₅ alkyl, C₂-C₅ alkenyl, C₂-C₅ alkynyl, C₃-C₈ cycloalkyl, aryl, halogen, trifluoromethyl, trifluoromethoxy, trifluoromethylthio, or C₁-C₅ alkylthio wherein the sulfur atom is optionally oxidized to a sulfoxide or sulfone,
wherein each substituent group of R⁷ and R⁸ is optionally independently substituted with one to three substituent groups selected from C₁-C₃ alkyl, C₁- C₃ alkoxy, halogen, hydroxy, oxo, cyano, amino, or trifluoromethyl,
R⁹ and R¹⁰ are each hydrogen or C₁-C₅ alkyl, each optionally substituted with one to three substituent groups,
wherein each substituent group of R⁹ and R¹⁰ is independently C₁-C₅ alkyl, C₂-C₅ alkenyl, C₂-C₅ alkynyl, C₃-C₈ cycloalkyl, aryl, halogen, trifluoromethyl, trifluoromethoxy, trifluoromethylthio, or C₁-C₅ alkylthio wherein the sulfur atom is optionally oxidized to a sulfoxide or sulfone,
wherein each substituent group of R⁹ and R¹⁰ is optionally independently substituted with one to three substituent groups selected from C₁-C₃ alkyl, C₁- C₃ alkoxy, halogen, hydroxy, oxo, cyano, amino, or trifluoromethyl,
or a tautomer, ester, amide, solvate, or salt thereof.

5. The compound of Formula (I) according to claim 3, wherein:
R² is a phenyl group substituted with one to three substituent groups,
wherein each substituent group of R² is independently C₁-C₅ alkyl or halogen,
R³, R⁴, R⁵, and R⁶ are each independently hydrogen or halogen;
R⁷ and R⁸ are each independently hydrogen or C₁-C₅ alkyl, each optionally substituted with one to three substituent groups,
wherein each substituent group of R⁷ and R⁸ is independently C₁-C₅ alkyl,
wherein each substituent group of R⁷ and R⁸ is optionally independently substituted with one to three substituent groups selected from C₁-C₃ alkyl; and
R⁹ and R¹⁰ are each hydrogen,
or a tautomer, ester, amide, solvate, or salt thereof.

6. The compound of Formula (I) according to claim 3, wherein at least one of R⁷, R⁸, R⁹, or R¹⁰ is C₁-C₅ alkyl, or a tautomer, ester, amide, solvate, or salt thereof.

7. The compound of Formula (I) according to claim 3, wherein at least two of R⁷, R⁸, R⁹, or R¹⁰ is C₁-C₅ alkyl, or a tautomer, ester, amide, solvate, or salt thereof.

8. The compound of Formula (I) according to claim 3, wherein at least three of R⁷, R⁸, R⁹, or R¹⁰ is C₁-C₅ alkyl, or a tautomer, ester, amide, solvate, or salt thereof.

9. The compound of Formula (I) according to claim 3, wherein each substituent group of R² is optionally independently substituted with one to three substituent groups selected from methoxy, chloro, bromo, or dimethylamino, or a tautomer, ester, amide, solvate, or salt thereof.

10. The compound of Formula (I) according to claim 1, wherein:
R² is a phenyl group substituted with three substituent groups,
wherein each substituent group of R² is independently C₁-C₅ alkyl or halogen,
wherein each substituent group of R² is optionally independently substituted with one to three substituent groups selected from C₁-C₅ alkyl;
R³, R⁴, R⁵, and R⁶ are each independently hydrogen or halogen;
R⁷ and R⁸ are each independently hydrogen or C₁-C₅ alkyl, each optionally substituted with one to three substituent groups,
wherein each substituent group of R⁷ and R⁸ is independently C₁-C₅ alkyl,
wherein each substituent group of R⁷ and R⁸ is optionally independently substituted with one to three substituent groups selected from C₁-C₃ alkyl; and
R⁹ and R¹⁰ are each hydrogen,
or a tautomer, ester, amide, solvate, or salt thereof.

11. The compound of Formula (I) according to claim 1, wherein each substituent group of R² is optionally independently substituted with three substituent groups selected from methyl, methoxy, chloro, bromo, or dimethylamino, or a tautomer, ester, amide, solvate, or salt thereof.

12. The compound of Formula (I) according to claim 3, wherein:
R² is a substituted phenyl or naphthyl group,
or a tautomer, ester, amide, solvate, or salt thereof.

13. The compound of Formula (I) according to claim 3, wherein X and Y are unsubstituted, or a tautomer, ester, amide, solvate, or salt thereof.

14. A compound according to claim 1, the compound selected from:
4-*tert*-Butyl-*N-*[2-(1*H*-indol-3-yl)-1-methylethyl]benzenesulfonamide;
2,5-Dichloro-*N-*[2-(1*H-*indol-3-yl)-1-methylethyl]benzenesulfonamide;
(*R*)-2-(4-*tert*-Butylbenzenesulfonylamino)-3-(1*H-*indol-3-yl)propionic acid methyl ester;
4-*tert*-Butyl-*N-*[2-(5-fluoro-1*H-*indol-3-yl)-1-methylethyl]benzenesulfonamide;
*4-tert*-Butyl-*N-*[1-methyl-2-(1-methyl-1*H-*indol-3-yl)ethyl]benzenesulfonamide;
4-*tert*-Butyl-*N-*[1-hydroxymethyl-2-(1*H-*indol-3-yl)ethyl]benzenesulfonamide;
5-Fluoro-*N-*[2-(1*H-*indol-3-yl)-1-methylethyl]-2-methoxybenzenesulfonamide;
5-Dimethylaminonaphthalene-1-sulfonic acid [2-(1*H-*indol-3-yl)-1-methylethyl]amide;
2,4,5-Trichloro-*N-*[2-(1*H-*indol-3-yl)-1-methylethyl]benzenesulfonamide;
N [2-(1*H-*Indol-3-yl)-1-methylethyl]-2,4,6-trimethylbenzenesulfonamide;
*N-*[2-(1*H-*Indol-3-yl)-1-methylethyl]-2,3,4,5,6-pentamethylbenzenesulfonamide;
*N-*[2-(1*H-*Indol-3-yl)-1-methylethyl]-4-methoxy-2,3,6-trimethylbenzenesulfonamide;
3,4-Dichloro-*N-*[2-(1*H-*indol-3-yl)-1-methylethyl]benzenesulfonamide;
4-(1,1-Dimethylpropyl)-*N-*[2-(1*H-*indol-3-yl)-1-methylethyl]benzenesulfonamide;
*N*-[2-(1*H-*Indol-3-yl)-1-methylethyl]-4-isopropylbenzenesulfonamide;
2-Chloro-*N-*[2-(1*H-*indol-3-yl)-1-methylethyl]-6-methylbenzenesulfonamide;
3-Chloro-*N-*[2-(1*H-*indol-3-yl)-1-methylethyl]-2-methylbenzenesulfonamide;
2,6-Dichloro-*N-*[2-(1*H-*indol-3-yl)-1-methylethyl]benzenesulfonamide;
2,4-Dichloro-*N-*[2-(1*H-*indol-3-yl)-1-methylethyl]benzenesulfonamide;
5-Fluoro-*N-*[2-(1*H-*indol-3-yl)-1-methylethyl]-2-methylbenzenesulfonamide;
2,4,6-Trichloro-*N-*[2-(1*H-*indol-3-yl)-1-methylethyl]benzenesulfonamide;
4-Butyl-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide;
2,5-Dibromo-3,6-difluoro-*N-*[2-(1*H-*indol-3-yl)-1-methylethyl]benzenesulfonamide;
*N-*[2-(1*H-*Indol-3-yl)-1-methylethyl]-2,3,5,6-tetramethylbenzenesulfonamide;
4-*tert*-Butyl-*N-*[1-(1*H-*indol-3-ylmethyl)propyl]benzenesulfonamide;
2,4,6-Trichloro-*N-*[1-(1*H-*indol-3-ylmethyl)propyl]benzenesulfonamide;
4-*tert*-Butyl-*N-*[2-(5-methoxy-1*H-*indol-3-yl)-1-methylethyl]benzenesulfonamide;
2,4,6-Trichloro-*N-*[2-(5-methoxy-1*H-*indol-3-yl)-1-methylethyl]benzenesulfonamide;
4-*tert*-Butyl-*N-*[1-methyl-2-(2-methyl-1*H-*indol-3-yl)ethyl]benzenesulfonamide;
2,4,6-Trichloro-*N-*[1-methyl-2-(2-methyl-1*H-*indol-3-yl)ethyl]benzenesulfonamide;
4-*tert*-Butyl-*N-*[2-(1*H-*indol-3-yl)-2-methylpropyl]benzenesulfonamide;
2,4,6-Trichloro-*N-*[2-(1*H-*indol-3-yl)-2-methylpropyl]benzenesulfonamide;
*N-*[2-(5-Fluoro-1*H-*indol-3-yl)-1-methylethyl]-2,4,6-trimethyl-benzenesulfonamide;
*N-*[1-Hydroxymethyl-2-(1*H-*indol-3-yl)ethyl]-2,4,6-trimethylbenzenesulfonamide;
2,4,6-Trimethyl-*N-*[1-methyl-2-(1-methyl-1*H-*indol-3-yl)ethyl]benzenesulfonamide;
(*S*)-3-(1*H-*Indol-3-yl)-2-(2,4,6-trimethylbenzenesulfonylamino)propionic acid methyl ester;
(*R*)-3-(1*H-*Indol-3-yl)-2-(2,4,6-trimethylbenzenesulfonylamino)propionic acid methyl ester;
*N-*[1-(1*H-*Indol-3-ylmethyl)propyl]-2,4,6-trimethylbenzenesulfonamide;
*N-*[2-(6-Fluoro-1*H-*indol-3-yl)-1-methylethyl]-2,4,6-trimethylbenzenesulfonamide;
2,4,6-Trimethyl-*N-*[1-methyl-2-(7-methyl-1*H-*indol-3-yl)ethyl]benzenesulfonamide;
2,4,6-Trimethyl-*N-*[1-methyl-2-(6-methyl-1*H-*indol-3-yl)ethyl]benzenesulfonamide;
*N-*[2-(1*H-*Indol-3-yl)-1-methylethyl]-4-iodobenzenesulfonamide;
*N-*[2-(1*H-*Indol-3-yl)-1-methylethyl]-2,4-dinitrobenzenesulfonamide;
*N-*[2-(1*H-*Indol-3-yl)-1-methylethyl]-2-nitrobenzenesulfonamide;
3,5-Dichloro-2-hydroxy-*N-*[2-(1*H*-indol-3-yl)-1-methylethyl]benzenesulfonamide;
*N-*[*2-*(1*H-*Indol-3-yl)*-*1-methylethyl]-3-nitrobenzenesulfonamide;
4-Bromo-*N-*[2-(1*H-*indol-3-yl)-1-methylethyl]benzenesulfonamide;
4-Fluoro-*N-*[²-(1*H-*indol-3-yl)-1-methylethyl]benzenesulfonamide;
4-Chloro-*N-*[2-(1*H-*indol-3-yl)-1-methylethyl]benzenesulfonamide;
4-Chloro-*N-*[2-(1*H-*indol-3-yl)-1-methylethyl]-3-nitrobenzenesulfonamide;
*N-*{4-[2-(1*H-*Indol-3-yl)-1-methylethylsulfamoyl]phenyl}acetamide;
*N-*[2-(1*H-*Indol-3-yl)-1-methylethyl]-4-nitrobenzenesulfonamide;
*N-*[2-(1*H-*Indol-3-yl)-1-methylethyl]-4-methoxybenzenesulfonamide;
*N-*[2-(1*H-*Indol-3-yl)-1-methylethyl]-3-methylbenzenesulfonamide;
*N-*[2-(1*H-*Indol-3-yl)-1-methylethyl]-2-nitro-4-trifluoromethylbenzenesulfonamide;
*N-*[2-(1*H-*Indol-3-yl)-1-methylethyl]-3-trifluoromethylbenzenesulfonamide;
2,3,4-Trichloro-*N-*[2-(1*H-*indol-3-yl)-1-methylethyl]benzenesulfonamide;
*N-*[2-(1*H-*Indol-3-yl)-1-methylethyl]-2,5-dimethoxybenzenesulfonamide;
3,4-Dichloro-*N-*[2-(1*H-*indol-3-yl)-1-methylethyl]benzenesulfonamide;
3-Chloro-4-fluoro-*N-*[2-(1*H-*indol-3-yl)-1-methylethyl]benzenesulfonamide;
4-Ethyl-*N-*[2-(1*H-*indol-3-yl)-1-methylethyl]benzenesulfonamide;
*N-*[2-(1*H-*Indol-3-yl)-1-methylethyl]-4-propylbenzenesulfonamide;
4-Bromo-2,5-difluoro-*N-*[2-(1*H-*indol-3-yl)-1-methylethyl]benzenesulfonamide;
2-Fluoro-*N-*[2-(1*H-*indol-3-yl)-1-methylethyl]benzenesulfonamide;
*N-*[2-(1*H-*Indol-3-yl)-1-methylethyl]-4-trifluoromethoxybenzenesulfonamide;
*N-*[2-(1*H-*Indol-3-yl)-1-methylethyl]-4-trifluoromethylbenzenesulfonamide;
2,4-Difluoro-*N-*[2-(1*H-*indol-3-yl)-1-methylethyl]benzenesulfonamide;
4-Chloro-*N-*[2-(1*H-*indol-3-yl)-1-methylethyl]-2,5-dimethylbenzenesulfonamide;
2-Chloro-*N-*[2-(1*H-*indol-3-yl)-1-methylethyl]benzenesulfonamide;
*N-*[2-(1*H-*Indol-3-yl)-1-methylethyl]-2-methyl-5-nitrobenzenesulfonamide;
*N-*[2-(1*H-*Indol-3-yl)-1-methylethyl]-2-trifluoromethylbenzenesulfonamide;
3-Chloro-*N-*[2-(1*H-*indol-3-yl)-1-methylethyl]benzenesulfonamide;
5-Bromo-*N-*[2-(1*H-*indol-3-yl)-1-methylethyl]-2-methoxybenzenesulfonamide;
*N-*[2-(1*H-*Indol-3-yl)-1-methylethyl]-3,4-dimethoxybenzenesulfonamide;
2,3-Dichloro-*N-*[2-(1*H-*indol-3-yl)-1-methylethyl]benzenesulfonamide;
2-Bromo-*N-*[2-(1*H-*indol-3-yl)-1-methylethyl]benzenesulfonamide;
3-Bromo-[2-(1*H-*indol-3-yl)-1-methylethyl]benzenesulfonamide;
*N-*[2-(1*H-*Indol-3-yl)-1-methylethyl]-2-trifluoromethoxybenzenesulfonamide;
3-Cyano-*N-*[2-(1*H-*indol-3-yl)-1-methylethyl]benzenesulfonamide;
2-Cyano-*N-*[2-(1*H-*indol-3-yl)-1-methylethyl]benzenesulfonamide;
*N-*[2-(1*H-*Indol-3-yl)-1-methylethyl]-2-methoxy-5-methylbenzenesulfonamide;
2-Chloro-4-fluoro-*N-*[2-(1*H-*indol-3-yl)-1-methylethyl]benzenesulfonamide;
5-Chloro-*N-*[2-(1*H-*indol-3-yl)-1-methylethyl]-2-methoxybenzenesulfonamide;
Biphenyl-4-sulfonic acid [2-(1*H-*indol-3-yl)-1-methylethyl]amide;
2,6-Difluoro-*N-*[2-(1*H-*indol-3-yl)-1-methylethyl]benzenesulfonamide;
*N-*[2-(1*H-*Indol-3-yl)-1-methylethyl]-4-methoxy-3,5-dinitrobenzenesulfonamide;
*N-*[2-(1*H-*Indol-3-yl)-1-methylethy)]-4-methanesulfonylbenzenesulfonamide;
*N-*[2-(1*H-*Indol-3-yl)-1-methylethyl]-2-methanesulfonylbenzenesulfonamide;
4-Acetyl-*N-*[2-(1*H-*indol-3-yl)-1-methylethyl]benzenesulfonamide;
3,5-Dichloro-*N-*[2-(1*H-*indol-3-yl)-1-methylethyl]benzenesulfonamide; and
*N-*[2-(1*H-*Indol-3-yl)-1-methylethyl]-3,5-bis-trifluoromethylbenzenesulfonamide;
or a tautomer, ester, amide, solvate, or salt thereof.

15. A compound according to claim 1, the compound selected from:
5-Dimethylaminonaphthalene-1-sulfonic acid [2-(1*H-*indol-3-yl)-1-methylethyl]amide;
*N-*[2-(1*H-*Indol-3-yl)-1-methylethyl]-2,4,6-trimethylbenzenesulfonamide;
*N-*[2-(1*H-*Indol-3-yl)-1-methylethyl]-2,3,4,5,6-pentamethylbenzenesulfonamide;
N [2-(1*H-*Indol-3-yl)-1-methylethyl]-4-methoxy-2,3,6-trimethylbenzenesulfonamide;
2-Chloro-*N-*[2-(1*H-*indol-3-yl)-1-methylethyl]-6-methylbenzenesulfonamide;
2,6-Dichloro-*N-*[2-(1*H-*indol-3-yl)-1-methylethyl]benzenesulfonamide;
2,4,6-Trichloro-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide;
4-*tert*-Butyl-*N-*[1-(1*H-*indol-3-ylmethyl)propyl]benzenesulfonamide;
2,4,6-Trichloro-*N-*[1-(1*H-*indol-3-ylmethyl)propyl]benzenesulfonamide;
*N-*[2-(5-Fluoro-1*H-*indol-3-yl)-1-methylethyl]-2,4,6-trimethylbenzenesulfonamide;
*N*-[1-(1*H-*Indol-3-ylmethyl)propyl]-2,4,6-trimethylbenzenesulfonamide;
*N-*[2-(6-Fluoro-1*H-*indol-3-yl)-1-methylethyl]-2,4,6-trimethylbenzenesulfonamide;
2,4,6-Trimethyl-*N-*[1-methyl-2-(6-methyl-1*H-*indol-3-yl)ethyl]benzenesulfonamide; and
2,4,6-Trimethyl-*N-*[1-methyl-2-(7-methyl-1*H-*indol-3-yl)ethyl]benzenesulfonamide,
or a tautomer, ester, amide, solvate, or salt thereof.

16. A compound according to claim 1, the compound selected from:
*N-*[2-(1*H-*Indol-3-yl)-1-methylethyl]-2,4,6-trimethylbenzenesulfonamide;
2,4,6-Trichloro-*N-*[2-(1*H-*indol-3-yl)-1-methylethyl]benzenesulfonamide;
*N-*[2-(1*H-*Indol-3-yl)-1-methylethyl]-2,4,6-triisopropylbenzenesulfonamide;
2,4,6-Trichloro-*N-*[1-(1*H-*indol-3-ylmlethyl)propyl]benzenesulfonamide;
*N-*[2-(5-Fluoro-1*H-*indol-3-yl)-1-methylethyl]-2,4,6-trimethylbenzenesulfonamide;
*N-*[1-(1*H-*Indol-3-ylmethyl)propyl]-2,4,6-trimethylbenzenesulfonamide;
*N-*[2-(6-Fluoro-1*H-*indol-3-yl)-l-methylethyl]-2,4,6-trimethylbenzenesulfonamide; and
2,4,6-Trimethyl-*N-*[1-methyl-2-(6-methyl-1*H-*indol-3-yl)ethyl]benzenesulfonamide,
or a tautomer, ester, amide, solvate, or salt thereof.

17. A pharmaceutical composition comprising an effective amount of a compound according to one of claims 1 to 16, or a tautomer, ester, amide, solvate, or salt thereof, and a pharmaceutically acceptable excipient or carrier.

18. Use of a compound according to one of claims I to 16, or a tautomer, ester, amide, solvate, or salt thereof for the preparation of a pharmaceutical composition to modulate the glucocorticoid receptor function in a patient.

19. Use of a compound according to one of claims 1 to 16, or a tautomer, ester, amide, solvate, or salt thereof for the preparation of a pharmaceutical composition to treat a disease-state or condition mediated by the glucocorticoid receptor function in a patient.

20. Use of a compound according to one of claims 1 to 16, or a tautomer, ester, amide, solvate, or salt thereof for the preparation of a pharmaceutical composition to treat a disease-state or condition selected from: type II diabetes, obesity, cardiovascular diseases, hypertension, arteriosclerosis, neurological diseases, adrenal and pituitary tumors, and glaucoma, in a patient.

21. Use of a compound according to one of claims 1 to 16, or a tautomer, ester, amide, solvate, or salt thereof for the preparation of a pharmaceutical composition to treat a disease **characterized by** inflammatory, allergic, or proliferative processes, in a patient.

22. The use according to claim 21, wherein the disease is selected from: (i) lung diseases; (ii) rheumatic diseases/autoimmune diseases/joint diseases; (iii) allergic diseases; (iv) vasculitis diseases; (v) dermatological diseases; (vi) renal diseases; (vii) hepatic diseases; (viii) gastrointestinal diseases; (ix) proctological diseases; (x) eye diseases; (xi) diseases of the ear, nose, and throat (ENT) area; (xii) neurological diseases; (xiii) blood diseases; (xiv) tumor diseases; (xv) endocrine diseases; (xvi) organ and tissue transplantations and graft-versus-host diseases; (xvii) severe states of shock; (xviii) substitution therapy; and (xix) pain of inflammatory genesis.

23. The use according to claim 21, wherein the disease is selected from: type I diabetes, osteoarthritis, Guillain-Barre syndrome, restenosis following percutaneous transluminal coronary angioplasty, Alzheimer disease, acute and chronic pain, atherosclerosis, reperfusion injury, bone resorption diseases, congestive heart failure, myocardial infarction, thermal injury, multiple organ injury secondary to trauma, acute purulent meningitis, necrotizing enterocolitis, and syndromes associated with hemodialysis, leukopheresis, and granulocyte transfusion.

24. Use of (a) a compound according to one of claims 1 to 16 or a tautomer, ester, amide, solvate, or salt thereof; and (b) a glucocorticoid for the preparation of a pharmaceutical composition to treat a disease-state or condition mediated by the glucocorticoid receptor function in a patient.

25. A kit for the *in vitro* diagnostic determination of the glucocorticoid receptor function in a sample, comprising:
(a) a diagnostically effective amount of a compound according to claim 1 or a tautomer, ester, amide, solvate, or salt thereof; and
(b) instructions for use of the diagnostic kit.

26. A method of making a compound of Formula (I) 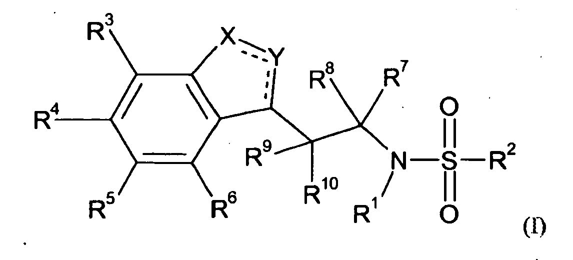 where R¹ is H and R², R³, R⁴, R⁵, R⁶, R⁷, R⁸, R⁹, R¹⁰, X, and Y are as defined in claim 1, the method comprising reacting the aminoethyl heterocycle of Formula (II) with a sulfonyl halide of Formula (III) in the presence of a suitable base to form the compound of Formula (I)

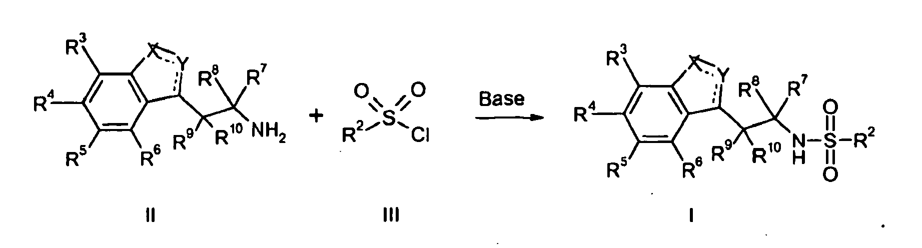

27. A method of making a compound of Formula (I) 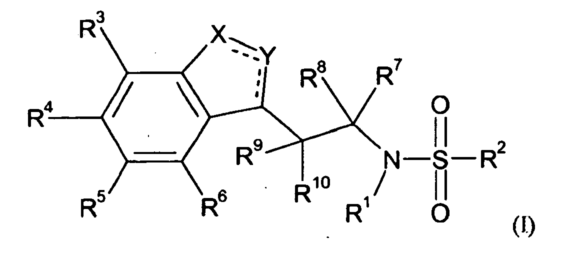 where R¹, R⁸, and R⁹ are each H and R² R³, R⁴, R⁵, R⁶, R⁷, R¹⁰, X, and Y are as defined in claim 1, the method comprising:
(a) reacting the aldehyde of Formula (V) with the nitro compound of Formula (VI) in the presence of ammonium acetate and acetic acid to form the nitroalkene of Formula (VII) 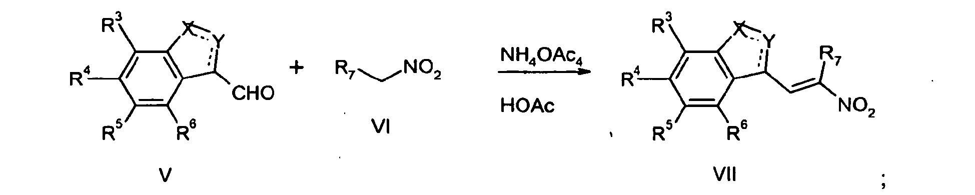
(b) reducing the nitroalkene of Formula (VII) with a suitable reducing agent to form the intermediate of Formula (II) 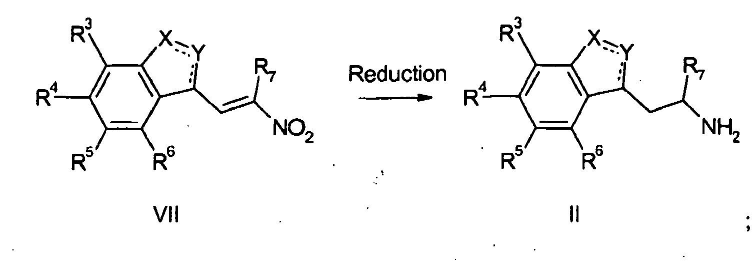 and
(c) reacting the intermediate of Formula (II) with a sulfonyl halide of Formula (III) in the presence of a suitable base to form the compound of Formula (I)

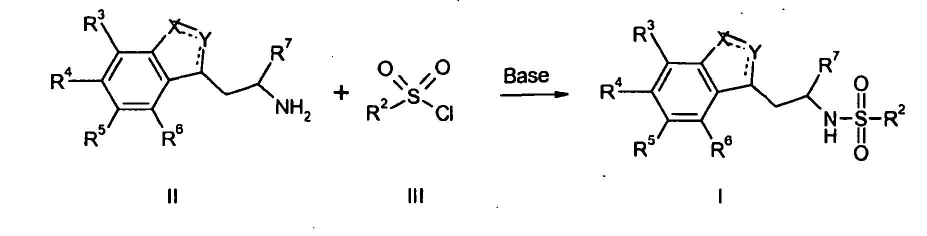

## Patentansprüche

1. Verbindung der Formel (I) 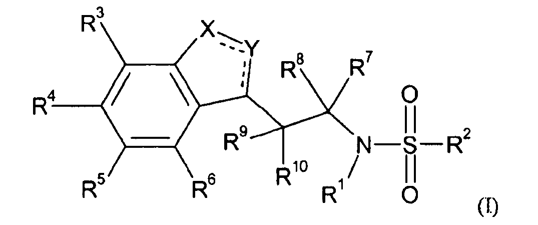 worin:
R¹ Wasserstoff darstellt;
R² eine Arylgruppe darstellt, substituiert mit ein bis fünf Substituentengruppen, vorausgesetzt dass die -SO₂-R²-Gruppe keine p-Toluolsulfonylgruppe dar- stellt,
worin jede Substituentengruppe von R² unabhängig darstellt: C₁-C₅-Alkyl, C₂- C₅-Alkenyl, C₂-C₅-Alkinyl, C₃-C₈-Cycloalkyl, Heterocyclyl, Aryl, Heteroaryl, C₁-C₅-Alkoxy, C₂-C₅-Alkenyloxy, C₂-C₅-Alkinyloxy, Aryloxy, Acyl, C₁-C₅- Alkoxycarbonyl, C₁-C₅-Alkanoyloxy, Aminocarbonyl, C₁-C₅-Alkylaminocar- bonyl, C₁-C₅-Dialkylaminocarbonyl, Aminocarbonyloxy, C₁-C₅-Alkylami- nocarbonyloxy, C₁-C₅-Dialkylaminocarbonyloxy, C₁-C₅-Alkanoylamino, C₁- C₅-Alkoxycarbonylamino, C₁-C₅-Alkylsulfonylamino, C₁-C₅-Alkylaminosul- fonyl, C₁-C₅-Dialkylaminosulfonyl, Halogen, Hydroxy, Carboxy, Cyano, Trifluormethyl, Trifluormethoxy, Nitro oder Amino, worin das Stickstoffatom gegebenenfalls unabhängig mono- oder disubstituiert ist mit C₁-C₅-Alkyl oder Aryl; oder Ureido, worin jedes Stickstoffatom gegebenenfalls unabhängig sub- stituiert ist mit C₁-C₅-Alkyl; oder C₁-C₅-Alkylthio, worin das Schwefelatom gegebenenfalls zu einem Sulfoxid oder Sulfon oxidiert ist;
wobei jede Substituentengruppe von R² gegebenenfalls unabhängig substituiert ist mit ein bis drei Substituentengruppen, ausgewählt aus C₁-C₅-Alkyl, C₁-C₅-Alkoxy, Halogen, Hydroxy, Oxo, Cyano oder A- mino, worin das Stickstoffatom gegebenenfalls unabhängig mono- o- der disubstituiert ist mit C₁-C₅-Alkyl,
R³, R⁴, R⁵ und R⁶ jeweils unabhängig darstellen: Wasserstoff oder C₁-C₅-Alkyl, C₂-C₅- Alkenyl, C₂-C₅-Alkinyl, C₃-C₈-Cycloalkyl, Heterocyclyl, Aryl, Heteroaryl, C₁- C₅-Alkoxy, C₂-C₅-Alkenyloxy, C₂-C₅-Alkinyloxy, Aryloxy, Acyl, C₁-C₅- Alkoxycarbonyl, C₁-C₅-Alkanoyloxy, Aminocarbonyl, C₁-C₅-Alkylaminocar- bonyl, C₁-C₅-Dialkylaminocarbonyl, Aminocarbonyloxy, C₁-C₅-Alkylami- nocarbonyloxy, C₁-C₅-Dialkylaminocarbonyloxy, C₁-C₅-Alkanoylamino, C₁- C₅-Alkoxycarbonylamino, C₁-C₆-Alkylsulfonylamino, C₁-C₅-Alkylaminosul- fonyl, C₁-C₅-Dialkylaminosulfonyl, Halogen, Hydroxy, Carboxy, Cyano, Trifluormethyl, Trifluormethoxy, Trifluormethylthio, Nitro oder Amino, worin das Stickstoffatom gegebenenfalls unabhängig mono- oder disubstituiert ist mit C₁-C₅-Alkyl; oder Ureido, worin jedes Stickstoffatom gegebenenfalls un- abhängig substituiert ist mit C₁-C₅-Alkyl; oder C₁-C₅-Alkylthio, worin das Schwefelatom gegebenenfalls zu einem Sulfoxid oder Sulfon oxidiert ist;
wobei R³, R⁴, R⁵ und R⁶ jeweils gegebenenfalls unabhängig substitu- iert sind mit ein bis drei Substituentengruppen, ausgewählt aus C₁-C₃- Alkyl, C₁-C₃-Alkoxy, Halogen, Hydroxy, Oxo, Cyano, Amino oder Trifluormethyl;
R⁷ und R⁸ sind jeweils unabhängig Wasserstoff oder C₁-C₅-Alkyl, jedes gegebenenfalls substituiert mit ein bis drei Substituentengruppen,
wobei jede Substituentengruppe von R⁷ und R⁸ unabhängig darstellt: C₁-C₅-Alkyl, C₂-C₅-Alkenyl, C₂-C₅-Alkinyl, C₃-C₈-Cycloalkyl, Hete- rocyclyl, Aryl, Heteroaryl, C₁-C₅-Alkoxy, C₂-C₅-Alkenyloxy, C₂-C₅- Alkinyloxy, Aryloxy, Acyl, C₁-C₅-Alkoxycarbonyl, C₁-C₅- Alkanoyloxy, Aminocarbonyl, C₁-C₅-Alkylaminocarbonyl, C₁-C₅- Dialkylaminocarbonyl, Aminocarbonyloxy, C₁-C₅-Alkylaminocarbo- nyloxy, C₁-C₅-Dialkylaminocarbonyloxy, C₁-C₅-Alkanoylamino, C₁- C₅-Alkoxycarbonylamino, C₁-C₅-Alkylsulfonylamino, C₁-C₅-Alkyl- aminosulfonyl, C₁-C₅-Dialkylaminosulfonyl, Halogen, Hydroxy, Car- boxy, Cyano, Trifluormethyl, Trifluormethoxy, Trifluormethylthio, Nitro oder Amino, worin das Stickstoffatom gegebenenfalls unabhän- gig mono- oder disubstituiert ist mit C₁-5₁₅-Alkyl; oder Ureido, worin jedes Stickstoffatom gegebenenfalls unabhängig substituiert ist mit C₁- C₅-Alkyl; oder C₁-C₅-Alkylthio, worin das Schwefelatom gegebenen- falls zu einem Sulfoxid oder Sulfon oxidiert ist;
wobei jede Substituentengruppe von R⁷ und R⁸ gegebenenfalls unab- hängig substituiert ist mit ein bis drei Substituentengruppen, ausge- wählt aus C₁-C₃-Alkyl, C₁-C₃-Alkoxy, Halogen, Hydroxy, Oxo, Cya- no, Amino oder Trifluormethyl;
R⁹ und R¹⁰ sind jeweils Wasserstoff oder C₁-C₅-Alkyl, jedes gegebenenfalls substitu- iert mit ein bis drei Substituentengruppen,
wobei jede Substituentengruppe von R⁹ und R¹⁰ unabhängig darstellt: C₁-C₅-Alkyl, C₂-C₅-Alkenyl, C₂-C₅-Alkinyl, C₃-C₈-Cycloalkyl, Hete- rocyclyl, Aryl, Heteroaryl, C₁-C₅-Alkoxy, C₂-C₅-Alkenyloxy, C₂-C₅- Alkinyloxy, Aryloxy, Acyl, C₁-C₅-Alkoxycarbonyl, C₁-C₅- Alkanoyloxy, Aminocarbonyl, C₁-C₅-Alkylaminocarbonyl, C₁-C₅- Dialkylaminocarbonyl, Aminocarbonyloxy, C₁-C₅-Alkylaminocarbo- nyloxy, C₁-C₅-Dialkylaminocarbonyloxy, C₁-C₅-Alkanoylamino, C₁- C₅-Alkoxycarbonylamino, C₁-C₅-Alkylsulfonylamino, C₁-C₅-Alkyl- aminosulfonyl, C₁-C₅-Dialkylaminosulfonyl, Halogen, Hydroxy, Car- boxy, Cyano, Trifluormethyl, Trifluormethoxy, Trifluormethylthio, Nitro oder Amino, worin das Stickstoffatom gegebenenfalls unabhän- gig mono- oder disubstituiert ist mit C₁-C₅-Alkyl; oder Ureido, worin jedes Stickstoffatom gegebenenfalls unabhängig substituiert ist mit C₁- C₅-Alkyl; oder C₁-C₅-Alkylthio, worin das Schwefelatom gegebenen- falls zu einem Sulfoxid oder Sulfon oxidiert ist;
wobei jede Substituentengruppe von R⁹ und R¹⁰ gegebenen- falls unabhängig substituiert ist mit ein bis drei Substituen- tengruppen, ausgewählt aus C₁-C₃-Alkyl, C₁-C₃-Alkoxy, Ha- logen, Hydroxy, Oxo, Cyano, Amino oder Trifluormethyl;
wobei mindestens eines von R⁷, R⁸, R⁹ oder R¹⁰ gegebenenfalls substituiertes C₁-C₅-Alkyl darstellt;
X eine NH-Gruppe darstellt, gegebenenfalls substituiert mit einer Substituen- tengruppe, wenn keine optionale Bindung zu X vorhanden ist; und
Y eine CH₂-Gruppe darstellt, gegebenenfalls substituiert mit ein oder zwei Sub- stituentengruppen, oder
eine CH-Gruppe darstellt, gegebenenfalls substituiert mit einer Substituen- tengruppe, wenn eine optionale Bindung zu Y vorliegt,
wobei jede Substituentengruppe von X und Y unabhängig C₁-C₃- Alkyl, C₁-C₃-Alkoxy, Halogen, Hydroxy, Oxo, Cyano, Amino oder Trifluormethyl darstellt,
wobei jede gestrichelte Linie eine optionale Bindung darstellt, vorausgesetzt, dass null oder eine der optionalen Bindungen in der Verbindung der Formel (I) vorhanden ist,
oder ein Tautomer, Ester, Amid, Solvat oder Salz hiervon.

2. Verbindung der Formel (I) nach Anspruch 1, worin R² eine substituierte Arylgruppe darstellt.

3. Verbindung der Formel (I) nach Anspruch 1 oder Anspruch 2, worin X eine NH-Gruppe darstellt und Y eine CH-Gruppe darstellt, oder ein Tautomer, Ester, Amid, Solvat oder Salz hiervon.

4. Verbindung der Formel (I) nach Anspruch 3, worin:
R² eine Phenyl- oder Naphthylgruppe darstellt, jede substituiert mit ein bis fünf Substituentengruppen,
worin jede Substituentengruppe von R² unabhängig darstellt: C₁-C₅-Alkyl, C₂- C₅-Alkenyl, C₂-C₅-Alkinyl, C₃-C₈-Cycloalkyl, Heterocyclyl, Phenyl, Naphthyl, Heteroaryl, C₁-C₅-Alkoxy, C₂-C₅-Alkenyloxy, C₂-C₅-Alkinyloxy, Aryloxy, Trifluormethyl, Trifluormethoxy, Halogen oder Amino, worin das Stickstoff- atom gegebenenfalls unabhängig mono- oder disubstituiert ist mit C₁-C₅-Alkyl oder Aryl, oder C₁-C₅-Alkylthio;
wobei jede Substituentengruppe von R² gegebenenfalls unabhängig substituiert ist mit ein bis drei Substituentengruppen, ausgewählt aus C₁-C₅-Alkyl, C₁-C₅-Alkoxy, Halogen, Hydroxy, Oxo, Cyano oder A- mino, worin das Stickstoffatom gegebenenfalls unabhängig mono- o- der disubstituiert ist mit C₁-C₅-Alkyl,
R³, R⁴, R⁵ und R⁶ jeweils unabhängig darstellen: Wasserstoff oder C₁-C₅-Alkyl, C₂-C₅- Alkenyl, C₂-C₅-Alkinyl, C₃-C₈-Cycloalkyl, C₁-C₅-Alkoxy, C₂-C₅-Alkenyloxy, C₂-C₅-Alkinyloxy, Aryloxy, Acyl, C₁-C₅-Alkoxycarbonyl, C₁-C₅- Alkanoyloxy, Halogen, Hydroxy, Carboxy, Cyano, Trifluormethyl, Trifluor- methoxy, Trifluormethylthio oder C₁-C₅-Alkylthio, worin das Schwefelatom gegebenenfalls zu einem Sulfoxid oder Sulfon oxidiert ist;
R⁷ und R⁸ sind jeweils unabhängig Wasserstoff oder C₁-C₅-Alkyl, jedes gegebenenfalls substituiert mit ein bis drei Substituentengruppen,
wobei jede Substituentengruppe von R⁷ und R⁸ unabhängig darstellt: C₁-C₅-Alkyl, C₂-C₅-Alkenyl, C₂-C₅-Alkinyl, C₃-C₈-Cycloalkyl, Aryl, Halogen, Trifluormethyl, Trifluormethoxy, Trifluormethylthio, oder C₁-C₅-Alkylthio, worin das Schwefelatom gegebenenfalls zu einem Sulfoxid oder Sulfon oxidiert ist;
wobei jede Substituentengruppe von R⁷ und R⁸ jeweils gege- benenfalls unabhängig substituiert ist mit ein bis drei Substi- tuentengruppen, ausgewählt aus C₁-C₃-Alkyl, C₁-C₃-Alkoxy, Halogen, Hydroxy, Oxo, Cyano, Amino oder Trifluormethyl;
R⁹ und R¹⁰ sind jeweils Wasserstoff oder C₁-C₅-Alkyl, jedes gegebenenfalls substitu- iert mit ein bis drei Substituentengruppen,
wobei jede Substituentengruppe von R⁹ und R¹⁰ unabhängig darstellt: C₁-C₅-Alkyl, C₂-C₅-Alkenyl, C₂-C₅-Alkinyl, C₃-C₈-Cycloalkyl, Aryl, Halogen, Trifluormethyl, Trifluormethoxy, Trifluormethylthio oder C₁-C₅-Alkylthio, worin das Schwefelatom gegebenenfalls zu einem Sulfoxid oder Sulfon oxidiert ist; wobei jede Substituentengruppe von R⁹ und R¹⁰ gegebenen- falls unabhängig substituiert ist mit ein bis drei Substituen- tengruppen, ausgewählt aus C₁-C₃-Alkyl, C₁-C₃-Alkoxy, Ha- logen, Hydroxy, Oxo, Cyano, Amino oder Trifluormethyl;
oder ein Tautomer, Ester, Amid, Solvat oder Salz hiervon.

5. Verbindung der Formel (I) nach Anspruch 3, worin
R² eine Phenylgruppe darstellt, substituiert mit ein bis drei Substituentengruppen,
worin jede Substituentengruppe von R² unabhängig C₁-C₅-Alkyl oder Halogen darstellt;
R³, R⁴, R⁵ und R⁶ jeweils unabhängig Wasserstoff oder Halogen darstellen;
R⁷ und R⁸ sind jeweils unabhängig Wasserstoff oder C₁-C₅-Alkyl, jedes gegebenenfalls substituiert mit ein bis drei Substituentengruppen,
wobei jede Substituentengruppe von R⁷ und R⁸ unabhängig C₁-C₅- Alkyl darstellt;
worin jede Substituentengruppe von R⁷ und R⁸ gegebenenfalls unabhängig substituiert ist mit ein bis drei Substituentengrup- pen, ausgewählt aus C₁-C₅-Alkyl; und
R⁹ und R¹⁰ jeweils Wasserstoff sind,
oder ein Tautomer, Ester, Amid, Solvat oder Salz hiervon.

6. Verbindung der Formel (I) nach Anspruch 3, worin mindestens eines von R⁷, R⁸, R⁹ oder R¹⁰ C₁-C₅-Alkyl darstellt, oder ein Tautomer, Ester, Amid, Solvat oder Salz hiervon.

7. Verbindung der Formel (I) nach Anspruch 3, worin mindestens zwei von R⁷, R⁸, R⁹ oder R¹⁰ C₁-C₅-Alkyl darstellen, oder ein Tautomer, Ester, Amid, Solvat oder Salz hiervon.

8. Verbindung der Formel (I) nach Anspruch 3, worin mindestens drei von R⁷, R⁸, R⁹ oder R¹⁰ C₁-C₅-Alkyl darstellen, oder ein Tautomer, Ester, Amid, Solvat oder Salz hiervon.

9. Verbindung der Formel (I) nach Anspruch 3, worin jede Substituentengruppe von R² gegebenenfalls unabhängig mit ein bis drei Substituentengruppen substituiert ist, ausgewählt aus Methoxy, Chlor, Brom oder Dimethylamino, oder ein Tautomer, Ester, Amid, Solvat oder Salz hiervon.

10. Verbindung der Formel (I) nach Anspruch 1, worin:
R² eine Phenylgruppe darstellt, substituiert mit drei Substituentengruppen,
worin jede Substituentengruppe von R² unabhängig C₁-C₅-Alkyl oder Halogen darstellt;
worin jede Substituentengruppe von R² gegebenenfalls unabhängig substituiert ist mit ein bis drei Substituentengruppen, ausgewählt aus C₁-C₅-Alkyl;
R³, R⁴, R⁵ und R⁶ jeweils unabhängig Wasserstoff oder Halogen darstellen;
R⁷ und R⁸ jeweils unabhängig Wasserstoff oder C₁-C₅-Alkyl sind, jedes gegebenenfalls substituiert mit ein bis drei Substituentengruppen,
wobei jede Substituentengruppe von R⁷ und R⁸ unabhängig C₁-C₅- Alkyl darstellt;
worin jede Substituentengruppe von R⁷ und R⁸ gegebenenfalls unabhängig substituiert ist mit ein bis drei Substituentengrup- pen, ausgewählt aus C₁-C₃-Alkyl; und
R⁹ und R¹⁰ jeweils Wasserstoff sind,
oder ein Tautomer, Ester, Amid, Solvat oder Salz hiervon.

11. Verbindung der Formel (I) nach Anspruch 1, worin jede Substituentengruppe von R² gegebenenfalls unabhängig mit drei Substituentengruppen substituiert ist, ausgewählt aus Methyl, Methoxy, Chlor, Brom oder Dimethylamino, oder ein Tautomer, Ester, Amid, Solvat oder Salz hiervon.

12. Verbindung der Formel (I) nach Anspruch 3, worin:
R² eine substituierte Phenyl- oder Naphthylgruppe darstellt,
oder ein Tautomer, Ester, Amid, Solvat oder Salz hiervon.

13. Verbindung der Formel (I) nach Anspruch 3, worin X und Y unsubstituiert sind, oder ein Tautomer, Ester, Amid, Solvat oder Salz hiervon.

14. Verbindung nach Anspruch 1, wobei die Verbindung ausgewählt ist aus:
4-tert-Butyl-N-[2-(1*H-*indol-3-yl)-1-methylethyl]benzolsulfonamid;
2,5-Dichlor-N-[2-(1*H-*indol-3-yl)-1-methylethyl]benzolsulfonamid;
(R)-2-(4-tert-Butylbenzolsulfonylamino)-3-(1*H-*indol-3-yl)propionsäuremethylester;
4-tert-Butyl-N-[2-(5-fluor-1*H-*indol-3-yl)-1-methylethyl]benzolsulfonamid;
4-tert-Butyl-N-[1-methyl-2-(1-methyl-1*H-*indol-3-yl)ethyl]benzolsulfonamid;
4-tert-Butyl-N*-*[1-hydroxymethyl-2-(1*H-*indol-3-yl)ethyl]benzolsulfonamid;
5-Fluor-N*-*[2-(1*H-*indol-3-yl)-1-methylethyl]-2-methoxybenzolsulfonamid;
5-Dimethylaminonaphthalin-1-sulfonsäure[2-(1*H-*indol-3-yl)-1-methylethyl]amid;
2,4,5-Trichlor-N-[2-(1*H-*indol-3-yl)-1-methylethyl]benzolsulfonamid;
N-[2-(1*H-*Indol-3-yl)-1-methylethyl]-2,4,6-trimethylbenzolsulfonamid;
N-[2-(1*H-*Indol-3-yl)-1-methylethyl]-2,3,4,5,6-pentamethylbenzolsulfonamid;
N-[2-(1*H-*Indol-3-yl)-1-methylethyl]-4-methoxy-2,3,6-trimethylbenzolsulfonamid;
3,4-Dichlor-N-[2-(1*H-*indol-3-yl)-1-methylethyl]benzolsulfonamid;
4-(1,1-Dimethylpropyl)-N-[2-(1*H-*indol-3-yl)-1-methylethyl]benzolsulfonamid;
N-[2-(1*H-*Indol-3-yl)-1-methylethyl]-4-isopropylbenzolsulfonamid;
2-Chlor-N-[2-(1*H-*indol-3-yl)-1-methylethyl]-6-methylbenzolsulfonamid;
3-Chlor-N-[2-(1*H-*indol-3-yl)-1-methylethyl]-2-methylbenzolsulfonamid;
2,6-Dichlor-N-[2-(1*H*-indol-3-yl)-1-methylethyl]benzenesulfonamid;
2,4-Dichlor-N-[2-(1*H-*indol-3-yl)-1-methylethyl]benzolsulfonamid;
5-Fluor-N-[2-(1*H-*indol-3-yl)-1-methylethyl]-2-methylbenzolsulfonamid;
2,4,6-Trichlor-N-[2-(1*H-*indol-3-yl)-1-methylethyl]benzolsulfonamid;
4-Butyl-N-[2-(1*H-*indol-3-yl)-1-methylethyl]benzolsulfonamid;
N-[2-(1*H-*Indol-3-yl)-1-methylethyl]-2,4,6-triisopropylbenzolsulfonamid;
2,5-Dibrom-3,6-difluor-N-[2-(1*H-*indol-3-yl)-1-methylethyl]benzolsulfonamid;
N*-*[2-(1*H-*Indol-3-yl)-1-methylethyl]-2,3,5,6-tetramethylbenzolsulfonamid;
4-tert-Butyl-N-[1-(1*H-*indol-3-ylmethyl)propyl]benzolsulfonamid;
2,4,6-Trichlor-N-[1-(1*H-*indol-3-ylmethyl)propyl]benzolsulfonamid;
4-tert-Butyl-N-[2-(5-methoxy-1*H-*indol-3-yl)-1-methylethyl]benzolsulfonamid;
2,4,6-Trichlor-N-[2-(5-methoxy-1*H-*indol-3-yl)-1-methylethyl]benzolsulfonamid;
4-tert-Butyl-N-[1-methyl-2-(2-methyl-1*H-*indol-3-yl)ethyl]benzolsulfonamid;
2,4,6-Trichlor-N-[1-methyl-2-(2-methyl-1*H-*indol-3-yl)ethyl]benzolsulfonamid;
4-tert-Butyl-N-[2-(1*H-*indol-3-yl)-2-methylpropyl]benzolsulfonamid;
2,4,6-Trichlor-N-[2-(1*H-*indol-3-yl)-2-methylpropyl]benzolsulfonamid;
N-[2-(5-Fluor-1*H-*indol-3-yl)-1-methylethyl]-2,4,6-trimethyl-benzolsulfonamid;
N-[1-Hydroxymethyl-2-(1*H-*indol-3-yl)ethyl]-2,4,6-trimethylbenzolsulfonamid;
2,4,6-Trimethyl-N*-*[1-methyl-2-(1-methyl-1*H-*indol-3-yl)ethyl]benzolsulfonamid;
(S)-3-(1*H-*Indol-3-yl)-2-(2,4,6-trimethylbenzolsulfonylamino)propionsäuremethylester;
(R)-3-(1*H-*Indol-3-yl)-2-(2,4,6-trimethylbenzolsulfonylamino)propionsäuremethylester;
N*-*[1-(1*H-*Indol-3-ylmethyl)propyl]-2,4,6-trimethylbenzolsulfonamid;
N*-*[2-(6-Fluor-1*H-*indol-3-yl)-1-methylethyl]-2,4,6-trimethylbenzolsulfonamid;
2,4,6-Trimethyl-N*-*[1-methyl-2-(7-methyl-1*H-*indol-3-yl)ethyl]benzolsulfonamid;
2,4,6-Trimethyl-N*-*[1-methyl-2-(6-methyl-1*H-*indol-3-yl)ethyl]benzolsulfonamid;
N*-*[2-(1*H-*Indol-3-yl)-1-methylethyl]-4-iodobenzolsulfonamid;
N*-*[2-(1*H-*Indol-3-yl)-1-methylethyl]-2,4-dinitrobenzolsulfonamid;
N*-*[2-(1*H-*Indol-3-yl)-1-methylethyl]-2-nitrobenzolsulfonamid;
3,5-Dichlor-2-hydroxy-N-[2-(1*H-*indol-3-yl)-1-methylethyl]benzolsulfonamid;
N-[2-(1*H-*Indol-3-yl)-1-methylethyl]-3-nitrobenzolsulfonamid;
4-Brom-N*-*[2-(1*H-*indol-3-yl)-l-methylethyl]benzolsulfonamid;
4-Fluor-N-[2-(1*H-*indol-3-yl)-1-methylethyl]benzolsulfonamid;
4-Chlor-N-[2-(1*H-*indol-3-yl)-1-methylethyl]benzolsulfonamid;
4-Chlor-N-[2-(1*H-*indol-3-yl)-1-methylethyl]-3-nitrobenzolsulfonamid;
N*-*{4-[2-(1*H-*Indol-3-yl)-1-methylethylsulfamoyl]phenyl}acetamid;
N*-*[1-(1*H-*Indol-3-yl)-1-methylethyl]-4-nitrobenzolsulfonamid;
N*-*[2-(1*H-*Indol-3-yl)-1-methylethyl]-4-methoxybenzolsulfonamid;
N*-*[2-(1*H*-Indol-3-yl)-1-methylethyl]-3-methylbenzolsulfonamid;
N*-*[2-(1*H-*Indol-3-yl)-1-methylethyl]-2-nitro-4-trifluormethylbenzolsulfonamid;
N*-*[2-(1*H*-Indol-3-yl)-1-methylethyl]-3-trifluormethylbenzolsulfonamid;
2,3,4-Trichlor-N*-*[2-(1*H-*indol-3-yl)-1-methylethyl]benzolsulfonamid;
N-[2-(1*H-*Indol-3-yl)-1-methylethyl]-2,5-dimethoxybenzolsulfonamid;
3,4-Dichlor-N-[2-(1*H-*indol-3-yl)-1-methylethyl]benzolsulfonamid;
3-Chlor-4-fluor-N-[2-(1*H-*indol-3-yl)-1-methylethyl]benzolsulfonamid;
4-Ethyl-N-[2-(1*H*-indol-3-yl)-1-methylethyl]benzolsulfonamid;
N*-*[2-(1*H-*Indol-3-yl)-1-methylethyl]-4-propylbenzolsulfonamid;
4-Brom-2,5-difluor-N-[2-(*H-*indol-3-yl)-1-methylethyl]benzolsulfonamid;
2-Fluor-N-[2-(1*H-*indol-3-yl)-1-methylethyl]benzolsulfonamid;
N-[2-(1*H-*Indol-3-yl)-1-methylethyl]-4-trifluormethoxybenzolsulfonamid;
N*-*[2-(1*H-*Indol-3-yl)-1-methylethyl]-4-trifluormethylbenzolsulfonamid;
2,4-Difluor-N*-*[2-(1*H-*indol-3-yl)-1-methylethyl]benzolsulfonamid;
4-Chlor-N*-*[2-(1*H-*indol-3-yl)-1-methylethyl]-2,5-dimethylbenzolsulfonamid;
2-Chlor-N-[2-(1*H-*indol-3-yl)-1-methylethyl]benzolsulfonamid;
N-[2-(1*H-*Indol-3-yl)-1-methylethyl]-2-methyl-5-nitrobenzolsulfonamid;
N-[2-(1*H-*Indol-3-yl)-1-methylethyl]-2-trifluormethylbenzolsulfonamid;
3 -Chlor-N-[2-(1*H-*indol-3-yl)-1-methylethyl]benzolsulfonamid;
5-Brom-N-[2-(1*H-*indol-3-yl)-1-methylethyl]-2-methoxybenzolsulfonamid;
N-[2-(1*H-*Indol-3-yl)-1-methylethyl]-3,4-dimethoxybenzolsulfonamid;
2,3-Dichlor-N**-**[2-(1-indol-3-yl)-1-methylethyl]benzolsulfonamid;
2-Brom-N-[2-(1*H-*indol-3-yl)-1-methylethyl]benzolsulfonamid;
3-Brom-N-[2-(1*H-*indol-3-yl)-1-methylethyl]benzolsulfonamid;
N-[2-(1*H-*Indol-3-yl)-1-methylethyl]-2-trifluormethoxybenzolsulfonamid;
3-Cyano-N-[2-(1*H-*indol-3-yl)-1-methylethyl]benzolsulfonamid;
2-Cyano-N*-*[2-(1*H-*indol-3-yl)-1-methylethyl]benzolsulfonamid;
N*-*[2-(1*H-*Indol-3-yl)-1-methylethyl]-2-methoxy-5-methylbenzolsulfonamid;
2-Chlor-4-fluor-N*-*[2-(1*H-* indol-3-yl)-1-methylethyl]benzolsulfonamid;
5-Chlor-N*-*[2-(1*H-*indol-3-yl)-1-methylethyl]-2-methoxybenzolsulfonamid;
Biphenyl-4-sulfonsäure[2-(1*H-*indol-3-yl)-1-methylethyl]amid;
2,6-Difluor-N*-*[2(1*H-*indol-3-yl)-1-methylethyl]benzolsulfonamid;
N-[2-(1*H-*Indol-3-yl)-1-methylethyl]-4-methoxy-3,5-dinitrobenzolsulfonamid;
N-[2-(1*H-*Indol-3-yl)-1-methylethyl]-4-methanesulfonylbenzolsulfonamid;
N-[2-(1*H-*Indol-3-yl)-1-methylethyl]-2-methanesulfonylbenzolsulfonamid;
4-Acetyl-N-[2-(1*H*-indol-3-yl)-1-methylethyl]benzolsulfonamid;
3,5-Dichlor-N-[2-(1*H-*indol-3-yl)-1-methylethyl]benzolsulfonamid; und
N-[2-(1*H-*Indol-3-yl)-1-methylethyl]-3,5-bis-trifluormethylbenzolsulfonamid;
oder ein Tautomer, Ester, Amid, Solvat oder Salz hiervon.

15. Verbindung nach Anspruch 1, wobei die Verbindung ausgewählt ist aus:
5-Dimethylaminonaphthalin-1-sulfonsäure[2-1*H-*indol-3-yl)-1-methylethyl]amid;
N-[2-(1*H-*Indol-3-yl)-1-methylethyl]-2,4,6-trimethylbenzolsulfonamid;
N-[2-(1*H-*Indol-3-yl)-1-methylethyl]-2,3,4,5,6-pentamethylbenzolsulfonamid;
N-[2-(1H-Indol-3-yl)-1-methylethyl]-4-methoxy-2,3,6-trimethylbenzolsulfonamid;
2-Chlor-N-[2-(2*H-*indol-3-yl)-1-methylethyl]-6-methylbenzolsulfonamid;
2,6-Dichlor-N-[2-(1*H-*indol-3-yl)-1-methylethyl]benzolsulfonamid;
2,4,6-Trichlor-N-[2-(1*H-*indol-3-yl)-1-methylethyl]benzolsulfonamid;
N-[2-(1*H-*Indol-3-yl)-1-methylethyl]-2,4,6-triisopropylbenzolsulfonamid;
4-tert-Butyl-N-[1-(1*H-*indol-3-ylmethyl)propyl]benzolsulfonamid;
2,4,6-Trichlor-N-[1-(1*H-*indol-3-ylmethyl)propyl]benzolsulfonamid;
N-[2-(5-Fluor-1*H-*indol-3-yl)-1-methylethyl]-2,4,6-trimethylbenzolsulfonamid;
N-[1-(1*H-*Indol-3-ylmethyl)propyl]-2,4,6-trimethylbenzolsulfonamid;
N-[2-(6-Fluor-1*H-*indol-3-yl)-1-methylethyl]-2,4,6-trimethylbenzolsulfonamid;
2,4,6-Trimethyl-N-[1-methyl-2-(6-methyl-1*H-*indol-3-yl)ethyl]benzolsulfonamid; und
2,4,6-Trimethyl-N-[1-methyl-2-(7-methyl-1*H-*indol-3-yl)ethyl]benzolsulfonamid
oder ein Tautomer, Ester, Amid, Solvat oder Salz hiervon.

16. Verbindung nach Anspruch 1, wobei die Verbindung ausgewählt ist aus:
N-[2-(1*H-*Indol-3-yl)-1-methylethyl]-2,4,6-trimethylbenzolsulfonamid;
2,4,6-Trichlor-N-[2-(1*H-*indol-3-yl)-1-methylethyl]benzolsulfonamid;
N-[2-(1*H-*Indol-3-yl)-1-methylethyl]-2,4,6-triisopropylbenzolsulfonamid;
2,4,6-Trichlor-N-[1-(1*H-*indol-3-ylmethyl)propyl]benzolsulfonamid;
N-[2-(5-Fluor-1*H-*indol-3-yl)-1-methylethyl]-2,4,6-trimethylbenzolsulfonamid;
N-[1-(1*H-*Indol-3-ylmethyl)propyl]-2,4,6-trimethylbenzolsulfonamid;
N-[2-(6-Fluor-1*H-*indol-3-yl)-1-methylethyl]-2,4,6-trimethylbenzolsulfonamid; und
2,4,6-Trimethyl-N-[1-methyl-2-(6-methyl-1*H-*indol-3-yl)ethyl]benzolsulfonamid
oder ein Tautomer, Ester, Amid, Solvat oder Salz hiervon.

17. Pharmazeutische Zusammensetzung, umfassend eine wirksame Menge einer Verbindung nach irgendeinem der Ansprüche 1 bis 16 oder eines Tautomers, Esters, Amids, Solvats oder Salzes hiervon, und einen pharmazeutisch akzeptablen Hilfsstoff oder Träger.

18. Verwendung einer Verbindung nach irgendeinem der Ansprüche 1 bis 16 oder eines Tautomers, Esters, Amids, Solvats oder Salzes hiervon zur Herstellung einer pharmazeutischen Zusammensetzung, um die Glucocorticoidrezeptorfunktion bei einem Patienten zu modulieren.

19. Verwendung einer Verbindung nach irgendeinem der Ansprüche 1 bis 16 oder eines Tautomers, Esters, Amids, Solvats oder Salzes hiervon zur Herstellung einer pharmazeutischen Zusammensetzung, um einen Krankheitszustand oder einen Zustand zu behandeln, der durch die Glucocorticoidfunktion bei einem Patienten vermittelt wird.

20. Verwendung einer Verbindung nach irgendeinem der Ansprüche 1 bis 16 oder eines Tautomers, Esters, Amids, Solvats oder Salzes hiervon zur Herstellung einer pharmazeutischen Zusammensetzung, um einen Krankheitszustand oder einen Zustand zu behandeln, ausgewählt aus Diabetes Typ II, Obesität, kardiovaskulären Erkrankungen, Bluthochdruck, Arteriosklerose, neurologischen Erkrankungen, adrenalen und pituitären Tumoren und Glaukom bei einem Patienten.

21. Verwendung einer Verbindung nach irgendeinem der Ansprüche 1 bis 16 oder eines Tautomers, Esters, Amids, Solvats oder Salzes hiervon zur Herstellung einer pharmazeutischen Zusammensetzung zur Behandlung einer Erkrankung, charakterisiert durch inflammatorische, allergische oder proliferative Prozesse bei einem Patienten.

22. Verwendung nach Anspruch 21, wobei die Erkrankung ausgewählt ist aus: (i) Lungenerkrankungen; (ii) rheumatischen Erkrankungen/Autoimunerkrankungen/Gelenkserkrankungen; (iii) allergischen Erkrankungen; (iv) Vaskulitiserkrankungen; (v) dermatologischen Erkrankungen; (vi) renalen Erkrankungen; (vii) hepatischen Erkrankungen; (viii) gastrointestinalen Erkrankungen; (ix) proktologischen Erkrankungen; (x) Augenerkrankungen; (xi) Erkrankungen des HNO-Bereichs (Hals-Nasen-Ohren); (xii) neurologischen Erkrankungen; (xiii) Bluterkrankungen; (xiv) Tumorerkrankungen; (xv) endokrinen Erkrankungen; (xvi) Organ- und Gewebetransplantationen und Graft-versus-Host-Erkrankungen; (xvii) schweren Schockzuständen; (xviii) Substitutionstherapie und (xix) Schmerzen aus inflammatorischer Genese.

23. Verwendung nach Anspruch 21, wobei die Erkrankung ausgewählt ist aus: Diabetes Typ I, Osteoarthritis, Guillan-Barre-Syndrom, Restenose infolge perkutaner transluminaler Koronarangioplastie, Alzheimer Erkrankung, akutem und chronischem Schmerz, Arteriosklerose, Reperfusionsverletzung, Knochenresorptionserkrankungen, kongestivem Herzfehler, Myokardinfarkt, thermischen Verletzungen, multiple Organverletzungen nach Trauma, akuter purulenter Meningitis, nekrotisierender Enterocolitis und mit Hämodialyse, Leukophorese und Granulocyttransfusion in Verbindung stehenden Syndromen.

24. Verwendung (a) einer Verbindung nach irgendeinem der Ansprüche 1 bis 16 oder eines Tautomers, Esters, Amids, Solvats oder Salzes hiervon; und (b) einem Glucocortikoid zur Herstellung einer pharmazeutischen Zusammensetzung zur Behandlung eines Krankheitszustands oder Zustands, die durch den Glucocortikoidrezeptorfunktion vermittelt wird, bei einem Patienten.

25. Kit zur diagnostischen in vitro Bestimmung der Glucocortikoidrezeptorfunktion in einer Probe, umfassend:
(a) eine diagnostisch wirksame Menge einer Verbindung nach Anspruch 1 oder eines Tautomers, Esters, Amids, Solvats oder Salzes hiervon; und
(b) Anweisungen für die Verwendung des diagnostischen Kits.

26. Verfahren zur Herstellung einer Verbindung der Formel (I) 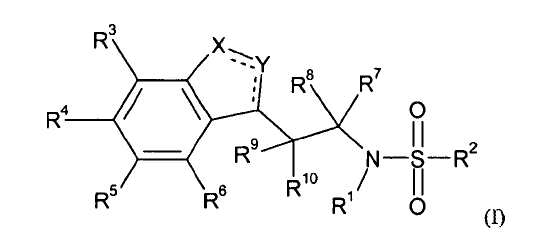 worin R¹ H darstellt und R², R³, R⁴, R⁵, R⁶, R⁷, R⁸, R⁹, R¹⁰, X und Y wie in Anspruch 1 definiert sind, wobei das Verfahren umfasst: Umsetzen des Aminoethylheterocyclus der Formel (II) mit einem Sulfonylhalogenid der Formel (III) in Gegenwart einer geeigneten Base, um die Verbindung der Formel (I) zu ergeben 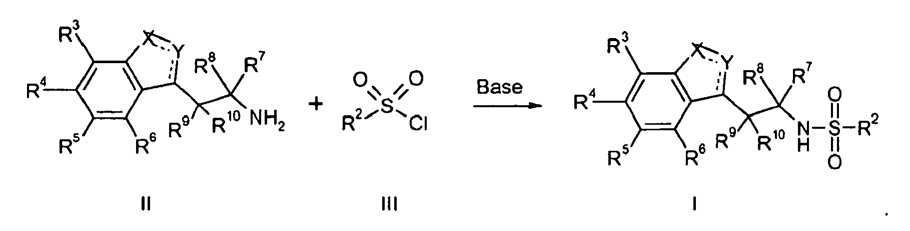 27. Verfahren zur Herstellung einer Verbindung der Formel (I) 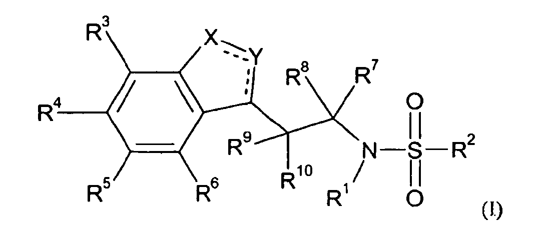 worin R¹, R⁸ und R⁹ jeweils H darstellen und R², R³, R⁴, R⁵, R⁶, R⁷, R¹⁰, X und Y wie in Anspruch 1 definiert sind, wobei das Verfahren umfasst:
(a) Umsetzen des Aldehyds der Formel (V) mit der Nitroverbindung der Formel (VI) in Gegenwart von Ammoniumacetat und Essigsäure, um das Nitroalken der Formel (VII) zu bilden 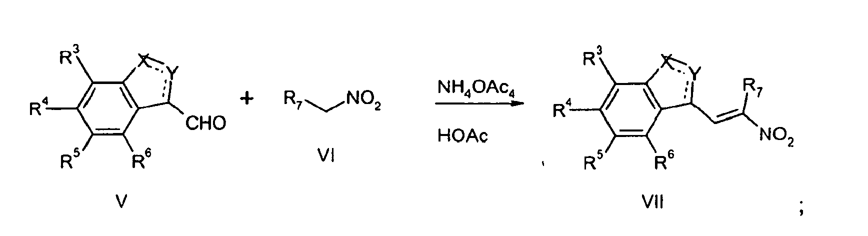
(b) Reduzieren des Nitroalkens der Formel (VII) mit einem geeigneten Reduktionsmittel, um die Zwischenverbindung der Formel (II) zu bilden 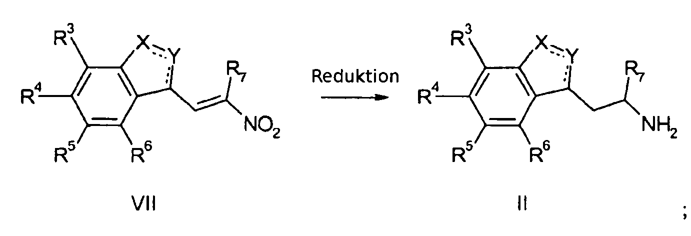 und
(c) Umsetzen der Zwischenverbindung der Formel (II) mit einem Sulfonylhalogenid der Formel (III) in Gegenwart einer geeigneten Base, um die Verbindung der Formel (I) zu bilden

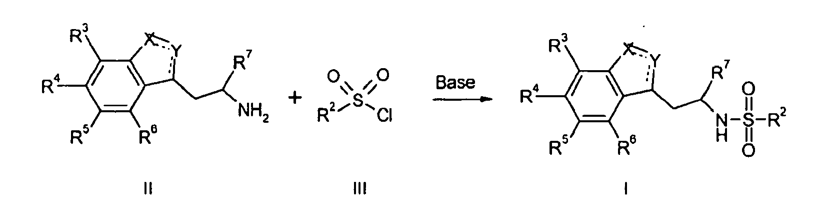

## Revendications

1. Composé de formule (I) 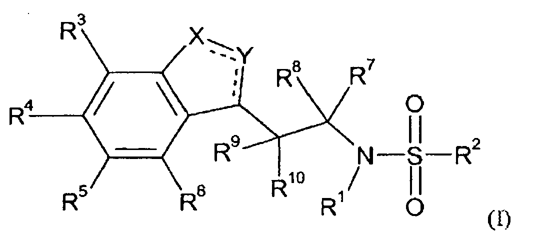 où :
R¹ est l'hydrogène ;
R² est un groupe aryle substitué avec un à cinq groupes substituants à condition que le groupe -SO₂-R² ne soit pas un groupe p-toluènesulfonyle, où chaque groupe substituant de R² est indépendamment C₁-C₅ alkyle, C₂-C₅ alcényle, C₂-C₅ alcynyle, C₃-C₈ cycloalkyle, hétérocyclyle, aryle, hétéroaryle, C₁-C₅ alcoxy, C₂-C₅ alcényloxy, C₂-C₅ alcynyloxy, aryloxy, acyle, C₁-C₅ alcoxycarbonyle, C₁-C₅ alcanoyloxy, aminocarbonyle, C₁-C₅ alkylaminocarbonyle, C₁-C₅ dialkylaminocarbonyle, aminocarbonyloxy, C₁- C₅ alkylaminocarbonyloxy, C₁-C₅ dialkylaminocarbonyloxy, C₁-C₅ alcanoylamino, C₁-C₅ alcoxycarbonylamino, C₁-C₅ alkylsulfonylamino, C₁- C₅ alkylaminosulfonyle, C₁-C₅ dialkylaminosulfonyle, halogène, hydroxy, carboxy, cyano, trifluorométhyle, trifluorométhoxy, nitro ou amino où l'atome d'azote est éventuellement indépendamment mono- ou disubstitué par C₁-C₅ alkyle ou aryle ; ou uréido où chaque atome d'azote est éventuellement substitué indépendamment avec C₁-C₅ alkyle ; ou C₁-C₅ alkylthio où l'atome de soufre est éventuellement oxydé en un sulfoxyde ou une sulfone ;
où chaque groupe substituant de R² est éventuellement substitué indépendamment avec un à trois groupes substituants choisis parmi C₁-C₅ alkyle, C₁-C₅ alcoxy, halogène, hydroxy, oxo, cyano ou amino où l'atome d'azote est éventuellement indépendamment mono- ou disubstitué par C₁-C₅ alkyle,
R³, R⁴, R⁵ et R⁶ sont chacun indépendamment l'hydrogène ou C₁-C₅ alkyle, C₂-C₅ alcényle, C₂-C₅ alcynyle, C₃-C₈ cycloalkyle, hétérocyclyle, aryle, hétéroaryle, C₁-C₅ alcoxy, C₂-C₅ alcényloxy, C₂-C₅ alcynyloxy, aryloxy, acyle, C₁-C₅ alcoxycarbonyle, C₁-C₅ alcanoyloxy, aminocarbonyle, C₁-C₅ alkylaminocarbonyle, C₁-C₅ dialkylaminocarbonyle, aminocarbonyloxy, C₁- C₅ alkylaminocarbonyloxy, C₁-C₅ dialkylaminocarbonyloxy, C₁-C₅ alcanoylamino, C₁-C₅ alcoxycarbonylamino, C₁-C₅ alkylsulfonylamino, C₁- C₅ alkylaminosulfonyle, C₁-C₅ dialkylaminosulfonyle, halogène, hydroxy, carboxy, cyano, trifluorométhyle, trifluorométhoxy, trifluorométhylthio, nitro ou amino où l'atome d'azote est éventuellement indépendamment mono- ou disubstitué par C₁-C₅ alkyle ; ou uréido où chaque atome d'azote est éventuellement substitué indépendamment avec C₁-C₅ alkyle ; ou C₁-C₅ alkylthio où l'atome de soufre est éventuellement oxydé en un sulfoxyde ou une sulfone,
où R³, R⁴, R⁵ et R⁶ sont chacun éventuellement substitués indépendamment avec un à trois groupes substituants choisis parmi C₁-C₃ alkyle, C₁-C₃ alcoxy, halogène, hydroxy, oxo, cyano, amino ou trifluorométhyle ;
R⁷ et R⁸ sont chacun indépendamment l'hydrogène ou C₁-C₅ alkyle, chacun éventuellement substitué avec un à trois groupes substituants,
où chaque groupe substituant de R⁷ et R⁸ est indépendamment C₁-C₅ alkyle, C₂-C₅ alcényle, C₂-C₅ alcynyle, C₃-C₈ cycloalkyle, hétérocyclyle, aryle, hétéroaryle, C₁-C₅ alcoxy, C₂-C₅ alcényloxy, C₂-C₅ alcynyloxy, aryloxy, acyle, C₁-C₅ alcoxycarbonyle, C₁-C₅ alcanoyloxy, aminocarbonyle, C₁-C₅ alkylaminocarbonyle, C₁-C₅ dialkylaminocarbonyle, aminocarbonyloxy, C₁- C₅ alkylaminocarbonyloxy, C₁-C₅ dialkylaminocarbonyloxy, C₁-C₅ alcanoylamino, C₁-C₅ alcoxycarbonylamino, C₁-C₅ alkylsulfonylamino, C₁- C₅ alkylaminosulfonyle, C₁-C₅ dialkylaminosulfonyle, halogène, hydroxy, carboxy, cyano, trifluorométhyle, trifluorométhoxy, trifluorométhylthio, nitro ou amino où l'atome d'azote est éventuellement indépendamment mono- ou disubstitué par C₁-C₅ alkyle ; ou uréido où chaque atome d'azote est éventuellement substitué indépendamment avec C₁-C₅ alkyle ; ou C₁-C₅ alkylthio où l'atome de soufre est éventuellement oxydé en un sulfoxyde ou une sulfone,
où chaque groupe substituant de R⁷ et R⁸ est éventuellement substitué indépendamment avec un à trois groupes substituants choisis parmi C₁-C₃ alkyle, C₁-C₃ alcoxy, halogène, hydroxy, oxo, cyano, amino ou trifluorométhyle,
R⁹ et R¹⁰ sont chacun indépendamment l'hydrogène ou C₁-C₅ alkyle, chacun éventuellement substitué avec un à trois groupes substituants, où chaque groupe substituant de R⁹ et R¹⁰ est indépendamment C₁-C₅ alkyle, C₂-C₅ alcényle, C₂-C₅ alcynyle, C₃-C₈ cycloalkyle, hétérocyclyle, aryle, hétéroaryle, C₁-C₅ alcoxy, C₂-C₅ alcényloxy, C₂-C₅ alcynyloxy, aryloxy, acyle, C₁-C₅ alcoxycarbonyle, C₁-C₅ alcanoyloxy, aminocarbonyle, C₁-C₅ alkylaminocarbonyle, C₁-C₅ dialkylaminocarbonyle, aminocarbonyloxy, C₁- C₅ alkylaminocarbonyloxy, C₁-C₅ dialkylaminocarbonyloxy, C₁-C₅ alcanoylamino, C₁-C₅ alcoxycarbonylamino, C₁-C₅ alkylsulfonylamino, C₁- C₅ alkylaminosulfonyle, C₁-C₅ dialkylaminosulfonyle, halogène, hydroxy, carboxy, cyano, trifluorométhyle, trifluorométhoxy, trifluorométhylthio, nitro ou amino où l'atome d'azote est éventuellement indépendamment mono- ou disubstitué par C₁-C₅ alkyle ; ou uréido où chaque atome d'azote est éventuellement substitué indépendamment avec C₁-C₅ alkyle ; ou C₁-C₅ alkylthio où l'atome de soufre est éventuellement oxydé en un sulfoxyde ou une sulfone,
où chaque groupe substituant de R⁹ et R¹⁰ est éventuellement substitué indépendamment avec un à trois groupes substituants choisis parmi C₁-C₃ alkyle, C₁-C₃ alcoxy, halogène, hydroxy, oxo, cyano, amino ou trifluorométhyle,
où au moins l'un de R⁷, R⁸, R⁹ et R¹⁰ est C₁-C₅ alkyle éventuellement substitué ;
X est un groupe NH éventuellement substitué avec un groupe substituant si aucune liaison éventuelle avec X n'est présente ; et
Y est un groupe CH₂ éventuellement substitué avec un ou deux groupes substituants, ou
est un groupe CH éventuellement substitué avec un groupe substituant si une liaison éventuelle avec Y est présente,
où chaque groupe substituant de X et Y est indépendamment C₁-C₃ alkyle, C₁-C₃ alcoxy, halogène, hydroxy, oxo, cyano, amino ou trifluorométhyle,
où chaque trait pointillé représente une liaison éventuelle, à condition que zéro ou l'une des liaisons éventuelles soit présente dans le composé de formule (I),
ou tautomère, ester, amide, solvate ou sel de celui-ci.

2. Composé de formule (I) selon la revendication 1 où R² est un groupe aryle substitué.

3. Composé de formule (I) selon la revendication 1 ou la revendication 2 où X est un groupe NH et Y est un groupe CH, ou tautomère, ester, amide, solvate ou sel de celui-ci.

4. Composé de formule (I) selon la revendication 3 où :
R² est un groupe phényle ou naphtyle, chacun substitué avec un à cinq groupes substituants,
où chaque groupe substituant de R² est indépendamment C₁-C₅ alkyle, C₂-C₅ alcényle, C₂-C₅ alcynyle, C₃-C₈ cycloalkyle, hétérocyclyle, phényle, naphtyle, hétéroaryle, C₁-C₅ alcoxy, C₂-C₅ alcényloxy, C₂-C₅ alcynyloxy, aryloxy, trifluorométhyle, trifluorométhoxy, halogène ou amino où l'atome d'azote est éventuellement indépendamment mono- ou disubstitué par C₁-C₅ alkyle ou aryle, ou C₁-C₅ alkylthio ;
où chaque groupe substituant de R² est éventuellement substitué indépendamment avec un à trois groupes substituants choisis parmi C₁-C₅ alkyle, C₁-C₅ alcoxy, halogène, hydroxy, oxo, cyano ou amino où l'atome d'azote est éventuellement indépendamment mono- ou disubstitué par C₁-C₅ alkyle,
R³, R⁴, R⁵ et R⁶ sont chacun indépendamment l'hydrogène ou C₁-C₅ alkyle, C₂-C₅ alcényle, C₂-C₅ alcynyle, C₃-C₈ cycloalkyle, C₁-C₅ alcoxy, C₂-C₅ alcényloxy, C₂-C₅ alcynyloxy, aryloxy, acyle, C₁-C₅ alcoxycarbonyle, C₁-C₅ alcanoyloxy, halogène, hydroxy, carboxy, cyano, trifluorométhyle, trifluorométhoxy, trifluorométhylthio ou C₁-C₅ alkylthio où l'atome de soufre est éventuellement oxydé en un sulfoxyde ou une sulfone ;
R⁷ et R⁸ sont chacun indépendamment l'hydrogène ou C₁-C₅ alkyle, chacun éventuellement substitué avec un à trois groupes substituants,
où chaque groupe substituant de R⁷ et R⁸ est indépendamment C₁-C₅ alkyle, C₂-C₅ alcényle, C₂-C₅ alcynyle, C₃-C₈ cycloalkyle, aryle, halogène, trifluorométhyle, trifluorométhoxy, trifluorométhylthio ou C₁-C₅ alkylthio où l'atome de soufre est éventuellement oxydé en un sulfoxyde ou une sulfone ;
où chaque groupe substituant de R⁷ et R⁸ est éventuellement substitué indépendamment avec un à trois groupes substituants choisis parmi C₁-C₃ alkyle, C₁-C₃ alcoxy, halogène, hydroxy, oxo, cyano, amino ou trifluorométhyle,
R⁹ et R¹⁰ sont chacun l'hydrogène ou C₁-C₅ alkyle, chacun éventuellement substitué avec un à trois groupes substituants,
où chaque groupe substituant de R⁹ et R¹⁰ est indépendamment C₁-C₅ alkyle, C₂-C₅ alcényle, C₂-C₅ alcynyle, C₃-C₈ cycloalkyle, aryle, halogène, trifluorométhyle, trifluorométhoxy, trifluorométhylthio ou C₁-C₅ alkylthio où l'atome de soufre est éventuellement oxydé en un sulfoxyde ou une sulfone,
où chaque groupe substituant de R⁹ et R¹⁰ est éventuellement substitué indépendamment avec un à trois groupes substituants choisis parmi C₁-C₃ alkyle, C₁-C₃ alcoxy, halogène, hydroxy, oxo, cyano, amino ou trifluorométhyle,
ou tautomère, ester, amide, solvate ou sel de celui-ci.

5. Composé de formule (I) selon la revendication 3 où :
R² est un groupe phényle substitué avec un à trois groupes substituants, où chaque groupe substituant de R² est indépendamment C₁-C₅ alkyle ou halogène,
R³, R⁴, R⁵ et R⁶ sont chacun indépendamment l'hydrogène ou halogène ;
R⁷ et R⁸ sont chacun indépendamment l'hydrogène ou C₁-C₅ alkyle, chacun éventuellement substitué avec un à trois groupes substituants, où chaque groupe substituant de R⁷ et R⁸ est indépendamment C₁-C₅ alkyle,
où chaque groupe substituant de R⁷ et R⁸ est éventuellement substitué indépendamment avec un à trois groupes substituants choisis parmi C₁-C₃ alkyle ; et
R⁹ et R¹⁰ sont chacun l'hydrogène,
ou tautomère, ester, amide, solvate ou sel de celui-ci.

6. Composé de formule (I) selon la revendication 3 où au moins l'un de R⁷, R⁸, R⁹ et R¹⁰ est C₁-C₅ alkyle, ou tautomère, ester, amide, solvate ou sel de celui-ci.

7. Composé de formule (I) selon la revendication 3 où au moins deux de R⁷, R⁸, R⁹ et R¹⁰ sont C₁-C₅ alkyle, ou tautomère, ester, amide, solvate ou sel de celui-ci.

8. Composé de formule (I) selon la revendication 3 où au moins trois de R⁷, R⁸, R⁹ et R¹⁰ sont C₁-C₅ alkyle, ou tautomère, ester, amide, solvate ou sel de celui-ci.

9. Composé de formule (I) selon la revendication 3 où chaque groupe substituant de R² est éventuellement substitué indépendamment avec un à trois groupes substituants choisis parmi méthoxy, chloro, bromo et diméthylamino, ou tautomère, ester, amide, solvate ou sel de celui-ci.

10. Composé de formule (I) selon la revendication 1 où :
R² est un groupe phényle substitué avec trois groupes substituants, où chaque groupe substituant de R² est indépendamment C₁-C₅ alkyle ou halogène,
où chaque groupe substituant de R² est éventuellement substitué indépendamment avec un à trois groupes substituants choisis parmi C₁-C₅ alkyle ;
R³, R⁴, R⁵ et R⁶ sont chacun indépendamment l'hydrogène ou halogène ;
R⁷ et R⁸ sont chacun indépendamment l'hydrogène ou C₁-C₅ alkyle, chacun éventuellement substitué avec un à trois groupes substituants,
où chaque groupe substituant de R⁷ et R⁸ est indépendamment C₁-C₅ alkyle, où chaque groupe substituant de R⁷ et R⁸ est éventuellement substitué indépendamment avec un à trois groupes substituants choisis parmi C₁-C₃ alkyle ; et
R⁹ et R¹⁰ sont chacun l'hydrogène,
ou tautomère, ester, amide, solvate ou sel de celui-ci.

11. Composé de formule (I) selon la revendication 1 où chaque groupe substituant de R² est éventuellement substitué indépendamment avec trois groupes substituants choisis parmi méthyle, méthoxy, chloro, bromo et diméthylamino, ou tautomère, ester, amide, solvate ou sel de celui-ci.

12. Composé de formule (I) selon la revendication 3 où :
R² est un groupe phényle ou naphtyle substitué, ou tautomère, ester, amide, solvate ou sel de celui-ci.

13. Composé de formule (I) selon la revendication 3 où X et Y sont non substitués, ou tautomère, ester, amide, solvate ou sel de celui-ci.

14. Composé selon la revendication 1, le composé choisi parmi :
4-*tert*-butyl-*N-*[2-(1*H-*indol-3-yl)-1-méthyléthyl]benzènesulfonamide;
2,5-dichloro-*N-*[2-(1*H-*indol-3-yl)-1-méthyléthyl]benzènesulfonamide;
(*R*)-2-(4-*tert*-butylbenzènesulfonylamino)-3-(1*H-*indol-3-yl)propionate de méthyle;
4-*tert*-butyl-*N*-[2-(5-fluoro-1*H-*indol-3-yl)-1-méthyléthyl]benzènesulfonamide;
4-*tert*-butyl-*N-*[1-méthyl-2-(1-méthyl-1*H-*indol-3-yl)éthyl]benzènesulfonamide;
4-*tert*-butyl-*N-*[1-hydroxyméthyl-2-(1*H-*indol-3-yl)éthyl]benzènesulfonamide;
5-fluoro-*N-*[2-(1*H-*indol-3-yl)-1-méthyléthyl]-2-méthoxybenzènesulfonamide;
[2-(1*H-*indol-3 -yl)-1-méthyléthyl]amide d'acide 5-diméthylaminonaphtalène-1-sulfonique ;
2,4,5- trichloro-*N-*[2-(1*H-*indol-3-yl)-1-méthyléthyl]benzènesulfonamide;
*N-*[2-(1*H-*indol-3-yl)-1-méthyléthyl]-2,4,6-triméthylbenzènesulfonamide;
*N-*[2-(1*H-*indol-3-yl)-1-méthyléthyl]-2,3,4,5,6-pentaméthylbenzènesulfonamide;
*N-*[2-(1*H-*indol-3-yl)-1-méthyléthyl]-4-méthoxy-2,3,6-triméthylbenzènesulfonamide;
3,4-dichloro-*N-*[2-(1*H-*indol-3-yl)-1-méthyléthyl]benzènesulfonamide;
4-(1,1-diméthylpropyl)-*N-*[2-(1*H-*indol-3-yl)-1-méthyléthyl]benzènesulfonamide;
*N-*[2-(1*H-*indol-3-yl)-1-méthyléthyl]-4-isopropylbenzènesulfonamide;
2-chloro-*N-*[2-(1*H-*indol-3-yl)-1-méthyléthyl]-6-méthylbenzènesulfonamide;
3-chloro-*N-*[2-(1*H-*indol-3-yl)-1-méthyléthyl]-2-méthylbenzènesulfonamide;
2,6-dichloro-*N-*[2-(1*H-*indol-3-yl)-1-méthyléthyl]benzènesulfonamide;
2,4-dichloro-*N-*[2-(1*H-*indol-3-yl)-1-méthyléthyl]benzènesulfonamide;
5-fluoro-*N-*[2-(1*H-*indol-3-yl)-1-méthyléthyl]-2-méthylbenzènesulfonamide;
2,4,6-trichloro-*N-*[2-(1*H-*indol-3-yl)-1-méthyléthyl]benzènesulfonamide;
4-butyl-*N-*[2-(1*H-*indol-3-yl)-1-méthyléthyl]benzènesulfonamide;
*N-*[2-(1*H-*indol-3-yl)-1-méthyléthyl]-2,4,6-triisopropylbenzènesulfonamide;
2,5-dibromo-3,6-difluoro-*N-*[2-(1*H-*indol-3-yl)-1-méthyléthyl]benzènesulfonamide;
*N-*[2-(1*H-*indol-3-yl)-1-méthyléthyl]-2,3,5,6-tétraméthylbenzènesulfonamide;
*4-tert-*butyl*-N-*[1-(1*H-*indol-3-ylméthyl)propyl]benzènesulfonamide;
2,4,6-trichloro-*N-*[1-(1*H-*indol-3-ylméthyl)propyl]benzènesulfonamide;
4-*tert*-butyl-*N-*[2-(5-méthoxy-1*H-*indol-3-yl)-1-méthyléthyl]benzènesulfonamide;
2,4,6-trichloro-*N-*[2-(5-méthoxy-1*H-*indol-3-yl)-1-méthyléthyl]benzènesulfonamide;
4-*tert*-butyl-*N-*[1-méthyl-2-(2-méthyl-1*H-*indol-3-yl)éthyl]benzènesulfonamide;
2,4,6-trichloro-*N*-[1-méthyl-2-(2-méthyl-1*H-*indol-3-yl)éthyl]benzènesulfonamide;
4-*tert*-butyl-*N-*[2-(1H indol-3-yl)-2-méthylpropyl]benzènesulfonamide;
2,4,6-trichloro-*N-*[2-(1*H-*indol-3-yl)-2-méthylpropyl]benzènesulfonamide;
*N*-[2-(5-fluoro-1*H-*indol-3-yl)-1-méthyléthyl]-2,4,6-triméthyl-benzènesulfonamide;
*N-*[1-hydroxyméthyl-2-(1*H-*indol-3-yl)éthyl]-2,4,6-triméthylbenzènesulfonamide;
2,4,6-triméthyl-*N*-[1-méthyl-2-(1-méthyl-1*H*-indol-3-yl)éthyl]benzènesulfonamide;
(*S*)-3-(1*H-*indol-3-yl)-2-(2,4,6-triméthylbenzènesulfonylamino)propionate de méthyle ;
(*R*)-3-(1*H-*indol-3-yl)-2-(2,4,6-triméthylbenzènesulfonylamino)propionate de méthyle ;
*N-*[1-(1*H-*indol-3-ylméthyl)propyl]-2,4,6-triméthylbenzènesulfonamide;
*N-*[2-(6-fluoro-1*H-*indol-3-yl)-1-méthyléthyl]-2,4,6-triméthylbenzènesulfonamide;
2,4,6-triméthyl-*N-*[1-méthyl-2-(7-méthyl-1*H-*indol-3-yl)éthyl]benzènesulfonamide;
2,4,6-triméthyl-*N-*[1-méthyl-2-(6-méthyl-1*H-*indol-3-yl)éthyl]benzènesulfonamide;
*N-*[2-(1*H-*indol-3-yl)-1-méthyléthyl]-4-iodobenzènesulfonamide;
*N-*[2-(1*H-*indol-3-yl)-1-méthyléthyl]-2,4-dinitrobenzènesulfonamide;
*N-*[2-(1*H-*indol-3-yl)-1-méthyléthyl]-2-nitrobenzènesulfonamide;
3,5-dichloro-2-hydroxy-*N-*[2-(1*H-*indol-3-yl)-1-méthyléthyl]benzènesulfonamide;
*N-*[2-(1*H-*indol-3-yl)-1-méthyléthyl]-3-nitrobenzènesulfonamide;
4-bromo-*N-*[2-(1*H-*indol-3-yl)-1-méthyléthyl]benzènesulfonamide;
4-fluoro-*N-*[2-(1*H-*indol-3-yl)-1-méthyléthyl]benzènesulfonamide;
4-chloro-*N-*[2-(1*H-*indol-3-yl)-1-méthyléthyl]benzènesulfonamide;
4-chloro-*N-*[2-(1*H-*indol-3-yl)-1-méthyléthyl]-3-nitrobenzènesulfonamide;
*N-*{4-[2-(1*H-*indol-3-yl)-1-méthyléthylsulfamoyl]phényl}acétamide;
*N-*[2-(1*H-*indol-3-yl)-1-méthyléthyl]-4-nitrobenzènesulfonamide;
*N-*[2-(1*H-*indol-3-yl)-1-méthyléthyl]-4-méthoxybenzènesulfonamide;
*N-*[2-(1*H-*indol-3-yl)-1-méthyléthyl]-3-méthylbenzènesulfonamide;
*N-*[2-(1*H-*indol-3-yl)-1-méthyléthyl]-2-nitro-4-trifluorométhylbenzènesulfonamide;
*N-*[2-(1*H-*indol-3-yl)-1-méthyléthyl]-3-tritluorométhylbenzènesulfonamide;
2,3,4-trichloro-*N-*[2-(1*H-*indol-3-yl)-1-méthyléthyl]benzènesulfonamide;
*N-*[2-(1*H-*indol-3-yl)-1-méthyléthyl]-2,5-diméthoxybenzènesulfonamide;
3,4-dichloro-*N-*[2-(1*H-*indol-3-yl)-1-méthyléthyl]benzènesulfonamide;
3-chloro-4-fluoro-*N-*[2-(1*H-*indol-3-yl)-1-méthyléthyl]benzènesulfonamide;
4-éthyl-*N-*[2-(1*H-*indol-3-yl)-1-méthyléthyl]benzènesulfonamide;
*N-*[2-(1*H-*indol-3-yl)-1-méthyléthyl]-4-propylbenzènesulfonamide;
4-bromo-2,5-difluoro-*N-*[2-(1*H-*indol-3-yl)-1-méthyléthyl]benzènesulfonamide;
2-fluoro-*N-*[2-(1*H-*indol-3-yl)-1-méthyléthyl]benzènesulfonamide;
*N-*[2-(1*H-*indol-3-yl)-1-méthyléthyl]-4-trifluorométhoxybenzènesulfonamide;
*N-*[2-(1*H-*indol-3-yl)-1-méthyléthyl]-4-tritluorométhylbenzènesulfonamide;
2,4-difluoro-*N-*[2-(1*H-*indol-3-yl)-1-méthyléthyl]benzènesulfonamide;
4-chloro-*N-*[2-(1*H-*indol-3-yl)-1-méthyléthyl]-2,5-diméthylbenzènesulfonamide;
2-chloro-*N-*[2-(1*H-*indol-3-yl)-1-méthyléthyl]benzènesulfonamide;
N*-*[2-(1*H-*indol-3-yl)-1-méthyléthyl]-2-méthyl-5-nitrobenzènesulfonamide;
*N-*[2-(1*H-*indol-3-yl)-1-méthyléthyl]-2-trifluorométhylbenzènesulfonamide;
3-chloro-*N-*[2-(1*H-*indol-3-yl)-1-méthyléthyl]benzènesulfonamide;
5-bromo-*N-*[2-(1*H-*indol-3-yl)-1-méthyléthyl]-2-méthoxybenzènesulfonamide;
*N-*[2-(1*H-*indol-3-yl)-1-méthyléthyl]-3,4-diméthoxybenzènesulfonamide;
2,3-dichloro-N-[2-(1*H-*indol-3-yl)-1-méthyléthyl]benzènesulfonamide;
2-bromo-*N*-[2-(1*H-*indol-3-yl)-1-méthyléthyl]benzènesulfonamide;
3-bromo-*N-*[2-(1*H-*indol-3-yl)-1-méthyléthyl]benzènesulfonamide;
*N-*[2-(1*H-*indol-3-yl)-1-méthyléthyl]-2-tritluorométhoxybenzènesulfonamide;
3-cyano-*N-*[2-(1*H-*indol-3-yl)-1-méthyléthyl]benzènesulfonamide;
2-cyano-*N-*[2-(1*H-*indol-3-yl)-1-méthyléthyl]benzènesulfonamide;
*N-*[2-(1*H-*indol-3-yl)-1-méthyléthyl]-2-méthoxy-5-méthylbenzènesulfonamide;
2-chloro-4-fluoro-*N-*[2-(1*H-*indol-3-yl)-1-méthyléthyl]benzènesulfonamide;
5-chloro-*N-*[2-(1*H-*indol-3-yl)-1-méthyléthyl]-2-méthoxybenzènesulfonamide;
[2-(1*H-*indol-3-yl)-1-méthyléthyl]amide d'acide biphényle-4-sulfonique;
2,6-difluoro-*N-*[2-(1*H-*indol-3-yl)-1-méthyléthyl]benzènesulfonamide;
*N*-[2-(1*H-*indol-3-yl)-1-méthyléthyl]-4-méthoxy-3,5-dinitrobenzènesulfonamide;
*N*-[2-(1*H-*indol-3-yl)-1-méthyléthyl]-4-méthanesulfonylbenzènesulfonamide;
*N*-[2-(1*H-*indol- 3-yl)-1-méthyléthyl]-2-méthanesulfonylbenzènesulfonamide;
4-acétyl-*N-*[2-(1*H-*indol-3-yl)-1-méthyléthyl]benzènesulfonamide;
3,5-dichloro-*N-*[2-(1*H-*indol-3-yl)-1-méthyléthyl]benzènesulfonamide; et
*N-*[2-(1*H-*indol-3-yl)-1-méthyléthyl]-3,5-bis-trifluorométhylbenzènesulfonamide;
ou tautomère, ester, amide, solvate ou sel de celui-ci.

15. Composé selon la revendication 1, le composé choisi parmi :
[2-(1*H-*indol-3-yl)-1-méthyléthyl]amide d'acide 5-diméthylaminonaphtalène-1-sulfonique ;
*N-*[2-(1*H-*indol-3-yl)-1-méthyléthyl]-2,4,6-triméthylbenzènesulfonamide;
*N-*[2-(1*H-*indol-3-yl)-1-méthyléthyl]-2,3,4,5,6-pentaméthylbenzènesulfonamide;
*N-*[2-(1*H-*indol-3-yl)-1-méthyléthyl]-4-méthoxy-2,3,6-triméthylbenzènesulfonamide;
2-chloro-*N-*[2-(1*H-*indol-3-yl)-1-méthyléthyl]-6-méthylbenzènesulfonamide;
2,6-dichloro-*N-*[2-(1*H-*indol-3-yl)-1-méthyléthyl]benzènesulfonamide;
2,4,6-trichloro-*N-*[2-(1*H-*indol-3-yl)-1-méthyléthyl]benzènesulfonamide;
*N-*[2-(1*H-*indol-3-yl)-1-méthyléthyl]-2,4,6-triisopropylbenzènesulfonamide;
*4-tert*-butyl*-N-*[1-(1*H-*indol-3-ylméthyl)propyl]benzènesulfonamide;
2,4,6-trichloro-*N-*[1-(1*H-*indol-3-ylméthyl)propyl]benzènesulfonamide;
*N-*[2-(5-fluoro-1*H-*indol-3-yl)-1-méthyléthyl]-2,4,6-triméthylbenzènesulfonamide;
*N-*[1-(1*H-*indol-3-ylméthyl)propyl]-2,4,6-triméthylbenzènesulfonamide;
*N-*[2-(6-fluoro-1*H-*indol-3-yl)-1-méthyléthyl]-2,4,6-triméthylbenzènesulfonamide,
2,4,6-triméthyl-*N-*[1-méthyl-2-(6-méthyl-1*H-*indol-3-yl)éthyl]benzènesulfonamide; et
2,4,6-triméthyl-*N-*[1-méthyl-2-(7-méthyl-1*H-*indol-3-yl)éthyl]benzènesulfonamide, ou tautomère, ester, imide, solvate ou sel de celui-ci.

16. Composé selon la revendication 1, le composé choisi parmi :
*N-*[2-(1*H-*indol-3-yl)-1-méthyléthyl]-2,4,6-triméthylbenzènesulfonamide;
2,4,6-trichloro-*N*-[2-(1*H-*indol-3-yl)-1-méthyléthyl]benzènesulfonamide;
*N-*[2-(1*H-*indol-3-yl)-1-méthyléthyl]-2,4,6-triisopropylbenzènesulfonamide;
2,4,6-trichloro-N*-*[1-(1*H-*indol-3-ylméthyl)propyl]benzènesulfonamide;
*N-*[2-(5-fluoro-1*H-*indol-3-yl)-1-méthyléthyl]-2,4,6-triméthylbenzènesulfonamide;
*N-*[1-(1*H-*indol-3-ylméthyl)propyl]-2,4,6-triméthylbenzènesulfonamide;
*N-*[2-(6-fluoro-1*H-*indol-3-yl)-1-méthyléthyl]-2,4,6-triméthylbenzènesulfonamide; et
2,4,6-triméthyl-*N*-[1-méthyl-2-(6-méthyl-1*H-*indol-3-yl)éthyl]benzènesulfonamide,
ou tautomère, ester, imide, solvate ou sel de celui-ci.

17. Composition pharmaceutique comprenant une quantité efficace d'un composé selon l'une des revendications 1 à 16, ou d'un tautomère, ester, amide, solvate ou sel de celui-ci, et un excipient ou vecteur pharmaceutiquement acceptable.

18. Utilisation d'un composé selon l'une des revendications 1 à 16, ou d'un tautomère, ester, amide, solvate ou sel de celui-ci pour la préparation d'une composition pharmaceutique pour moduler la fonction de récepteur de glucocorticoïdes chez un patient.

19. Utilisation d'un composé selon l'une des revendications 1 à 16, ou d'un tautomère, ester, amide, solvate ou sel de celui-ci pour la préparation d'une composition pharmaceutique pour traiter un état morbide ou affection à médiation par la fonction de récepteur de glucocorticoïdes chez un patient.

20. Utilisation d'un composé selon l'une des revendications 1 à 16, ou d'un tautomère, ester, amide, solvate ou sel de celui-ci pour la préparation d'une composition pharmaceutique pour traiter un état morbide ou affection choisi parmi : le diabète de type II, l'obésité, les maladies cardiovasculaires, l'hypertension, l'artériosclérose, les maladies neurologiques, les tumeurs surrénales et pituitaires et le glaucome, chez un patient.

21. Utilisation d'un composé selon l'une des revendications 1 à 16, ou d'un tautomère, ester, amide, solvate ou sel de celui-ci pour la préparation d'une composition pharmaceutique pour traiter une maladie **caractérisée par** des processus inflammatoires, allergiques ou prolifératifs, chez un patient.

22. Utilisation selon la revendication 21 où la maladie est choisie parmi : (i) les maladies pulmonaires ; (ii) les maladies rhumatismales/maladies auto-immunes/maladies articulaires ; (iii) les maladies allergiques ; (iv) les maladies de type vascularite ; (v) les maladies dermatologiques ; (vi) les maladies rénales ; (vii) les maladies hépatiques ; (viii) les maladies gastro-intestinales ; (ix) les maladies proctologiques ; (x) les maladies des yeux ; (xi) les maladies de la sphère oto-rhino-laryngologique (ORL) ; (xii) les maladies neurologiques ; (xiii) les maladies du sang ; (xiv) les maladies tumorales ; (xv) les maladies endocriniennes ; (xvi) les transplantations d'organes et de tissus et les maladies du greffon contre l'hôte ; (xvii) les états de choc graves ; (xviii) la thérapie substitutive ; et (xix) la douleur de genèse inflammatoire.

23. Utilisation selon la revendication 21 où la maladie est choisie parmi : le diabète de type I, l'arthrose, le syndrome de Guillain-Barré, la resténose consécutive à une angioplastie coronaire transluminale percutanée, la maladie d'Alzheimer, la douleur aiguë et chronique, l'athérosclérose, les lésions de reperfusion, les maladies de résorption osseuse, l'insuffisance cardiaque congestive, l'infarctus du myocarde, les lésions thermiques, les lésions d'organes multiples secondaires à un traumatisme, la méningite purulente aiguë, l'entérocolite nécrosante et les syndromes associés avec l'hémodialyse, la leucophérère et la transfusion de granulocytes.

24. Utilisation (a) d'un composé selon l'une des revendications 1 à 16 ou d'un tautomère, ester, amide, solvate ou sel de celui-ci ; et (b) d'un glucocorticoïde pour la préparation d'une composition pharmaceutique pour traiter un état morbide ou affection à médiation par la fonction de récepteur de glucocorticoïdes chez un patient.

25. Kit pour la détermination diagnostique *in vitro* de la fonction de récepteur de glucocorticoïdes dans un échantillon, comprenant :
(a) une quantité efficace du point de vue diagnostique d'un composé selon la revendication 1 ou d'un tautomère, ester, amide, solvate ou sel de celui-ci ; et
(b) des instructions pour l'utilisation du kit de diagnostic.

26. Procédé de production d'un composé de formule (I) 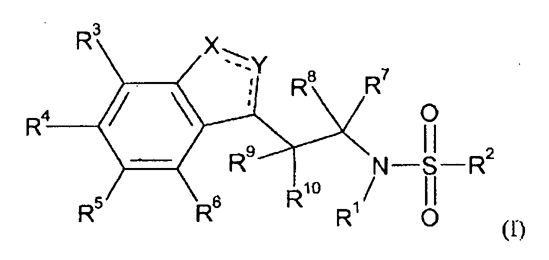 où R¹ est H et R², R³, R⁴, R⁵, R⁶, R⁷, R⁸, R⁹ R¹⁰, X et Y sont définis comme dans la revendication 1, le procédé comprenant la réaction de l'aminoéthyl hétérocycle de formule (II) avec un halogénure de sulfonyle de formule (III) en présence d'une base appropriée pour former le composé de formule (I)

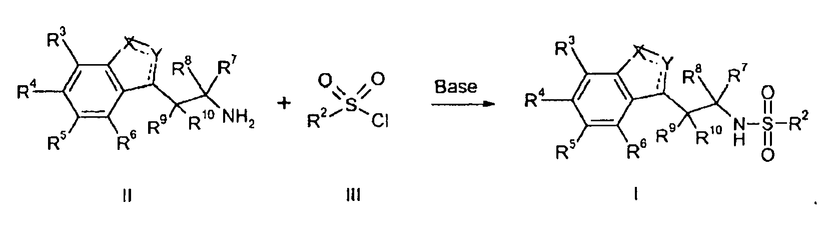

27. Procédé de production d'un composé de formule (I) 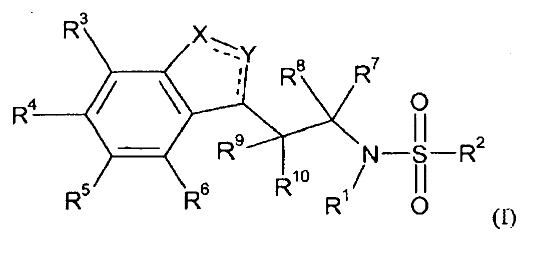 où R¹, R⁸ et R⁹ sont chacun H et R², R³, R⁴, R⁵, R⁶, R⁷, R¹⁰, X et Y sont définis comme dans la revendication 1, le procédé comprenant :
(a) la réaction de l'aldéhyde de formule (V) avec le composé nitro de formule (VI) en présence d'acétate d'ammonium et d'acide acétique pour former le nitroalcène de formule (VII) 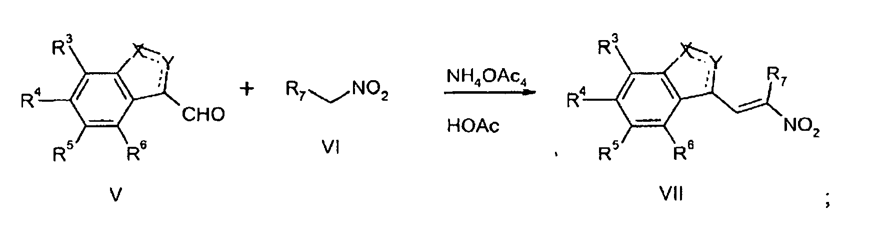
(b) la réduction du nitroalcène de formule (VII) avec un agent réducteur approprié pour former l'intermédiaire de formule (II) 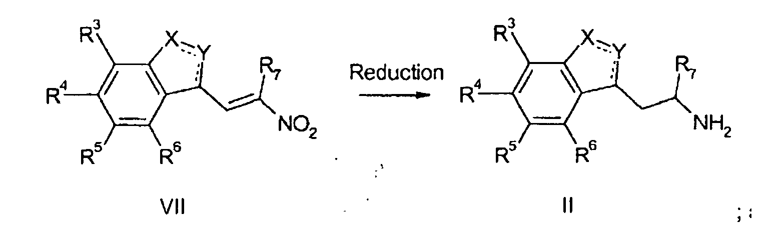 et
(c) la réaction de l'intermédiaire de formule (II) avec un halogénure de sulfonyle de formule (III) en présence d'une base appropriée pour former le composé de formule (I)
